# EUROPEAN PATENT SPECIFICATION

(11) **EP 0 904 299 B1**
(45) Date of publication and mention of the grant of the patent: **29.08.2001**
(21) Application number: 97928337.1
(22) Date of filing: 11.06.1997
(51) Int. Cl.: C08B 37/00, A61K 31/715

(54) **SYNTHETIC POLYSACCHARIDES, METHOD FOR PREPARING SAME AND PHARMACEUTICAL COMPOSITIONS CONTAINING SAID POLYSACCHARIDES**
SYNTHETISCHE POLYSACCHARIDE, VERFAHREN ZU DEREN HERSTELLUNG UND DIESE ENTHALTENDE PHARMAZEUTISCHE ZUSAMMENSETZUNGEN
POLYSACCHARIDES SYNTHETIQUES, PROCEDE POUR LEUR PREPARATION ET COMPOSITIONS PHARMACEUTIQUES LES CONTENANT

(30) Priority: 14.06.1996 FR 9607457
(43) Date of publication of application: 31.03.1999
(73) Proprietor: SANOFI-SYNTHELABO, 75635 Paris Cédex 13 (FR); Akzo Nobel N.V., 6824 BM Arnhem (NL)
(72) Inventor: DUCHAUSSOY, Philippe, F-31300 Toulouse (FR); HERBERT, Jean-Marc, F-31170 Tournefeuille (FR); JAURAND, Guy, F-04700 Sisteron (FR); PETITOU, Maurice, F-75645 Paris Cedex 13 (FR); VAN BOECKEL, Constant, NL-5345 LX Oss (NL)
(74) Representative: Le Guen, Gérard
(86) International application number: PCT/FR97/01048
(87) International publication number: WO 97/47659

(56) References cited:
- US-A- 5 378 829

## Description

La présente invention concerne de nouveaux polysaccharides de synthèse possédant les activités pharmacologiques de l'héparine mais exercées de façon sélective.

L'héparine appartient à la famille des glycosaminoglycanes (GAG), qui sont des polysaccharides sulfatés naturels hétérogènes.

Les préparations d'héparine sont des mélanges de chaînes comprenant un nombre d'unités monosaccharidiques allant de 10 jusqu'à 100 et plus. A cette hétérogénéité de taille s'ajoute une hétérogénéité de structure, au niveau de la nature des monosaccharides constitutifs, mais également au niveau des substituants qu'ils portent (L. Rodén in : The Biochemistry of Glycoproteins and Glycosaminoglycans, Ed by Lennarz W.J., Plenum Press, 1980, New York and London, 267-371).

Chaque famille de GAG naturel possède en général un éventail d'activités pharmacologiques. Toutes sont réunies dans les préparations que l'on peut obtenir à partir de produits naturels. Ainsi, par exemple, les héparines et les héparanes sulfate possèdent une activité antithrombotique qui est liée à l'action simultanée sur plusieurs facteurs de la coagulation. Ils exercent également une action sur de nombreux facteurs de croissance, le plus notoire étant le facteur de croissance basique des fibroblastes (bFGF, basic fibroblast growth factor).

Cette action se manifeste par un effet sur la prolifération des cellules musculaires lisses et sur l'angiogénèse. L'héparine exerce en outre des effets sur l'autoimmunité, l'inflammation et la formation de métastases tumorales.

L'héparine catalyse notamment l'inhibition de deux enzymes qui interviennent dans la cascade de la coagulation du sang à savoir, le facteur Xa et le facteur Ila (ou thrombine). Les préparations d'héparines de bas poids moléculaire (HBPM), contiennent des chaînes formées de 4 à 30 monosaccharides et ont la propriété d'agir plus sélectivement sur le facteur Xa que sur la thrombine.

Certains oligosaccharides de synthèse notamment ceux décrits dans EP 84999 ont la propriété d'inhiber sélectivement, via l'antithrombine III, le facteur Xa sans aucune activité sur la thrombine.

Le brevet US 5,378,829 décrit des dérivés glycosaminoglycanoïdes sulfatés du type héparine ou héparane sulfate ayant une activité antithrombotique et inhibitrice de la prolifération des cellules musculaires lisses. Ce document ne décrit cependant que des oligosaccharides ayant au maximum 7 unités monosaccharidiques.

Au cours des années précédentes, la tendance dans le domaine des GAGS était de rechercher notamment des oligosaccharides de masse moléculaire la plus faible possible, mais il a été par la suite observé que plusieurs des activités biologiques sus-mentionnées sont soutenues par des fragments ayant au moins 8 unités saccharidiques. Par exemple, l'activité inhibitrice de la thrombine nécessiterait des fragments contenant au moins entre 14 et 20 unités saccharidiques tandis que pour l'activation du bFGF au moins 12 unités saccharidiques seraient nécessaires (M. Maccarana *et al*., *J.* Biol. Chem., 1993, 268, 23998-23905 ; J.E. Turnbull *et al*., J. Biol. Chem., 1992,267, 10337-10341).

Une synthèse de polysaccharides de cette taille présente de grandes difficultés et, en fait, elle n'a jamais été réalisée.

Dans le but de retrouver l'activité de produits de longue taille, il a été proposé de relier deux oligosaccharides de petite taille par une entité n' intervenant pas dans I' activité biologique. EP 649854 décrit de tels dérivés possédant une activité antithrombotique. De façon analogue, WO 95 05182 décrit des conjugués actifs sur la régulation du bFGF.

Ces produits présentent en effet une activité sélective sur les différents facteurs de la coagulation et des propriétés inhibitrices des facteurs de croissance se traduisant par une inhibition de la prolifération cellulaire.

Il a maintenant été trouvé que des fragments de GAGs (glycosaminoglycanes) contenant 8 ou plus unités saccharidiques et pouvant être synthétisés de façon relativement simple possèdent des activités biologiques qui sont non seulement sélectives mais aussi quantativement élevées.

Plus particulièrement, il a été trouvé qu'il est possible de moduler quantitativement l'activité desdits polysaccharides selon la longueur des chaines et la distribution des substituants fonctionnels.

Ainsi, par exemple, il a été trouvé de façon surprenante que des décasaccharides sulfatés et alkylés peuvent être des puissants antithrombotiques ou des inhibiteurs sélectifs du bFGF selon la disposition des groupes alkyles et des groupes sulfates dans l'enchainement décaccharidiques et qu'il est également possible d'obtenir des inhibiteurs sélectifs du facteur Xa par exemple avec un tétradécasaccharide ou des produits ayant une activité du type héparine, à savoir une activité aussi bien sur le facteur Xa que sur la thrombine, par exemple avec un hexadécasaccharide.

De façon plus générale, il a été trouvé que par réalisation de séquences disaccharidiques formées par un acide uronique et par un hexose de façon à constituer des polysaccharides de 8 à 24 unités monosaccharidiques dont les groupes hydroxyles sont tous substitués par des groupes alkyles ou par des groupes sulfates il est possible de moduler avec précision les activités du type GAGs pour obtenir des produits très actifs et sélectifs.

Ainsi, selon un de ses aspects, la présente invention concerne un nouveau polysaccharide de synthèse contenant de 8 à 24 unités monosaccharidiques formé par un enchaînement de disaccharides constitués d'un acide uronique et d'un hexose, ledit polysaccharide étant caractérisé en ce que tous ses groupes hydroxyles sont éthérifiés avec un groupe (C₁-C₆)alkyle ou estérifiés sous la forme de groupe sulfo, chaque disaccharide étant au moins monoéthérifié ; ainsi que ses sels, notamment pharmaceutiquement acceptables.

De préférence, l'invention se rapporte à un polysaccharide tel que défini ci-dessus, caractérisé en ce que tous ses groupes hydroxyle sont éthérifiés par un groupe méthyle ou estérifiés sous la forme de groupe sulfo, chaque disaccharide étant au moins monoéthérifié; et à ses sels, notamment pharmaceutiquement acceptables.

Des produits avantageux sont alkylés, de préférence méthylés, sur les hydroxyles en position 2 et 3 de l'acide uronique, ledit acide uronique étant de préférence l'acide glucuronique ou l'acide iduronique et trisulfatés sur l'hexose, ledit hexose étant de préférence le glucose. Un autre groupe de produits préférés est constitué des polysaccharides qui sont alkylés, de préférence méthylés, sur les hydroxyles en position 3 de l'acide uronique ledit acide étant de préférence l'acide glucuronique ou l'acide iduronique et alkylés, de préférence méthylés, sur l'hydroxyle en position 3 du glucose.

Les produits de la présente invention sont donc de préférence constitués de séquences régulières de disaccharides répétés à leur tour formés d'acide glucuronique et de glucose ou d'acide iduronique et de glucose représentés par la formule suivante : 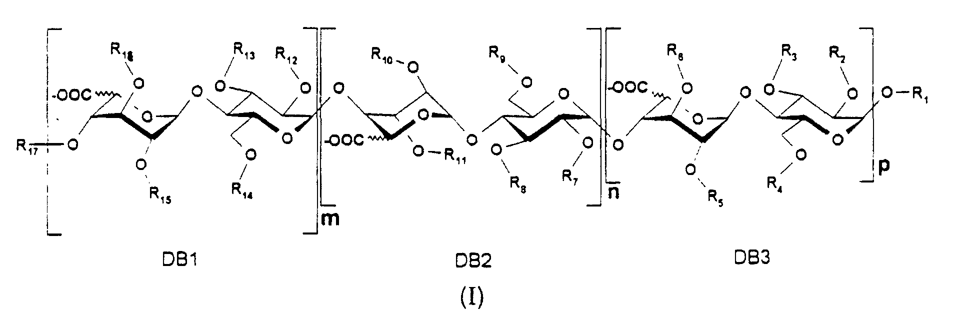 dans laquelle
- le trait ondulé désigne soit une liaison en dessous soit en dessus du plan du cycle pyranosique ;
- R₁, R₆, R₁₁ et R₁₆ représentent un (C₁-C₆)alkyle ;
- R₂, R₃, R₄, R₅, R₇, R₈, R₉, R₁₀, R₁₂, R₁₃, R₁₄, R₁₅ et R₁₇ représentent un (C₁-C₆)alkyle ou un groupe SO₃⁻;
- m, n et p sont tels que la somme m + n + p est supérieure ou égale à 4 et inférieure ou égale à 12, un ou deux des trois pouvant représenter zéro, ou un de leurs sels, notamment pharmaceutiquement acceptables.

On notera que de façon générale dans la présente description, un trait ondulé désigne soit une liaison en dessous soit en dessus du plan du cycle pyranosique.

Dans la formule générale (I) et dans la présente description, les structures (a) ou (b) ci-dessous représentent un squelette de glucose dans la conformation ⁴*C*₁. Les structures (c) ou (d) ci-dessous représentent un squelette d'acide uronique qui est soit de l'acide L-iduronique (représenté ici dans sa conformation ¹*C*₄) soit de l'acide D-glucuronique (représenté ici dans sa conformation ⁴C₁).

Sur ces structures (a), (b), (c) et (d) les substituants Rₓ ont les définitions attribuées pour R₁ à R₁₇ dans (I).

Ainsi, les structures (a) et (b) sont la même représentation d'un squelette de glucose dans la conformation ⁴*C*₁.

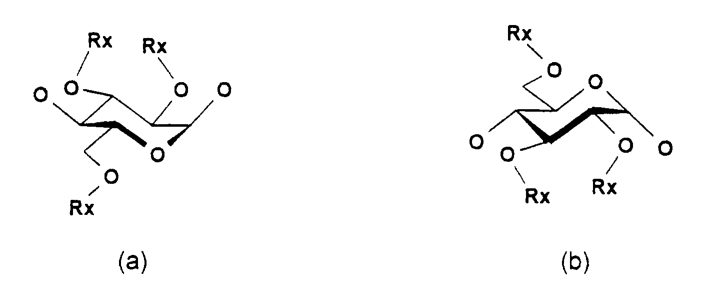

Les structures (c) et (d) représentent un squelette d'acide uronique qui est soit de l'acide L-iduronique (représenté ici dans sa conformation ¹*C*₄) soit de l'acide D-glucuronique (représenté ici dans sa conformation ⁴*C*₁).

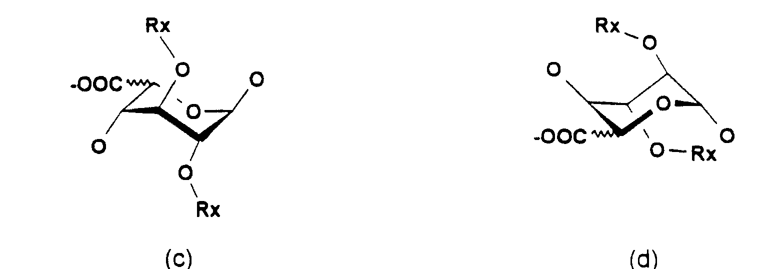

Lorsqu'il s'agit d'acide L-iduronique (c) et (d) sont les conformères suivants:

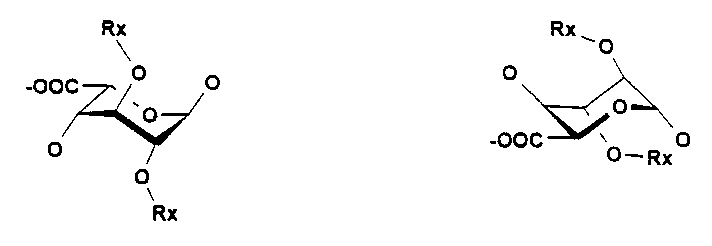

Lorsqu'il s'agit d'acide D-glucuronique (c) et (d) sont les conformères suivants:

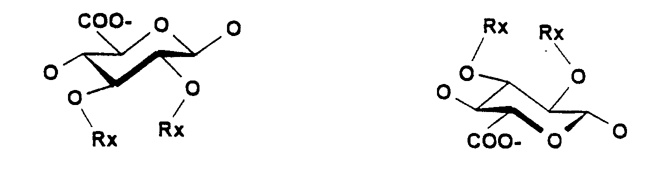

Dans la présente description, il a été choisi de représenter les conformations ¹*C*₄ pour l'acide-L-iduronique, ⁴*C*₁ pour l'acide D-glucuronique, mais il est notoire que la conformation en solution des unités monosaccharides est fluctuante.

Les disaccharides DB1, DB2 et DB3 représentent des disaccharides identiques ou différents.

Des composés préférés selon l'invention sont ceux de formule (I) dans lesquels n et p sont égaux à zéro et m représente 4 à 10 et leurs sels, notamment pharmaceutiquement acceptables.

Egalement avantageux sont les composés de formule (I) dans lesquels n et p sont égaux à zéro, m représente 4 à 10 ; un au moins des substituants R₁₂, R₁₃, R₁₄ et R₁₅ représente un groupe sulfate ; R₁, R₁₆ et R₁₇ étant tels que définis pour (I) ainsi que leurs sels, notamment pharmaceutiquement acceptables.

Egalement avantageux sont les composés de formule (I) dans lesquels n et p sont égaux à zéro, m représente 4 à 10 ; deux au moins des substituants R₁₂, R₁₃, R₁₄ et R₁₅ représentent un groupe sulfate ; R₁, R₁₆ et R₁₇ étant tels que définis pour (I) ainsi que leurs sels, notamment pharmaceutiquement acceptables.

Egalement avantageux sont les composés de formule (I) dans lesquels n et p sont égaux à zéro, m représente 4 à 10 ; trois au moins des substituants R₁₂, R₁₃, R₁₄ et R₁₅ représentent un groupe sulfate ; R₁, R₁₆ et R₁₇ étant tels que définis pour (I), ainsi que leurs sels notamment pharmaceutiquement acceptables.

Egalement avantageux sont les composés de formule (I) dans lesquels n et p sont égaux à zéro, m représente 4 à 10 ; les groupes R₁₂, R₁₃, R₁₄ et R₁₅ représentent tous un groupe sulfate ; R₁, R₁₆ et R₁₇ étant tels que définis pour (I) et leurs sels, notamment pharmaceutiquement acceptables.

D'autres composés avantageux sont les sels constitués d'un anion et d'un cation, l'anion répondant à la formule (I.1) : 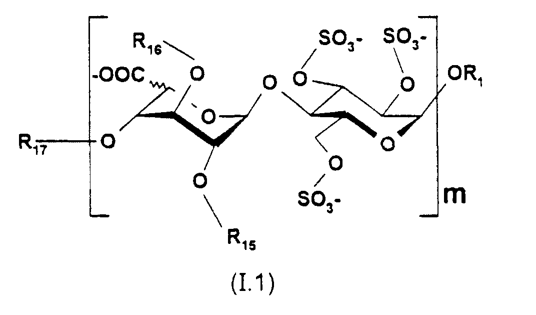 dans laquelle m représente 4 à 10 ; R₁, R₁₅, R₁₆ et R₁₇ sont tels que définis pour (I), chaque acide uronique étant soit un acide iduronique soit glucuronique, et, le cation étant un cation monovalent pharmaceutiquement acceptable, ainsi que leurs acides correspondants.

Les sels constitués d'un anion et d'un cation où l'anion répond à la formule (I.2): 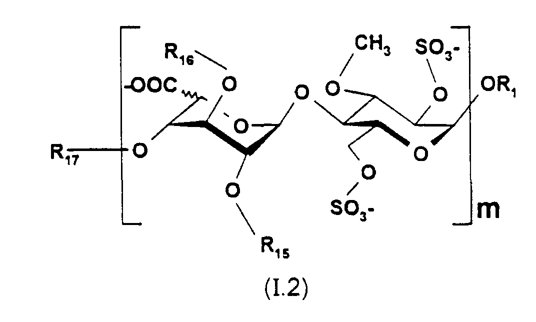 dans laquelle m représente 4 à 10 ; R₁, R₁₅, R₁₆ et R₁₇ sont tels que définis pour (I), chaque acide uronique étant soit un acide iduronique soit glucuronique et où le cation est un cation monovalent pharmaceutiquement acceptable, ainsi que leurs acides correspondants, sont également avantageux.

Dans les formules (I), (I.1), (I.2) ci-dessus, les groupes alkyle éthérifiants sont de préférence des méthyles.

Les sels constitués d'un anion et d'un cation, où l'anion a pour formule (I.3) : 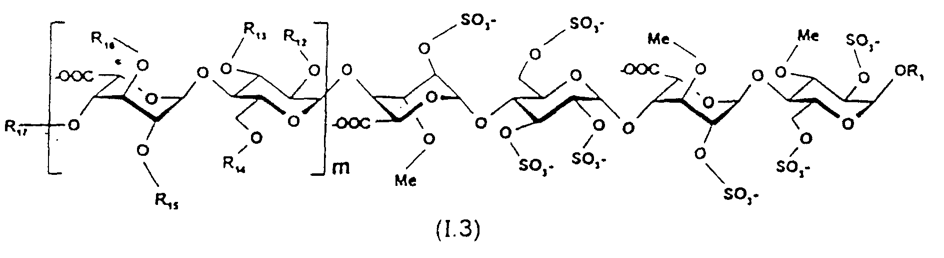 dans laquelle m représente 2 ou 3, R₁, R₁₂, R₁₃, R₁₄, R₁₅, R₁₆ et R₁₇ étant tels que définis pour (I), chaque acide uronique étant soit un acide iduronique soit glucuronique et où le cation est un cation monovalent pharmaceutiquement acceptable, ainsi que leurs acides correspondants, sont particulièrement avantageux.

Parmi ces composés (1.3), on préfère tout particulièrement ceux pour lesquels R₁ représente un méthyle, R₁₃ en position 3 du glucose représente un méthyle, R₁₂ en position 2 et R₁₄ en position 6 du glucose représentent un SO₃⁻ et R₁₆ en position 3 de l'unité iduronique ou glucuronique représente un méthyle, m étant égal à 2 ou 3.

Les sels préférés de l'invention sont ceux dont le cation est choisi parmi les cations des métaux alcalins et plus préférablement encore ceux dont le cation est Na⁺ ou K⁺.

Particulièrement préférés sont les polysaccharides suivants :
1) Méthyl *O*-(acide 2,3-di-*O*-méthyl-4-O-sulfo-α-L-idopyranosyluronique)-(1-4)-[*O*-(2,3,6-tri-*O*-sulfo-α-D-glucopyranosyl)-(1-4)-*O*-(acide 2,3-di-*O*-méthyl-α-L-idopyranosyluronique)-(1-4)]₉-2,3,6-tri-*O*-sulfo-α-D-glucopyranoside, sel de sodium.
2) Méthyl *O*-(acide 2,3-di-*O*-méthyl-4-O-sulfo-α-L-idopyranosyluronique)-(1-4)-[*O*-(2,3,6-tri-*O*-sulfo-α-D-glucopyranosyl)-(1-4)-*O*-(acide 2,3-di-*O*-méthyl-α-L-idopyranosyluronique)-(1-4)]₄-2,3,6-tri-*O*-sulfo-α-D-glucopyranoside, sel de sodium.
3) Méthyl *O*-(acide 2,3-di-*O*-méthyl-4-O-sulfo-α-L-idopyranosyluronique)-(1-4)-[*O*-(2,3,6-tri-*O*-sulfo-α-D-glucopyranosyl)-(1-4)-*O*-(acide 2,3-di-*O*-méthyl-α-L-idopyranosyluronique)-(1-4)]₅-2,3,6-tri-*O*-sulfo-α-D-glucopyranoside, sel de sodium.
4) Méthyl *O*-(acide 2,3-di-*O*-méthyl-4-O-sulfo-α-L-idopyranosyluronique)-(1-4)-[*O*-(2,3,6-tri-*O*-sulfo-α-D-glucopyranosyl)-(1-4)-*O*-(acide 2,3-di-*O*-méthyl-α-L-idopyranosyluronique)-(1-4)]₆-2,3,6-tri-*O*-sulfo-α-D-glucopyranoside, sel de sodium.
5) Méthyl *O*-(acide 2,3-di-*O*-méthyl-4-O-sulfo-α-L-idopyranosyluronique)-(1-4)-[*O*-(2,3,6-tri-*O*-sulfo-α-D-glucopyranosyl)-(1-4)-*O*-(acide 2,3-di-*O*-méthyl-α-L-idopyranosyluronique)-(1-4)]₇-2,3,6-tri-*O*-sulfo-α-D-glucopyranoside, sel de sodium.
6) Méthyl *O*-(acide 2,3-di-*O*-méthyl-4-O-sulfo-α-L-idopyranosyluronique)-(1-4)-[*O*-(2,3,6-tri-*O*-sulfo-α-D-glucopyranosyl)-(1-4)-*O*-(acide 2,3-di-*O*-méthyl-α-L-idopyranosyluronique)-(1-4)]₈-2,3,6-tri-*O*-sulfo-α-D-glucopyranoside, sel de sodium.
7) Méthyl *O*-(acide 2,3-di-*O*-méthyl-4-O-sulfo-β-D-glucopyranosyluronique)-(1-4)-[*O*-(2,3,6-tri-*O*-sulfo-α-D-glucopyranosyl)-(1-4)-*O*-(acide 2,3-di-*O*-méthyl-β-D-glucopyranosyluronique)-(1-4)]4-2,3,6-tri-*O*-sulfo-α-D-glucopyranoside, sel de sodium.
8) Méthyl *O*-(acide 2,3-di-*O*-méthyl-4-O-sulfo-β-D-glucopyranosyluronique)-(1-4)-[*O*-(2,3,6-tri-*O*-sulfo-α-D-glucopyranosyl)-(1-4)-*O*-(acide 2,3-di-*O*-méthyl-β-D-glucopyranosyluronique)-(1-4)]₃-2,3,6-tri-*O*-sulfo-α-D-glucopyranoside, sel de sodium.
9) Méthyl *O*-(acide 3-*O*-méthyl-2,4-di-*O*-sulfo-α-L-idopyranosyluronique)-(1-4)-[*O*-(3-*O*-méthyl-2,6-di-*O*-sulfo-α-D-glucopyranosyl)-(1-4)-*O*-(acide 3-*O*-méthyl-2-*O*-sulfo-α-L-idopyranosyluronique)-(1-4)]₄-3-*O*-méthyl-2,6-di-*O*-sulfo-α-D-glucopyranoside, sel de sodium.
10) Méthyl *O*-(acide 3-*O*-méthyl-2,4-di-*O*-sulfo-α-L-idopyranosyluronique)-(1-4)-[*O*-(3-*O*-méthyl-2,6-di-*O*-sulfo-α-D-glucopyranosyl)-(1-4)-*O*-(acide 3-*O*-méthyl-2-*O*-sulfo-α-L-idopyranosyluronique)-(1-4)]₃-3-*O*-méthyl-2,6-di-*O*-sulfo-α-D-glucopyranoside, sel de sodium.
11) Méthyl *O*-(acide 3-*O*-méthyl-2,4-di-*O*-sulfo-α-L-idopyranosyluronique)-(1-4)-[*O*-(3-*O*-méthyl-2,6-di-*O*-sulfo-α-D-glucopyranosyl)-(1-4)-*O*-(acide 3-*O*-méthyl-2-*O*-sulfo-α-L-idopyranosyluronique)-(1-4)]₅-3-*O*-méthyl-2,6-di-*O*-sulfo-α-D-glucopyranoside, sel de sodium.
12) Méthyl *O*-(acide 3-*O*-méthyl-2,4-di-*O*-sulfo-β-D-glucopyranosyluronique)-(1-4)-[*O*-(3-*O*-méthyl-2,6-di-*O*-sulfo-α-D-glucopyranosyl)-(1-4)-*O*-(acide 3-*O*-méthyl-2-*O*-sulfo-β-D-glucopyranosyluronic acid)-(1-4)]₄-3-*O*-méthyl-2,6-di-*O*-sulfo-α-D-glucopyranoside, sel de sodium.
13) Méthyl *O*-(acide 3-*O*-méthyl-2,4-di-*O*-sulfo-α-L-idopyranosyluronique)-(1-4)-[*O*-(3-*O*-méthyl-2,6-di-*O*-sulfo-α-D-glucopyranosyl)-(1-4)-*O*-(acide 3-*O*-méthyl-2-*O*-sulfo-α-L-idopyranosyluronique)-(1-4)]₂-*O*-(2,3,6-tri-*O*-sulfo-α-D-glucopyranosyl)-(1-4)-*O*-(acide 3-*O*-méthyl-2-*O*-sulfo-α-L-idopyranosyluronique)-(1-4)-3-*O*-méthyl-2,6-di-*O*-sulfo-α-D-glucopyranoside, sel de sodium.
14) Méthyl *O*-(acide 3-*O*-méthyl-2,4-di-*O*-sulfo-α-L-idopyranosyluronique)-(1-4)-[*O*-(3-*O*-méthyl-2,6-di-*O*-sulfo-α-D-glucopyranosyl)-(1-4)-*O*-(acide 3-*O*-méthyl-2-*O*-sulfo-α-L-idopyranosyluronique)-(1-4)]₃-*O*-(2,3,6-tri-*O*-sulfo-α-D-glucopyranosyl)-(1-4)-*O*-(acide 3-*O*-méthyl-2-*O*-sulfo-α-L-idopyranosyluronique)-(1-4)-3-*O*-méthyl-2,6-di-*O*-sulfo-α-D-glucopyranoside, sel de sodium.

La présente invention concerne également un procédé pour la préparation des composés de formule (I) caractérisé en ce que :
(a) on couple selon les méthodes classiques de la chimie des sucres un monosaccharide donneur de liaison glycosidique à un monosaccharide accepteur de liaison glycosidique pour obtenir après les modifications chimiques bien connues de l'homme du métier un synthon saccharidique intermédiaire de type disaccharide complètement protégé de formule (A) : 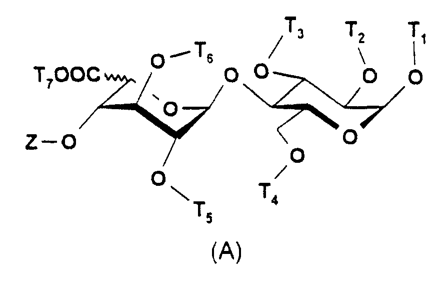 dans laquelle les substituants T₁, T₂, T₃, T₄, T₅, T₆, T₇, et Z identiques ou différents sont choisis parmi les groupes protecteurs utilisés en chimie des sucres comme groupe protecteur permanent, semi-permanent ou temporaire,
(b) on modifie chimiquement le disaccharide de formule (A) ci-dessus de façon à obtenir un synthon saccharidique intermédiaire de type disaccharide donneur de liaison glycosidique de formule (B) : 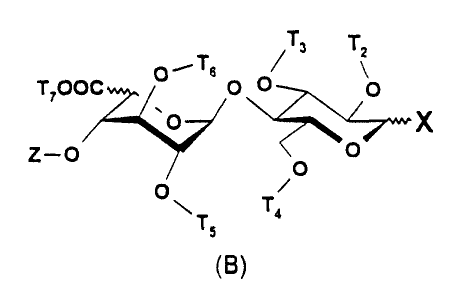 dans laquelle T₂ à T₇ et Z sont tels que définis ci-dessus pour (A) et X représente un groupe activateur du carbone anomérique, tel qu'un imidate, un thioglycoside, un pentényl glycoside, un xanthate, un phosphite, un halogénure ou tout autre groupement bien connu pour activer le carbone anomérique, puis
(c) on modifie chimiquement le disaccharide de formule (A) ci-dessus de façon à obtenir un synthon saccharidique intermédiaire de type disaccharide accepteur de liaison glycosidique de formule (C) : 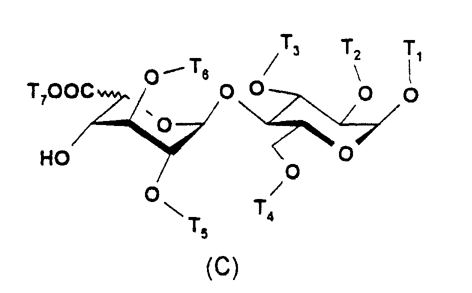 dans laquelle T₁ à T₇ sont tels que définis ci-dessus pour (A), en éliminant sélectivement le groupe protecteur Z selon des méthodes bien connue de l'homme de l'art, puis
(d) on couple un disaccharide donneur de liaison glycosidique de formule (B) obtenu ci-dessus et un disaccharide accepteur de liaison glycosidique de formule (C) obtenu ci-dessus de façon à obtenir un tétrasaccharide complètement protégé de formule (D) : 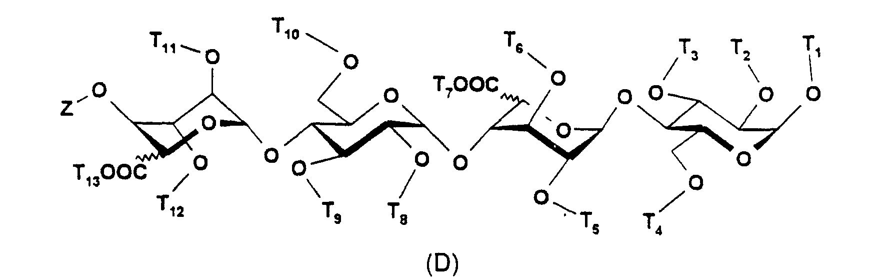 dans laquelle T₁ à T₇ et Z sont tels que définis ci-dessus pour (A) et T₈, T₉, T₁₀, T₁₁, T₁₂ et T₁₃ sont tels que définis pour T₂ à T₇ puis,
(e) on modifie ensuite chimiquement le tétrasaccharide de formule (D) de façon à obtenir un synthon saccharidique intermédiaire de type tétrasaccharide donneur de liaison glycosidique de formule (E) : 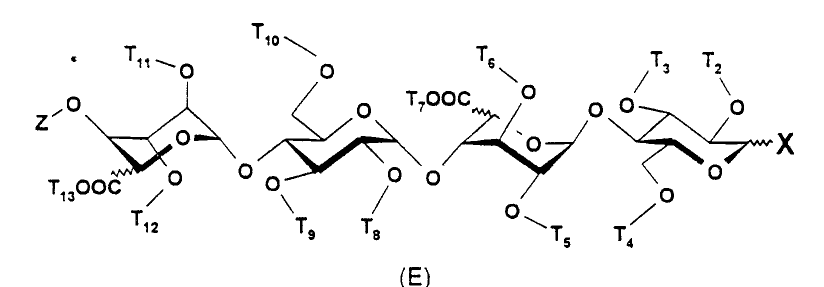 dans lequel X a la même définition que pour (B) et T₂ à T₁₃ sont tels que définis pour (D) puis,
(f) on déprotège ensuite sélectivement le tétrasaccharide de formule (D) de façon à obtenir un tétrasaccharide accepteur de liaison glycosidique de formule (F) : 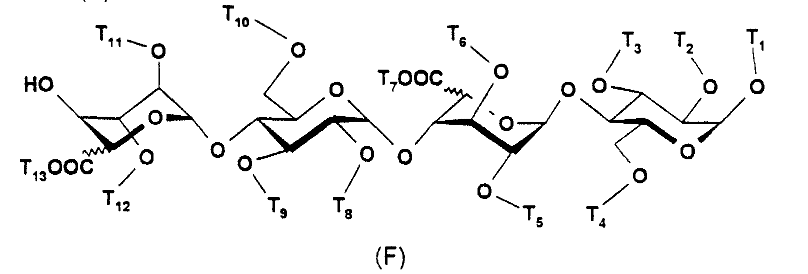 dans laquelle T₁ à T₁₃ sont tels que définis précédemment pour (D) puis,
(g) on couple le tétrasaccharide accepteur de liaison glycosidique de formule (F) et un disaccharide donneur de liaison glycosidique de formule (B) tel que ceux obtenus ci-dessus pour former un synthon saccharidique intermédiaire de type hexasaccharide complètement protégé de formule (G) : 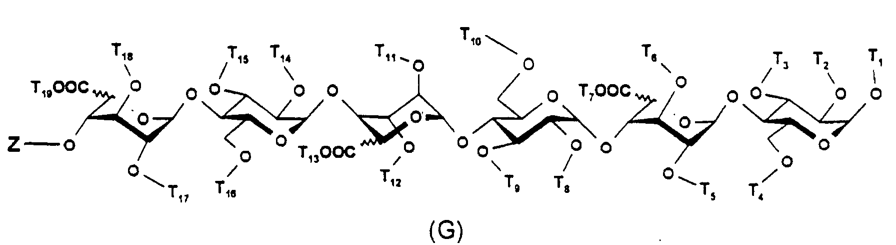 dans laquelle T₁ à T₁₃ sont tels que définis précédemment pour (D) et T₁₄ à T₁₉ sont tels que définis pour T₂ à T₇ pour (B),
   ou bien on couple le tétrasaccharide accepteur de liaison glycosidique de formule (F) et un tétrasaccharide donneur de liaison glycosidique de formule (E) de façon à obtenir un octasaccharide complètement protégé de formule (H): 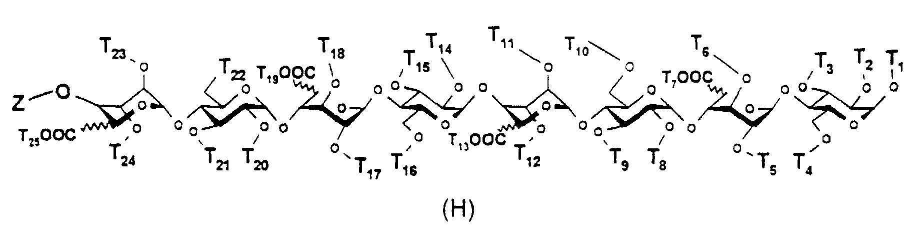 dans laquelle T₁ à T₁₉ et Z sont tels que définis précédemment et T₂₀ à T₂₅ sont tels que définis pour T₂ à T₇ pour (B) puis,
(h) on modifie chimiquement l'hexasaccharide de formule (G) ou l'octasaccharide de formule (H) obtenus ci-dessus de façon à obtenir un synthon saccharidique intermédiaire de type hexasaccharide accepteur de liaison glycosidique de formule (G) dans lequel Z représente l'hydrogène ou bien un octasaccharide accepteur de liaison glycosidique de formule (H) dans lequel Z représente l'hydrogène,
(i) on répète les étapes de déprotection et de couplage précédentes jusqu'à l'obtention de l'oligosaccharide complètement protégé possédant la structure désirée, les synthons saccharidiques intermédiaires donneurs de glycosyle et accepteur de glycosyle étant choisis en fonction de la structure finale pour obtenir ainsi le précurseur protégé du polysaccharide final désiré de formule (I), dans lequel la nature des groupes protecteurs Ti détermine la position des groupes sulfates et alkyles sur le produit final (I), et
(j) on procède à la déprotection des fonctions alcools qui doivent être sulfatées, et acide carboxyliques, en éliminant les groupes protecteurs Ti qui protégeaient ces fonctions au cours des étapes d'élaboration du squelette, puis, finalement
(k) on procède à la sulfatation pour obtenir les composés (I), ou un de leurs sels.

Le procédé décrit ci-dessus est le procédé préféré de l'invention. Toutefois, les composés de formule (I) peuvent être préparés par d'autres méthodes connues de la chimie des sucres décrites par exemple dans Monosaccharides, Their chemistry and their roles in natural products, P.M. Collins et R.J. Ferrier, J. Wiley & sons, 1995 et dans G.J. Boons, Tetrahedron, 1996, 52, 1095-1121.

En variante, les composés de formule (I) peuvent être préparés selon le procédé décrit dans M. Dreef, XVIIe Symposium sur les carbohydrates, Ottawa, 1-22 juillet 1994, Abstract D 2.5.

Par groupements semi-permanents, on entend les groupements éliminables en premier lieu après les réactions de glycosylation lorsque le squelette glucidique comporte le nombre de motifs désirés, sans enlèvement ou altération des autres groupes présents, permettant alors l'introduction de groupements fonctionnels souhaités aux positions qu'ils occupent.

Les groupements permanents sont des groupements capables de maintenir la protection des radicaux -OH durant l'introduction de groupements fonctionnels à la place des groupements semi-permanents.

Ces groupements sont choisis parmi ceux compatibles avec les groupes fonctionnels introduits après élimination des groupes semi-permanents. Il s'agit, en outre, de groupements inertes vis-à-vis des réactions effectuées pour la mise en place de ces groupes fonctionnels et qui sont éliminables sans que ces groupements fonctionnels ne soient altérés.

Selon l'invention, les groupes permanents sont les groupes alkyle en C₁-C₆.

Comme exemple de groupe semi-permanent et/ou temporaire on peut citer les groupes benzyle et acétate.

Les substituants en position 3 des motifs uroniques du composé cible peuvent être déjà présents dans les synthons de départ de formule (A), ainsi que le substituant R₁.

Dans le procédé ci-dessus, les substituants T₁, T₆, T₁₂, T₁₈ et T₂₄ ont la même définition que R₁, R₆, R₁₁ et R₁₆ dans la formule (I) c'est à dire qu'ils représentent un (C₁-C₆)alkyle.

Les groupes protecteurs utilisés dans le procédé de préparations des composés (I) sont ceux couramment utilisés dans la chimie des sucres par exemple dans Protective Groups in Organic Synthesis, TW Greene, John Wiley & sons, New-York, 1981.

Les groupes protecteurs sont avantageusement choisis par exemple parmi les groupes acétyles, halogénométhyles, benzoyles, levulinyles, benzyles, benzyles substitués, trityles éventuellement substitués, tétrahydropyranyles, allyles, pentenyles, *tert*-butyldiméthylsilyles (tBDMS) ou triméthylsilyléthyles (...).

Les groupes activateurs sont ceux classiquement utilisés en chimie des sucres selon par exemple G.J.Boons, Tetrahedron, 1996, 52, 1095-1121. Ces groupes activateurs sont choisis par exemple parmi les imidates, les thioglycosides, les penténylglycosides, les xanthates, les phosphites ou les halogénures.

Le procédé décrit ci-dessus permet d'obtenir les composés de l'invention sous forme de sels. Pour obtenir les acides correspondants, les composés de l'invention sous forme de sels, sont mis en contact avec une résine échangeuse de cations sous forme acide.

Les composés de l'invention sous forme d'acides peuvent être ensuite neutralisés par une base pour obtenir un sel souhaité.

Pour la préparation des sels des composés de formule (I), on peut utiliser toute base minérale ou organique, donnant avec les composés de formule (I), des sels pharmaceutiquement acceptables.

On utilise de manière préférentielle comme base l'hydroxyde de sodium, de potassium, de calcium ou de magnésium. Les sels de sodium et de calcium des composés de formule (I), sont les sels préférés.

Dans l'étape (a) du procédé, les groupes protecteurs utilisés sont ceux habituellement utilisés par l'homme du métier en chimie des sucres par exemple selon EP 084999 ou encore selon Protective Groups in Organic Synthesis, TW Greene, J.Wiley & sons, 1995.

Les groupes protecteurs Z, protégeant la position 4 de l'extrémité terminale non-réductrice, et T₁, protégeant la position 1 de l'unité terminale réductrice, sont éliminables de façon sélective pour permettre la fonctionalisation des positions correspondantes du disaccharide au cours des étapes suivantes de la synthèse. La nature des autres groupements protecteurs T₂ à T₈ est choisie en tenant compte des fonctions alcool devant être sulfatées dans le produit final. Une position porteuse d'un groupe alkyle dans le produit final est protégée par ce groupe dès le départ de la synthèse, alors qu'une position devant être sulfatée est protégée par un groupement protecteur temporaire tels que aralkyle (benzyle ou benzyle substitué), esters (acétates, benzoates). La position et la nature des substituants Ti déterminent donc dans le produit final le profil de sulfatation du fragment disaccharidique issu de (A). On peut obtenir une grande variété de disaccharides du type de ceux de formule (A). La préparation des disaccharides de formule (A) est réalisée essentiellement comme décrit dans la demande EP 90201006 ou encore dans la publication C.A.A. van Boeckel et M. Petitou, Angew. Chem. Int. Ed. Engl., 1993, 32, 1671-1690 ou encore selon G.J. Boons Tetrahedron, cité précédemment.

Selon l'étape (b), on peut lorsque T1 représente un alkyle, utiliser une réaction d'acétolyse dans l'anhydride acétique, qui sert alors de solvant et de réactif, en présence d'un acide fort tel que l'acide sulfurique ou l'acide trifluoroacétique pour libérer sélectivement le groupement protecteur de la position anomérique. Lorsque T1 représente un ester on peut éliminer de façon sélective cet ester en utilisant par exemple la benzylamine et un solvant aprotique tel que l'éther diéthylique ou le dichlorométhane, ou bien encore la N-méthylmorpholine dans le tétrahydrofurane. Lorsque T1 est un groupe allyle on peut l'isomériser en vinyle et procéder ensuite à une hydrolyse acide douce de l'éther vinylique par exemple en présence de chlorure mercurique dans une solution acétonique. Par les méthodes ci-dessus on obtient un composé hydroxylé qui est ensuite transformé en composé (B) par exemple au moyen d'une réaction avec le trichloroacétonitrile en présence d'une base pour obtenir dans ce cas un composé (B) dans lequel X représente un imidate. Dans le cas où X représente un thioglycoside, ou un pentényle glycoside, le groupeT1 dans le composé (A) peut être égal à X.

Selon l'étape (c), on peut obtenir un disaccharide accepteur de liaison glycosidique en éliminant sélectivement le groupement Z selon un procédé bien connu dépendant de la nature de Z, mettant par exemple en oeuvre (1) l'acétate d'hydrazine ou l'hydrate d'hydrazine, dans la pyridine ou le toluène, dans le cas où Z est un groupe lévulinique ou bien (2) la thiourée dans l'éthanol dans le cas où Z est un groupe trichloroacétyle.

Selon l'étape (d) on couple un disaccharide donneur de liaison glycosidique de formule (B) obtenu ci-dessus et un disaccharide accepteur de liaison glycosidique de formule (C) obtenu ci-dessus de façon à obtenir un tétrasaccharide complètement protégé de formule (D), on utilise pour cela un activateur connu du donneur de glycosyle X par exemple le triflate de triméthylsilyle ou TMS ou le triflate de *tert*butyldiméthylsilyle ou TBDMS ou encore le trifluorure de bore dans l'éther diéthylique par exemple lorsqu'il s'agit d'un imidate, ou bien le système N-iodosuccinimide ou NIS, acide triflique lorsqu'il s'agit d'un thioglycoside ou tout autre système connu pour activer X selon les références précédemment citées. Les solvants utilisés pour la réaction sont de façon préférée le dichlorométhane, le dichloroéthane, le chloroforme, le chlorobenzène, l'éther diéthylique, le toluène, le benzène ; on opère dans des conditions anhydre, et généralement à basse température entre -70 et 0°C. On peut également opérer à température ambiante.

Pour l'étape (e) on procède essentiellement comme décrit dans l'étape (b) à partir des tétrasaccharides obtenus selon l'étape (d).

Pour l'étape (f) on procède comme décrit en (c) pour déprotéger sélectivement les tétrasaccharides obtenus selon l'étape (d), et on obtient les tétrasaccharides de formule (F).

Pour l'étape (g) on procède comme décrit dans l'étape (d), avantageusement on sépare les produits de la réaction de formule (G) au moyen d'une chromatographie par perméation sur gel.

Pour l'étape (h) on procède essentiellement comme décrit dans l'étape (b) à partir des hexasaccharides ou des octasaccharides de formule (H) obtenus selon l'étape (g).

On répète les étapes de déprotection et de couplage précédentes jusqu'à l'obtention de l'oligosaccharide complètement protégé possédant la structure désirée, les synthons saccharidiques intermédiaires donneurs de glycosyle et accepteur de glycosyle étant choisis en fonction de la structure finale pour obtenir ainsi le précurseur protégé du polysaccharide final désiré de formule (I), dans lequel la nature des groupes protecteurs Ti. détermine la position des groupes sulfates et alkyles sur le produit final (I).

On élimine ensuite selon l'étape (j), par saponification avec de l'hydroxyde de sodium ou de l'hydroxyde de lithium et/ou par hydrogénation catalytique par exemple en présence de palladium sur charbon, les groupements protecteurs des fonctions alcools qui doivent être sulfatées.

Selon l'étape (k) on procède à la sulfatation à l'aide d'un agent sulfatant tel qu' un complexe SO₃-amine, par exemple le complexe SO₃- pyridine, ou à l'aide d'acide chlorosulfonique dans un solvant aprotique tel que le diméthylformamide de préférence à une température comprise entre 0°C et 100°C pour obtenir les composés (I) qui sont éventuellement purifiés par chromatographie par perméation sur gel en utilisant l'eau ou une solution de chlorure de sodium comme éluant.

Les composés (I) ainsi obtenus peuvent être éventuellement salifiés.

Les composés de la formule (I) ci-dessus comprennent également ceux dans lesquels un ou plusieurs atomes d'hydrogène ou de carbone ont été remplacés par leur isotope radioactif par exemple le tritium ou le carbone-14. De tels composés marqués sont utiles dans les travaux de recherche, de métabolisme ou de pharmacocinétique, dans les essais biochimiques en tant que ligands.

Les composés selon l'invention ont fait l'objet d' études biochimiques et pharmacologiques qui ont montré qu'ils possèdent des propriétés très interessantes.

Les composés de la présente invention qui se lient à l'AT III (K_{D} ≤ 200nM) ou à l'héparine co-facteur (HC II) avec une affinité égale ou supérieure à celle de l'héparine, possèdent les propriétés anticoagulantes de l'héparine.

Ils inhibent de ce fait plusieurs facteurs de la coagulation tel que le facteur Xa (titre ≥ 10u anti-Xa/mg) ou la thrombine (CI₅₀ ≤ 10 µg/ml) et sont à ce titre d'excellents agents antithrombotiques dans les modèles de thrombose veineuse et de thrombose artérielle.

L'affinité des composés de formule (I) pour l'AT III, a été déterminée par spectrofluorométrie, dans les conditions décrites par D. Atha *et al*. dans Biochemistry, 1987, 26, 6454-6461. Les résultats des essais ont démontré que les composés de l'invention possèdent une très grande affinité pour l'AT III (K_{D} compris entre 0.1 µM et 1 nM).

L'activité anti-facteur Xa (anti-Xa) des produits de l'invention a été évaluée à pH 8,4, selon la méthode décrite par Teien A.N. et Lie M., dans Thrombosis Research, 1977, *10,* 399-410, et il a été démontré que des composés de l'invention possèdent une activité anti-Xa égale ou supérieure à celle des héparinoïdes de synthèse déjà connus, notamment est supérieure à 50 u anti-Xa/mg.

Comme cela a été mentionné précédemment, dans la cascade de coagulation le facteur Xa active la prothrombine en thrombine, qui protéolyse le fibrinogène soluble avec libération de la fibrine insoluble, constituant principal du caillot sanguin. L'inhibition du facteur Xa constitue donc un moyen privilégié pour obtenir une activité anticoagulante et antithrombotique.

L'activité antithrombotique globale des produits de formule (I) a été évaluée par voie intraveineuse ou sous cutanée chez le rat, par un modèle de stase veineuse et induction par la thromboplastine, selon la méthode décrite par J. Reyers *et al*. dans Thrombosis Research, 1980, 18, 669-674. La DE₅₀ des composés de l'invention est au moins du même ordre ou inférieure à celle des autres héparinoïdes de synthèse déjà connus (DE₅₀ comprises entre 5 et 500µg/kg). Les composés de l'invention présentent donc une spécificité d'action et une activité anticoagulante et antithrombotique particulièrement intéressantes.

Les composés de la présente invention qui sont capables de moduler, notamment d'inhiber l'activité des facteurs de croissance en particulier du bFGF permettent d'inhiber la prolifération des cellules musculaires lisses vasculaires en culture (CML) avec un effet inhibiteur légèrement supérieur à celui de l'héparine, sur la croissance des cellules musculaires lisses.

L' action des composés de I' invention sur l' activité du bFGF a été évaluée par fixation du FGF iodé aux CML humaines en culture.

L'inhibition de la prolifération des CML a été évaluée *in vitro* sur des cultures de CML aortiques humaines (R. Ross, J. Cell. Biol., 1971, 172-186).

Les composés de la présente invention qui possèdent également des propriétés antivirales, hypolipidémiantes, des propriétés antiradicaux libres par libération de la superoxyde dismutase, des propriétés antimétastasiques, antiangiogéniques, anti-inflammatoires, peuvent également agir sur la croissance et la différenciation des cellules neuronales en culture. Ils sont de ce fait utilisables dans toutes les pathologies où des perturbations de ces mécanismes biologiques interviennent.

Certains composés de l'invention exercent aussi une action protectrice et régénératrice sur les fibres nerveuses.

Grâce à leur activité biochimique et pharmaceutique sélective, les composés de la présente invention sont des médicaments très interessants. Leur toxicité est parfaitement compatible avec cette utilisation. Ils sont également très stables et sont donc particulièrement appropriés pour constituer le principe actif de spécialités pharmaceutiques.

Pour leur activité sur les facteurs de la coagulation, les composés de l'invention peuvent être utilisés dans diverses pathologies liées à la coagulation et en particulier dans les troubles du système cardiovasculaire et cérébrovasculaire. Ils possèdent plus particulièrement une forte affinité pour l'antithrombine III ainsi qu' une importante activité anti-facteur Xa et antithrombine. L'inhibition de ces facteurs de la coagulation constitue donc un moyen privilègié pour obtenir une activité anticoagulante et antithrombotique.

Ils peuvent être utilisés dans diverses pathologies consécutives à une modification de I' hémostasie du système de la coagulation apparaissant en particulier lors des troubles du système cardiovasculaire et cérébrovasculaire comme les troubles thromboemboliques associés à l'arthérosclérose et au diabète tels l'angine instable, l'attaque cérébrale, la resténose après angioplastie, l'endartérectomie, la pose de prothèses endovasculaires; ou les troubles thromboemboliques associés à la rethrombose après thrombolyse, à l'infarctus, à la démence d'origine ischémique, aux maladies artérielles périphériques, à l'hémodialyse, aux fibrillations auriculaires ou encore lors de l'utilisation de prothèses vasculaires de pontages aorto-coronariens. Ces produits peuvent par ailleurs être utilisés pour le traitement ou la prévention de pathologies thrombo-emboliques d' origine veineuse telles les embolies pulmonaires. Ils peuvent être utillisés ou pour prévenir ou pour traiter les complications thrombotiques apparaissant lors d'interventions chirurgicales ou conjointement à d'autres pathologies telles que cancer, infections bactériennes ou virales. Dans le cas de leur utilisation lors de la pose de prothèses, les composés de la présente invention peuvent recouvrir des prothèses et les rendre ainsi hémocompatibles. En particulier, ils peuvent être fixés à des prothèses intravasculaires (stents). Dans ce cas, ils peuvent éventuellement être modifiés chimiquement par introduction à l'extrémité non réductrice ou réductrice d'un bras approprié, comme décrit selon EP 649 854.

L' utilisation dans la resténose après angioplastie est favorisée par les propriétés inhibitrices de certains facteurs de croissance, tels que le bFGF.

Les composés de la présente invention peuvent également être utilisés comme adjuvant lors d' endartérectomie réalisée avec des ballonets poreux.

Les résultats obtenus lors de différentes études pharmacocinétiques effectuées avec les produits de l'invention, ont démontré qu'ils sont très bien absorbés par le tractus gastro-intestinal et que leur demi-vie est longue. Cela permet d'envisager lors de leurs utilisations en thérapeutique, la possibilité d'une administration unique journalière.

Ces études ont aussi démontré que les compositions pharmaceutiques préparées avec les produits de formule (I), objet de la présente invention, sont absorbés par le tractus digestif, sans que les quantités administrées ne soient prohibitives pour une utilisation en thérapeutique humaine. Les composés de l'invention sont donc utiles pour la préparation des compositions pharmaceutiques, administrables aussi bien par voie parentérale, que par voie orale.

Les composés de l'invention sont très stables et sont donc ainsi particulièrement appropriés pour constituer le principe actif de médicaments.

Selon un autre de ses aspects, la présente invention a pour objet une composition pharmaceutique contenant, en tant que principe actif, un polysaccharide de synthèse contenant de 8 à 24 unités monosaccharidiques formé par un enchainement de disaccharides constitués d'un acide uronique et d' un hexose, ledit polysaccharide étant caractérisé en ce que tous ses groupes hydroxyles sont éthérifiés avec un groupe (C₁-C₆)alkyle ou estérifiés sous la forme de groupe sulfo, chaque dissaccharide étant au moins monoéthérifié; ou un de ses sels pharmaceutiquement acceptables. Ledit polysaccharide de synthèse, principe actif des compositions de la présente invention est de préférence alkylé par un groupe méthyle.

L'invention concerne de préférence, des compositions pharmaceutiques contenant comme principe actif, un composé de formule (I), (I.1), (I.2), (I.3) ou l'un de ses sels pharmaceutiquement acceptables, éventuellement en association avec un ou plusieurs excipients inertes et appropriés.

Dans chaque unité de dosage le principe actif est présent dans les quantités adaptées aux doses journalières envisagées. En général, chaque unité de dosage est convenablement ajustée selon le dosage et le type d' administration prévu, par exemple comprimés, gélules et similaires, sachets, ampoules, sirops et similaires, gouttes, patch transdermique ou transmucosal de façon à ce qu'une telle unité de dosage contienne de 0,1 à 100 mg de principe actif, de préférence 0,5 à 50 mg.

Les composés selon l'invention peuvent également être utilisés en association avec un autre principe actif utile pour la thérapeutique souhaitée tels que par exemple des antithrombotiques, des anticoagulants, des antiagrégants plaquettaires tels que par exemple le dipyridamole, l'aspirine, la ticlopidine, le clopidogrel ou des antagonistes du complexe de la glycoproteine IIb/IIIa.

Les compositions pharmaceutiques sont formulées pour l'administration aux mammifères, y compris l'homme, pour le traitement des maladies susdites.

Les compositions pharmaceutiques ainsi obtenues sont présentées avantageusement sous des formes diverses, telles que par exemple, des solutions injectables ou buvables, dragées, comprimés ou gélules. Les solutions injectables sont les formes pharmaceutiques préférées. Les compositions pharmaceutiques de la présente invention sont notamment utiles pour le traitement à titre préventif ou curatif, des troubles de la paroi vasculaire, tels que l'athérosclérose, les états d'hypercoagulabilité observés par exemple à la suite d'opérations chirurgicales de développement tumoraux ou de dérèglements de la coagulation, induits par des activateurs bactériens, viraux, ou enzymatiques. La posologie peut largement varier en fonction de l'âge, du poids et de l'état de santé du patient, de la nature et de la sévérité de l'affection, ainsi que de la voie d'administration. Cette posologie comprend l'administration d'une ou plusieurs doses d'environ 0,1 mg à 100 mg par jour, de préférence d'environ 0,5 à 50 mg par jour, par voie intramusculaire ou sous cutanée, en administrations discontinues ou à intervalles réguliers.

La présente invention a donc également pour objet les compositions pharmaceutiques qui contiennent à titre de principe actif un des composés ci-dessus éventuellement en association avec un autre principe actif. Ces compositions sont réalisées de façon à pouvoir être administrées par la voie digestive ou parentérale.

Dans les compositions pharmaceutiques de la présente invention pour l'administration orale, sublinguale, sous-cutanée, intramusculaire, intraveineuse, transdermique, transmucosale, locale ou rectale, l'ingrédient actif peut être administré sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol ou le butylèneglycol.

Pour une administration transmucosale le principe actif peut être formulé en présence d'un promoteur tel qu'un sel biliaire, d'un polymère hydrophile tel que par exemple l'hydroxypropylcellulose, l'hydroxypropylméthylcellulose, l'hydroxyéthylcellulose, l'éthylcellulose, la carboxyméthylcellulose, le dextran, la polyvinylpyrrolidone, les pectines, les amidons, la gelatine, la caséine, les acides acryliques, les esters acryliques et leurs copolymères, les polymères ou copolymères de vinyle, les alcools vinyliques, les alcoxypolymères, les polymères d'oxyde de polyéthylène, les polyéthers ou leur mélange.

Le principe actif peut être formulé également sous forme de microcapsules, éventuellement avec un ou plusieurs supports ou additifs.

Le principe actif peut être également présenté sous forme de complexe avec une cyclodextrine, par exemple α, β ou y cyclodextrine, 2-hydroxypropyl-β-cyclodextrine ou méthyl-β-cyclodextrine.

Le principe actif peut également être libéré par un balonnet le contenant ou par un extenseur endovasculaire introduit dans les vaisseaux sanguins. L' efficacité pharmacologique du principe actif n'est ainsi pas affectée.

L'administration par voie sous-cutanée est la voie préférée.

Les METHODES, les PREPARATIONS et les SCHEMAS suivants illustrent la synthèse des différents intermédiaires utiles à l'obtention des polysaccharides selon l'invention.

Les EXEMPLES ci-après illustrent également l'invention sans toutefois la limiter.

Pour une meilleure compréhension du procédé selon l'invention, l'obtention des composés (I) peut être schématisé comme suit :

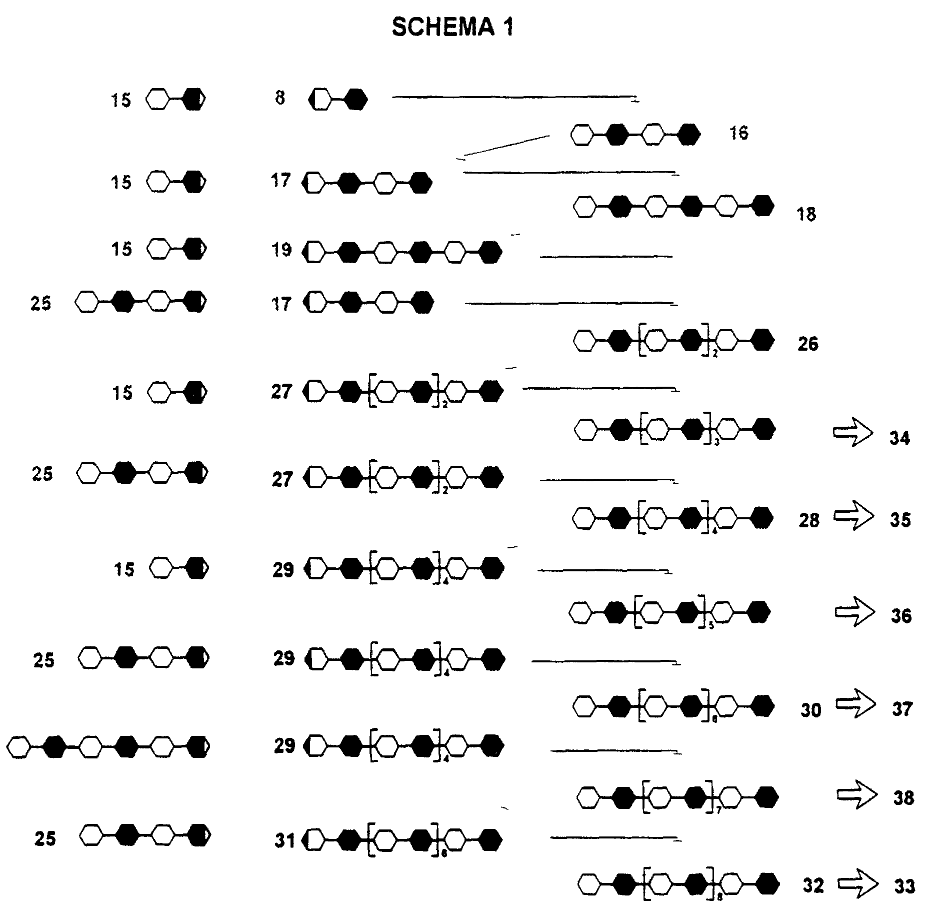

*Stratégie employée pour la synthèse d'oligomères du disaccharide méthyl 4-O-(2,3-di-O-méthyl-α-L-idopyranosyluronate)-2,3,6-tri-O-sulfo-α-D-glucopyranoside. En condensant les imidates (colonne de gauche, triangle blanc à l'extrémité réductrice) et les accepteurs de glycosyle (colonne du centre, triangle noir à l'extrémité non-réductrice) on obtient les oligosaccharides complètement protégés de la colonne de droite. Ces derniers sont ensuite déprotégés et fonctionnalisés pour livrer les composés de l'EXEMPLE 1 et du TABLEAU I*.

Tous les composés décrits ci-dessous sont homogènes en chromatographie sur couche mince (CCM) et ont des propriétés spectrales en accord avec leur structure. Les points de fusion sont déterminés dans des tubes capillaires à l'aide d'un appareil Mettler, et ne sont pas corrigés. Les pouvoirs rotatoires sont mesurés à l'aide d'un polarimètre Perkin-Elmer 241 à 22 ± 3 °C. La pureté des composés est vérifiée par CCM sur Silica Gel 60® F₂₅₄ (E. Merck) avec détection par carbonisation en présence d'acide sulfurique. Sauf mention particulière les chromatographie sur colonne sont réalisées sur Silica Gel 60® , 40-63 or 63-200 µm (E. Merck). Les spectres de ¹H RMN sont enregistrés sur des appareils Bruker AC 200, AM 250, AC 300 ou AM500, sur des solutions des produits dans CDCl₃ ou D₂O. Avant analyse dans D20 les échantillons sont passés au travers d'une colonne de résine échangeuse d'ions Chelex® (Bio-Rad) puis lyophilisés trois fois dans D₂O. Les déplacements chimiques sont relatifs au TMS externe quand les spectres sont enregistrés dans CDCl₃, et au TSP externe quand les spectres sont enregistrés dans D₂O. Les analyses de spectrometrie de masse sont effectuées sur un instrument ZAB-2E (Fisons). Les analyses élémentaires sont effectuées sur un analyseur Fisons.

Les abréviations suivantes sont utilisées :
TBDMS : *tert*-butyldiméthylsilyle; Lev : levulinyle; Bn : benzyle; Bz : benzoyle; MCA : chloroacétyle; CCM : chromatographie sur couche mince; Olm : trichloroacétimidyle; LSIMS : est le sigle anglais de *Liquid Secondary Ion Mass Spectrometry*, ESIMS : est le sigle anglais de *Electron Spray Ionisation Mass Spectrometry*; TMS : triméthylsilyle; TSP : triméthylsilyle tétradeuterio propionate de sodium; Tf : triflate; MS : tamis moléculaire.
Dowex® , Sephadex® , Chelex® , Gel 60® sont des marques déposées.

Dans les METHODES, les PREPARATIONS et dans les EXEMPLES décrits ci-après, des modes opératoires généraux concernant le clivage des esters levuliniques, le couplage catalytique des imidates, la déprotection et la sulfatation des oligo et des polysaccharides par hydrogénolyse des esters ou des éthers benzyliques, la saponification des esters ou encore les sulfatations peuvent êtres réalisés en appliquant les méthodes générales ci-après aux intermédiaires appropriés.

### METHODES GENERALES

### METHODE 1. Coupure du groupe Lev.

Une solution d'hydrate d'hydrazine (1 M dans 3:2 pyridine/acide acétique) est ajoutée (5 ml/mmole) à une solution refroidie (0 °C) dans la pyridine (5 ml/mmole) du composé à traiter. Après 15-30 minutes (CCM) la solution est concentrée. Le résidu est dissous dans l'acétate d'éthyle, lavé avec de l'eau, une solution à 10% d'hydrogénosulfate de potassium, une solution à 2% d'hydrogénocarbonate de sodium, à l'eau, séché (sulfate de sodium), et concentré.

### METHODE 2. Couplage aux imidates catalysé par le triflate de tertbutyldiméthylsilyle.

Du triflate de *tert*-butyldiméthylsilyle (0,5 mol/mol d'imidate) est ajouté goutte à goutte, sous argon, à une solution agitée et refroidie (-20 °C) de l'alcool accepteur et de l'imidate donneur, dans le toluène (35 ml/mmol), en présence de tamis moléculaire 4 Å en poudre. Après 15-30 minutes (CCM), on introduit sous agitation de l'hydrogénocarbonate de sodium solide. Après 5 minutes du toluène est ajouté, la solution est filtrée, lavée avec une solution à 2% d'hydrogénocarbonate de sodium, à l'eau, séchée (sulfate de sodium), et concentrée.

### METHODE 3. Couplage aux imidates catalysé par le triflate de triméthylsilyle

Du triflate de triméthylsilyle (0,04 M dans le toluène; 0,06 mol/mol d'imidate) est ajouté goutte à goutte, sous argon, à une solution agitée et refroidie (-20 °C) de l'alcool accepteur et de l'imidate donneur, dans le toluène (15 ml/mmol), en présence de tamis moléculaire 4 Å en poudre. Après 15-30 minutes (CCM), on introduit sous agitation de l'hydrogénocarbonate de sodium solide. Après 5 minutes du toluène est ajouté, la solution est filtrée, lavée avec une solution à 2% d'hydrogénocarbonate de sodium, à l'eau, séchée (sulfate de magésium), et concentrée

### METHODE 4. Déprotection et sulfatation des oligo- et polysaccharides.

Hydrogénolyse des benzyl éthers et benzyl esters. Une solution du composé (5 mg/ml), dans le diméthylformamide ou le méthanol, est agitée pendant 2-6 heures (contrôle CCM) sous une atmosphère d'hydrogène (5 bar) en présence de catalyseur Pd/C 10% (2 x masse du composé). Après filtration le produit est engagé directement dans l'étape suivante.

Saponification des esters. Une solution aqueuse d'hydroxyde de sodium 5 M est ajoutée (en quantité telle que la concentration d'hydroxyde de sodium soit de 0,5 M en fin d'addition) à une solution d'un ester dans le méthanol (150 ml/mmol). Après 2-5 heures (CCM) de l'eau est introduite suivie par de la résine Dowex® 50 H⁺ jusqu'à pH 1-2. Après filtration et concentration le résidu est passé au travers d'une colonne de gel Sephadex® G-25 (1,6 × 115 cm) éluée par l'eau. Le composé complètement déprotégé est alors obtenu après lyophilisation. A ce stade on vérifie par ¹H RMN que tous les groupes protecteurs ont été enlevés. Si cela est nécessaire le produit est de nouveau soumis à l'hydrogénation et/ou la saponification.

Sulfatation. Du complexe pyridine/trioxyde de soufre (5 mmol/mmol fonction hydroxyle) est ajouté à une solution dans le diméthylformamide (10 mg/ml) du composé à sulfater. Après un jour à 55 °C la solution est déposée au sommet d'une colonne de Sephadex® G-25 (1,6 × 115 cm) éluée par du chlorure de sodium 0,2 M. Les fractions contenant le produit sont concentrées et dessalées en utilisant la même colonne éluée par l'eau. Le composé final est obtenu après lyophilisation.

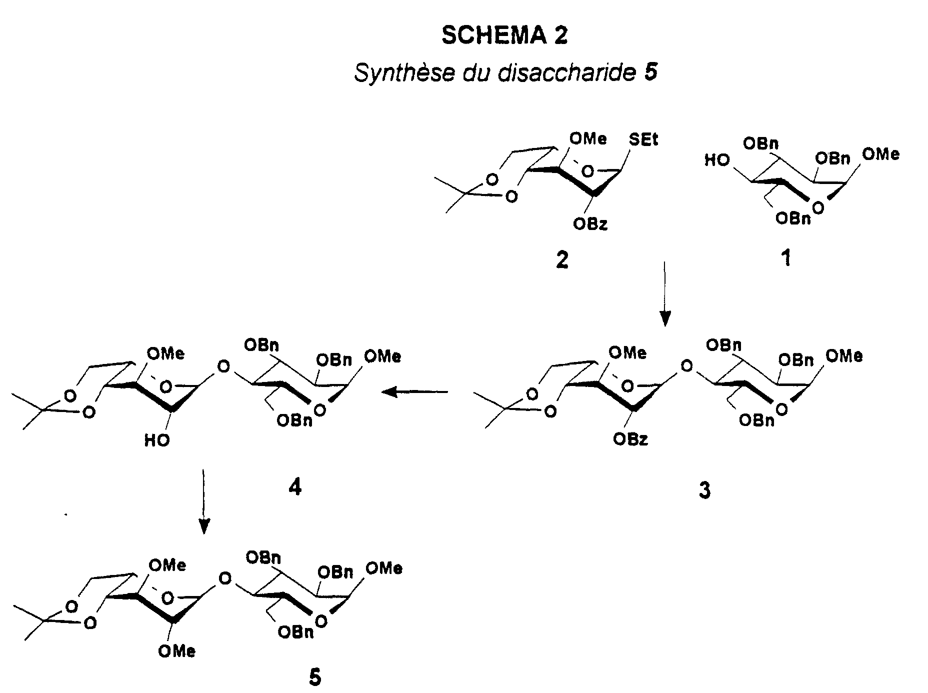

### PREPARATION 1

### Méthyl 4-O-(2-O-benzoyl-4,6-isopropylidène-3-O-méthyl-α-L-idopyranosyl)-2,3,6-tri-O-benzyl-α-D-glucopyranoside (3).

Une solution d'acide triflique dans le toluène (0,15 M, 0,27 ml) est ajoutée, sous agitation sous argon, à une solution refroidie (-20 °C) d'éthyl 2-O-benzoyl-4,6-O-isopropylidène-3-O-méthyl-1-thio-α-L-idopyranoside **2** (Jaurand,G. *et al*., BioMed. Chem. Lett. 1992, 2, 897-900). (1,1 g, 2,87 mmol), de **1** (Garegg P.J., Hultberg H., Carbohydr. Res. 93, 1981 C10-C11) (1,34 g, 2,87 mmol), et de *N*-iodosuccinimide (1,61 g, 7,2 mmol), dans le toluène (40 ml) contenant des tamis moléculaires 4 Å en poudre. La même quantité d'acide est ajoutée après 25 et 50 minutes. Après 1,5 h, du bicarbonate de sodium solide (20 mg) est introduit, et, 15 minutes plus tard la solution est filtrée, diluée avec du dichlorométhane, lavée avec une solution de thiosulfate de sodium, de l'eau, séchée (sulfate de sodium) et évaporée. Le produit brut ainsi obtenu (2,49 g) est utilisé directement pour la préparation de **4**.

Après chromatographie sur colonne (3:1 cyclohexane/acétate d'éthyle) on obtient le composé 3 pur. CCM, R_{F} = 0,36, 3:1 cyclohexane/acétate d'éthyle; [α]_{D} + 31 (c = 1, dichlorométhane). ESI SM, mode positif: m/z + NaCl, 345 (M+Na)⁺; + KF, 361 (M+K)⁺. ¹H RMN (CDCl₃) δ 7,17-7,35 (m, 20H, 4Ph), 5,10 (d, 1H, H-1'), 4,60 (d, 1H, *J* = 3,0 Hz, H-1), 3,37 (s, 3H, OMe), 1,95; 2,04; 2,09 (3s, 9H, 3Ac), 1,24; 1,33 (2s, 6H, :C(C*H*₃)₂).
Anal. Calculé pour C₄₅H₅₂O₁₂ (784,86) : C, 68,86; H, 6,68. Trouvé : C, 68,61; H, 6,77.

### PREPARATION 2

### Méthyl 2,3,6-tri-O-benzyl-4-O-(4,6-isopropylidène-3-O-méthyl-α-L-idopyranosyl)-α-D-glucopyranoside (4).

Une solution 2 M de méthylate de sodium (2,2 ml, 4,4 mmol) est ajoutée à une solution du composé 3 (2,34 g) dans un mélange 1:1 méthanol/dichlorométhane (13 ml). Après 2,5 heures à température ambiante le mélange est neutralisé avec de la résine Dowex® 50 (H⁺), filtré et concentré, pour donner **4** (1,74 g; 86% par rapport à **1** et **2**) après chromatographie sur colonne (3:1 puis 2:1 cyclohexane/acétate d'éthyle); [α ]_{D} + 23 (c = 1, dichlorométhane). ESI SM, mode positif: m/z + NaCl, 703 (M+Na)⁺; + KF, 719 (M+K)⁺. ¹H RMN (CDCl₃) δ 7,31-7,21 (m, 15H, 3Ph), 4,94 (d, 1H, H-1'),
4,60 (d, 1H, *J* = 3,6 Hz, H-1), 3,44; 3,36 (2s, 6H, OMe); 3,06 (dd, 1H, *J* = 3,6 Hz, *J* = 12,2 Hz, H-6'), 1,31; 1,28 (2s, 6H, :C(C*H*₃)₂).
Anal. Calculé pour C₃₈H₄₈O₁₁ (680,76) : C, 67,04; H, 7,11. Trouvé : C, 67,05; H, 7,16.

### PREPARATION 3

### Méthyl 2,3,6-tri-O-benzyl-4-O-(4,6-isopropylidène-2,3-di-O-méthyl-α-L-idopyranosyl)-α-D-glucopyranoside (5).

De l'iodure de méthyle (3,2 ml, 50,8 mmol) est ajouté, à 0 °C, à une solution de **4** (26,6 g, 39,1 mmol) et d'hydrure de sodium (1,48 g, 58,7 mmol) dans le diméthylformamide (60 ml). Une nouvelle addition d'iodure de méthyle (1,6 ml, 25,4 mmol) et d'hydrure de sodium (0,74 g, 29,3 mmol) est réalisée après 5 heures à température ambiante. Après 1 nuit, du méthanol (10 ml) est introduit goutte à goutte, et après 1,5 heure le mélange réactionnel est concentré. Le produit est extrait avec de l'acétate d'éthyle (1,5 I). La solution est lavée avec de l'eau, séchée (sulfate de sodium) et concentrée. Le composé brut **5,** ainsi obtenu (32,1 g), est utilisé tel quel dans l'étape suivante. CCM, R_{F} = 0,55, 3:2 cyclohexane-acétate d'éthyle.

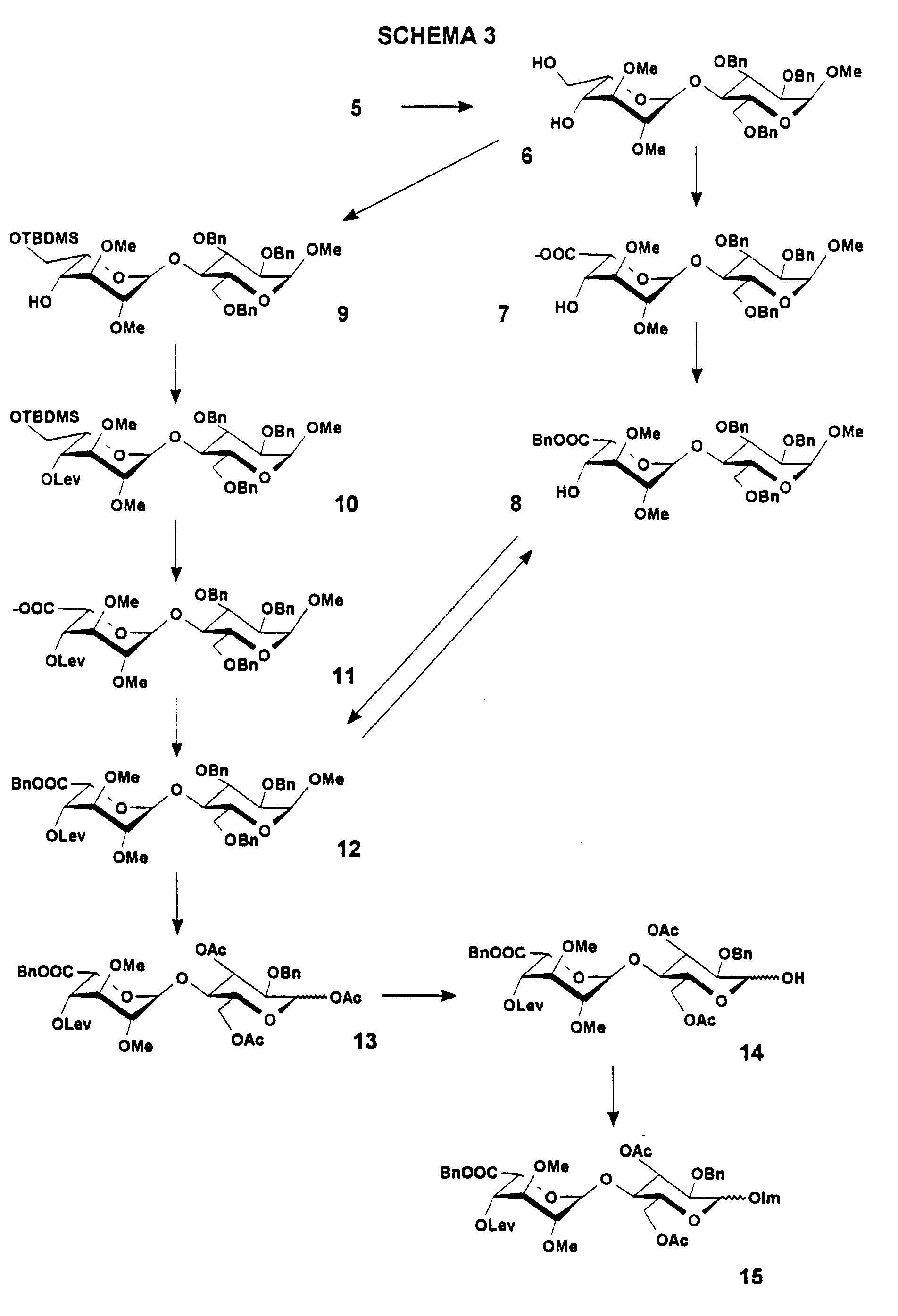

### Synthèse des disaccharides de base pour la préparation d'oligomères du méthyl 4-O-(2,3-di-O-méthyl-α-L-idopyranosyluronate)-2,3,6-tri-O-sulfo-α-D-glucopyranoside.

### PREPARATION 4

### Méthyl 2,3,6-tri-O-benzyl-4-O-(2,3-di-O-méthyl-α-L-idopyranosyl)-α-D-glucopyranoside (6).

Une solution aqueuse d'acide trifluoroacétique (70%, 43 ml) est ajoutée goutte à goutte pendant 10 minutes à une solution du composé brut ci-dessus (32,1 g) dans du dichlorométhane (215 ml). Après 25 minutes à température ambiante la solution est diluée avec du dichlorométhane (11), lavée avec une solution aqueuse saturée d'hydrogénocarbonate de sodium, avec de l'eau et séchée (sulfate de sodium). Le composé brut **6** obtenu après concentration (27,5 g) est utilisé tel quel dans l'étape suivante. CCM, R_{F} = 0,29, 2:3 cyclohexane-acétate d'éthyle.

### PREPARATION 5

### Méthyl 2,3,6-tri-O-benzyl-4-O-(6-O-tert-butyldiméthylsilyl-4-O-lévulinyl-2,3-di-O-méthyl-α-L-idopyranosyl)-α-D-glucopyranoside (10).

Une solution de **6** (1,7 g), de triéthylamine (0,54 ml, 3,8 mmol), de 4-diméthylaminopyridine (38 mg, 0,3 mmol) et de chlorure de *tert*-butyldiméthylsilyle (0,54 g, 3,6 mmol), dans le chlorure de méthylène (6 ml), est chauffée à 50° C pendant 3 heures pour donner **9** qui n'es pas isolé. Après refroidissement à température ambiante, de l'anhydride lévulinique (0,771 g, 3,6 mmol), de la triéthylamine (0,50 ml, 3,6 mmol) et de la 4-diméthylaminopyridine (59 mg, 0,48 mmol) sont ajoutés. Après 4 heures le mélange est dilué avec du chlorure de méthylène, et lavé successivement avec une solution aqueuse d'hydrogénosulfate de potassium, de l'eau, une solution aqueuse saturée d'hydrogénocarbonate de sodium, de l'eau, séché (sulfate de sodium), et concentré pour donner **10** (2,45 g) qui est utilisé tel quel dans l'étape suivante. TLC, R_{F} 0,5, 12:1 cyclohexane/acétate d'éthyle.

### PREPARATION 6

### Méthyl 2,3,6-tri-O-benzyl-4-O-(benzyl 4-O-levulinyl-2,3-di-O-méthyl-α-L-idopyranosyluronate)-α-D-glucopyranoside (12).

Une solution de trioxyde de chrome (0,64 g, 6,4 mmol) dans l'acide sulfurique aqueux (3,5 M, 2,7 ml) est ajoutée lentement à une solution refroidie (0° C) de **10** (2,45 g) dans l'acétone (18 ml). Après 5 heures du chlorure de méthylène est introduit, puis le mélange est versé dans de l'eau galcée, agité vigoureusement, lavé à l'eau jusqu'à un pH neutre et séché (sulfate de sodium). La concentration donne **11** (2,45 g), sous forme de sirop. TLC, R_{F} 0,56, 12:1 chlorure de méthylène/méthanol, Ce produit est ensuite dissous dans le diméthylformamide (19 ml) et traité une nuit à température ambiante avec du bromure de benzyle (2,9 ml, 12 mmol). Du méthanol (1,5 ml) est ajouté et le produit est extrait avec de l'éther, lavé à l'eau, séché et concentré. Après chromatographie sur colonne (2:1 puis 3:2 cyclohexane/acétate d'éthyle) on obtient **12** (1,07 g). TLC, R_{F} 0,53, 5:1 chlorure de méthylène-acétate d'éthyle.

### PREPARATION 7

### Méthyl 2,3,6-tri-O-benzyl-4-O-(benzyl 2,3-di-O-méthyl-α-L-idopyranosyluronate)-α-D-glucopyranoside (8).

Le disaccharide **12** (0,65 g, 0,76 mmol) traité selon la méthode générale 1 donne **8** (0,52 g, 91 %) après chromatographie sur colonne (2:1 puis 3:1 cyclohexane/acétate d'éthyle). [α]_{D} +34 (c = 0,97, chlorure de méthylène).

### PREPARATION 8

### 1,3,6-tri-O-Acétyl-2-O-benzyl-4-O-(4-O-lévulinyl-2,3-di-O-méthyl-α-L-idopyranosyluronate de benzyle)-D-glucopyranose (13).

De l'acide trifluoroacétique (28 ml, 0,364 mol) est ajouté à une solution de **12** (7,8 g, 9,1 mmol) dans de l'anhydride acétique (194 ml, 2,06 mol) et de l'acide acétique (7,8 ml, 0,136 mol). Après chauffage à 60 °C pendant 4 heures la solution est refroidie à 0 °C et de l'eau (30 ml) est introduite goutte à goutte, suivie de triéthylamine (69 ml). Après evaporation le résidu est dissous dans dichlorométhane, lavé avec une solution saturée d'hydrogénocarbonate de sodium, de l'eau, séché (sulfate de sodium), et concentré. Une chromatographie sur colonne (5:1 dichlorométhane/acétate d'éthyle) donne un mélange (α/β=8/2) des anomères de **13** (4,7 g, 67%). CCM, R_{F} 0,35; 3:2 cyclohexane-acétone. ¹H RMN (CDCl₃) δ 7,37-7,20 (m, 10H, 2Ph), 6,30 (d, J = 3,6 Hz, H-1α), 5,62 (d, J = 7,6 Hz, H-1β), 5,04 (t, 1H, H-4'), 3,45; 3,41 (2s, 6H, 2 OMe), 2,6-2,3 (m, 4H, O(C:O)C*H*₂C*H*₂(C:O)CH₃), 2,15; 2,12; 2,06; 1,94; 1,88 (5s, 12H, 3 Ac et O(C:O)CH₂CH₂(C:O)C*H*₃).

### PREPARATION 9

### 3,6-di-O-Acétyl-2-O-benzyl-4-O-(4-O-lévulinyl-2,3-di-O-méthyl-α-L-ido pyranosyluronate de benzyle)-D-glucopyranose (14).

Une solution d'éthanolamine (1,3 ml, 21,6 mmol) et de **13** (4,25 g, 5,28 mmol) dans le tétrahydrofurane (80 ml) est abandonnée une nuit à 4 °C. De l'éthanolamine (0,65 ml, 10,8 mmol) est alors ajoutée, puis le mélange est laissé à température ambiante pendant 3 h. Après refroidissement à 0 °C, on ajoute de l'acide chlorhydrique 1 M jusqu'à pH acide, puis du dichlorométhane (150 ml). La solution est lavée avec de l'eau, séchée, et concentrée. Une chromatographie sur colonne (2:1 puis 1:1 dichlorométhane/acétate d'éthyle) donne **14** (3 g, 74%). CCM, R_{F} 0,21, 1:1 toluène-acétate d'éthyle. [α]_{D} +3 (c = 1, dichlorométhane). LSIMS, mode positif: m/z thioglycérol + NaCI, 769 (M+Na)⁺; thioglycérol + KF, 785 (M+K)⁺. ¹H RMN (CDCl₃) δ 7,36-7,25 (m, 10H, 2Ph), 5,21 (d, J = 3,5 Hz, H-1α), 4,98 (d, 1H, J = 3,4 Hz, H-1'), 4,79 (d, J = 8 Hz, H-1β), 3,45; 3,42 (2s, 6H, 2 OMe), 2,56-2,23 (m, 4H, O(C:O)C*H*₂C*H*₂(C:O)CH₃), 2,23; 2,12; 1,94; 1,88 (4s, 9H, 2 Ac, α et β O(C:O)CH₂CH₂(C:O)C*H*₃).
Anal. Calculé pour C₃₇H₄₆O₁₆ (746,72): C, 59,51; H, 6,21. Trouvé: C, 58,87, H, 6,13.

### PREPARATION 10

### 3,6-di-O-acétyl-2-O-benzyl-4-O-(4-O-lévulinyl-2,3-di-O-méthyl-α-L-idopyranosyluronate de benzyle)-D-glucopyranose trichloroacétimidate (15).

Un mélange de trichloroacetonitrile (0,7 ml; 6,92 mmol), de **14** (1,03 g; 1,38 mmol), et de carbonate de potassium (191 mg; 2,21 mmol), dans le chlorure de méthylène (26 ml) est agité pendant 1,5 heure à température ambiante. La solution est alors filtrée et concentrée. Une chromatographie sur colonne (4:1 toluène/acétone) donne **15** (1.16 g; 94%). CCM, R_{F} 0,31 et 0,48, 2:3 cyclohexane-acétate d'éthyle. LSIMS, mode positif: m/z thioglycérol + LiCI, 896 (M+Li)⁺; thioglycérol + NaCI, 912 (M+Na)⁺; thioglycérol + KF, 928 (M+K)⁺. ¹H RMN (CDCl₃) δ 8,67 (s, NH-β), 8,60 (s, NH-α), 7,37-7,22 (m, 10H, 2Ph); 6,44 (d, J = 3,6 Hz, H-1α), 5,83 (d, J = 7,3 Hz, H-1β), 3,47; 3,44; 3,42; 3,40 (4s, 6H, 2 OMe), 2,7-2,2 (m, 4H, O(C:O)C*H*₂C*H*₂(C:O)CH₃), 2,15; 2,08; 1,94; 1,88 (4s, 9H, α et β Ac, et O(C:O)CH₂CH₂(C:O)C*H*₃).

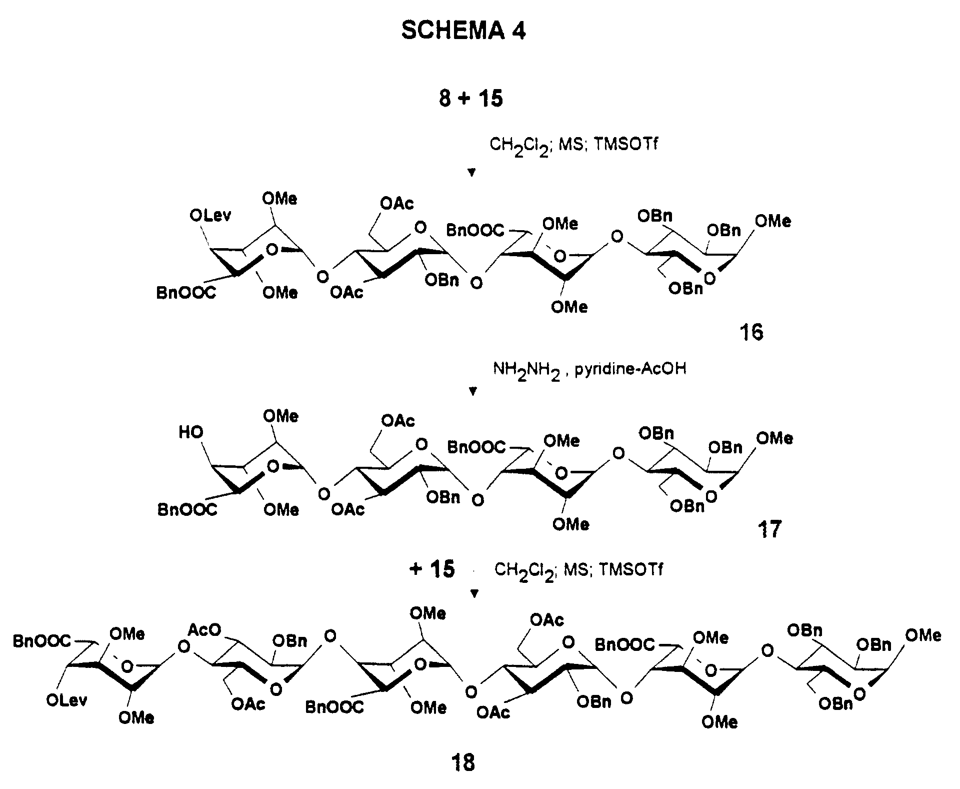

*Synthèse de l'hexasaccharide* ***18****. La préparation d'oligomères de taille supérieure est effectuée selon la même stratégie (coupure du groupe Lev pour obtenir un accepteur de glycosyle, couplage avec un di-, tetra-, ou hexasaccharide imidate -comme indiqué sur le schéma 1- et finalement, déprotection et sulfatation). Le groupe Lev de* ***18*** est *sélectivement éliminé pour donner l'hexasaccharide accepteur* ***19*** *(SCHEMA 1)*.

### PREPARATION 11

### Méthyl O-(4-O-lévulinyl-2,3-di-O-méthyl-α-L-idopyranosyluronate de benzyle)-(1-4)-O-(3,6-di-O-acétyl-2-O-benzyl-α-D-glucopyranosyl)-(1-4)-O-(2,3-di-O-méthyl-α-L-idopyranosyluronate de benzyle)-(1-4)-2,3,6-tri-O-benzyl-α-D-glucopyranoside (16).

Un mélange de **8** (2,90 g, 3,83 mmol) et **15** (4,16 g, 4,67 mmol) est traité selon la méthode 2. Une chromatographie sur colonne (1:1 cyclohexane/acétate d'éthyle) donne **16** pur ( 3,2 g; 54%). CCM, R_{F} 0,52, 2:3 cyclohexane/acétate d'éthyle. ¹H RMN (CDCl₃) δ 7,21-7,36 (m, 30H, 6Ph), 5,27 (d, 1H, H-1, unité non réductrice), 5,14 (d, 1H, H-1 unité "central next to non reducing"), 4,90 (d, 1H, H-1, unité "central next to reducing"), 4,56 (d, 1H, H-1 unité réductrice), 3,43; 3,39; 3,35; 3,25 (5s, 15H, 5 OMe), 2,25-2,60 (m, 4H, O(C:O)C*H*₂C*H*₂(C:O)CH₃), 2,12; 2,00; 1,91 (3s, 9H, 2Ac et O(C:O)CH₂CH₂(C:O)C*H*₃).

### PREPARATION 12

### Méthyl O-(2,3-di-O-méthyl-α-L-idopyranosyluronate de benzyle)-(1-4)-O-(3,6-di-O-acétyl-2-O-benzyl-α-D-glucopyranosyl)-(1-4)-O-(2,3-di-O-méthyl-α-L-idopyranosyluronate de benzyle)-(1-4)-2,3,6-tri-O-benzyl-α-D-glucopyranoside (17).

Le composé **16** (1 g; 0,672 mmol) est traité selon la méthode 1 pour donner quantitativement **17** après chromatographie sur colonne (1:1 cyclohexane/acétone).

### PREPARATION 13

### Méthyl O-(4-O-lévulinyl-2,3-di-O-méthyl-α-L-idopyranosyluronate de benzyle)-(1-4)-[O-(3,6-di-O-acétyl-2-O-benzyl-α-D-glucopyranosyl)-(1-4)-O-(2,3-di-O-méthyl-α-L-idopyranosyluronate de benzyle)-(1-4)]₂-2,3,6-tri-O-benzyl-α-D-glucopyranoside (18).

Un mélange de **15** (386 mg, 434 µmol) et **17** (500 mg; 360 µmol) est traité selon la méthode 2. Une chromatographie sur colonne (Sephadex® LH 20, 195 x 3,7 cm; 1:1 dichlorométhane/éthanol) donne l'hexasaccharide **18** ( 495 mg; 64%). CCM, R_{F} 0,36, 10:1 dichlorométhane/acétone.
¹H RMN (CDCl₃) δ: 5,23; 5,12; 5,10; 4,92; 4,89; 4,56 ppm.

### PREPARATION 14

### Méthyl O-(2,3-di-O-méthyl-α-L-idopyranosyluronate de benzyle)-(1-4)-[O-(3,6-di-O-acétyl-2-O-benzyl-α-D-glucopyranosyl)-(1-4)-O-(2,3-di-O-méthyl-α-L-idopyranosyluronate de benzyle)-(1-4)]₂-2,3,6-tri-O-benzyl-α-D-glucopyranoside (19).

Le composé **18** (485 mg; 229 µmol) est traité selon la méthode 1. Une chromatographie sur colonne (10:1 dichlorométhane/acétone) donne **19** (392 mg; 85%). CCM, R_{F} 0,38, 10:1 dichlorométhane/acétone.

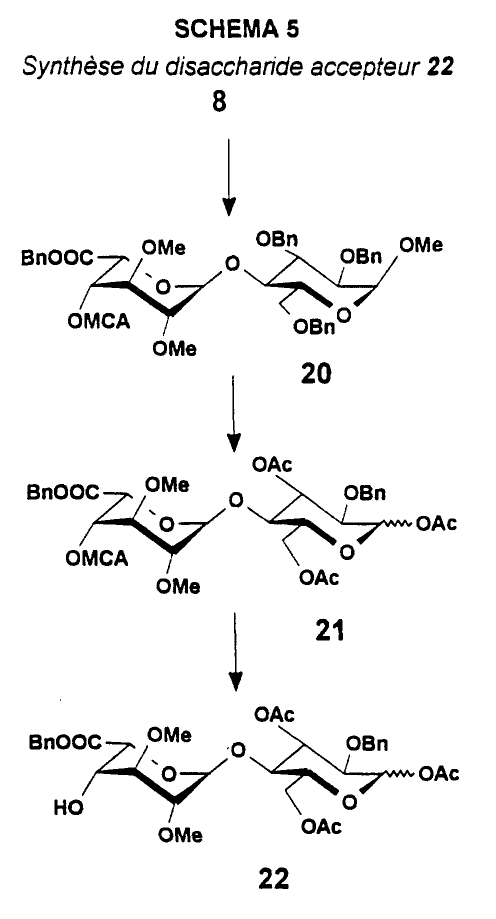

### PREPARATION 15

### Méthyl 2,3,6-tri-O-benzyl-4-O-(4-O-chloroacétyl-2,3-di-O-méthyl-α-L-idopyranosyluronate de benzyle)-α-D-glucopyranoside (20).

Un mélange de **8** (326 mg, 0,43 mmol), d'anhydride chloroacétique (103 g, 0,6 mmol), de 4-diméthylaminopyridine (5,3 mg, 42 µmol), et de triéthylamine (90 µl, 64 µmol) dans le dichlorométhane (96 ml) est agitée à température ambiante pendant 30 minutes. Du méthanol (0,5 ml) est alors ajouté, la solution est diluée avec du dichlorométhane, lavée à l'eau, séchée (sulfate de sodium), et concentrée. Une chromatographie sur colonne (2:3 puis 1:2 cyclohexane/éther) donne **20** pur (242 mg, 67%). CCM, R_{F} 0,35, 1:2 cyclohexane/éther. ¹H RMN (CDCl₃) δ 7,36-7,19 (m, 20H, 4Ph), 5,20 (d, 1H, H-1'), 4,56 (d, 1H, H-1), 3,70 et 3,36 (système AB, J = 15,3 Hz, ClC*H*₂(C:O)O), 3,49, 3,35; 3,26 (3s, 3 OCH₃).

### PREPARATION 16

### 1,3,6-tri-O-acétyl-2-O-benzyl-4-O-(4-O-chloroacétyl-2,3-di-O-méthyl-α-L-idopyranosyluronate de benzyle)-α,β-D-glucopyranose (21).

Une solution d'acide trifluoroacétique (183 µl, 2,4 mmol) est ajoutée à une solution de **20** (50 mg, 0,06 mmol) dans l'anhydride acétique (1,28 ml, 13,5 mmol) et l'acide acétique (52 µl, 0,9 mol). Après chauffage à 60 °C pendant 4 heures la solution est refroidie à 0 °C et neutralisée avec de la triéthylamine. Après évaporation, une chromatographie sur colonne du résidu (1:2 puis 2:5 cyclohexane/éther) donne un mélange (α/β = 8/2) des anomères de **21** (28 mg, 60%). CCM, R_{F} 0,31, 2:5 cyclohexane/éther. ¹H RMN (CDCl₃) δ 7,20-7,35 (m, 10H, 2Ph), 6,30 (d, J = 3,6 Hz, H-1α), 5,63 (d, J = 8,1 Hz, H-1β), 3,39 et 3,46 (2s, 2 OCH₃)

### PREPARATION 17

### 1,3,6-tri-O-acétyl-2-O-benzyl-4-O-(2,3-di-O-méthyl-α-L-idopyranosyluronate de benzyle)-α,β-D-glucopyranose (22).

De la thiourée (678 mg; 8,9 mmol) est ajoutée à une solution de **21** (1,71 g; 2,23 mmol) dans un mélange de pyridine (108 ml) et d'éthanol (22 ml), et le mélange est chauffé à 110 °C pendant 30 minutes. Après refroidissement et évaporation le résidu est dissous dans du dichlorométhane. La solution est lavée à l'aide d'une solution saturée aqueuse d'hydrogénocarbonate de sodium, puis une solution à 5% d'hydrogénosulfate de potassium, séchée (sulfate de sodium), et concentrée. Une chromatographie sur colonne (1:1, puis 1:2 cyclohexane/acétate d'éthyle) donne **22** pur (1,17 g; 76%). CCM, R_{F} 0,34, 3:1 dichlorométhane/éther. ¹H RMN (CDCl₃) δ 7,36-7,20 (m, 10H, 2Ph), 6,30 (d, J = 3,6 Hz, H-1α), 5,65 (d, J = 8 Hz, H-1β), 4,90 (1H, H-1'), 3,41 et 3,40 (2s, 2 OCH₃)

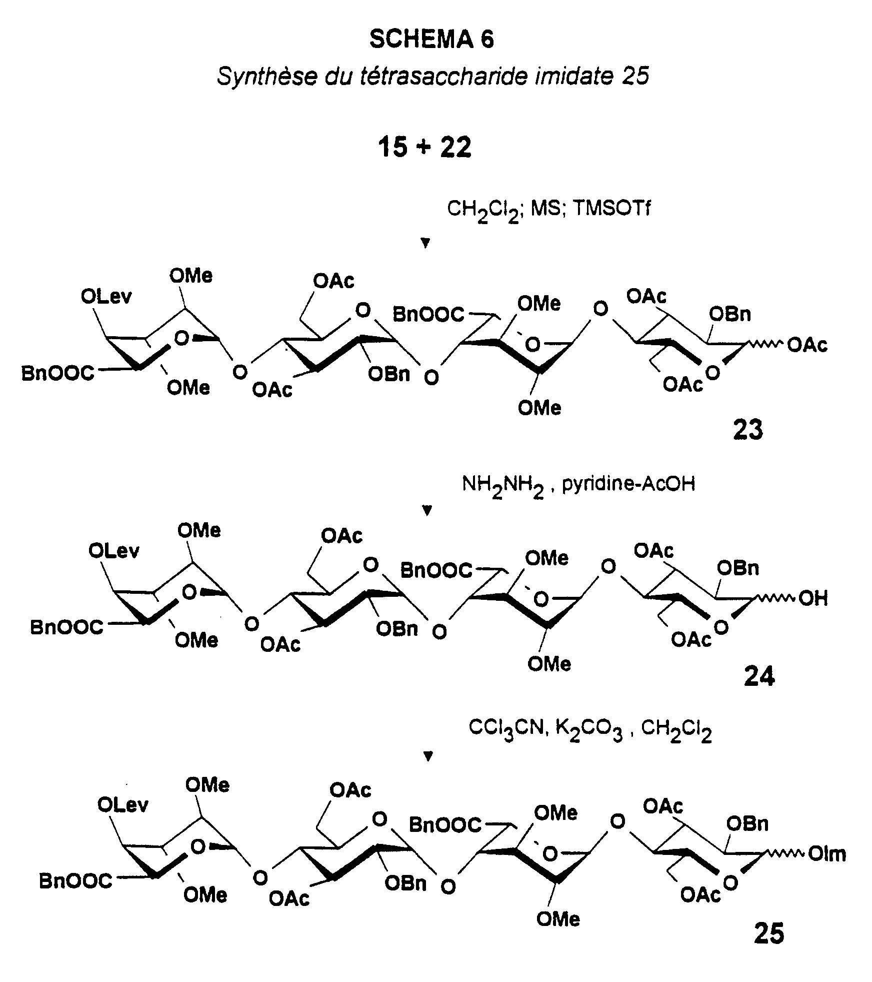

### PREPARATION 18

### O-(4-O-lévulinyl-2,3-di-O-méthyl-α-L-idopyranosyluronate de benzyle)-(1-4)-O-(3,6-di-O-acétyl-2-O-benzyl-α-D-glucopyranosyl)-(1-4)-O-(2,3-di-O-méthyl-α-L-idopyranosyluronate de benzyle)-(1-4)-1,3,6-tri-O-acétyl-2-O-benzyl-α,β-D-glucopyranose (23).

Un mélange de **15** (1,5 g, 1,7 mmol) et **22** (1,18 g; 1,7 mmol) est traité selon la méthode 3 (en remplaçant le toluène par du dichlorométhane). Une chromatographie par perméation sur gel sur une colonne de LH-20, equilibrée dans dichlorométhane/éthanol 1:1, donne **23** pur (1,75 g; 73%). [α]_{D} +19 (c = 0,9, dichlorométhane). LSIMS, mode positif: m/thioglycérol + NaCl, 1441 (M+Na)⁺; thioglycérol + KF, 1457 (M+K)⁺.
¹H RMN (CDCl₃) δ: 6,27; 5,45; 5,10; 4,96; 4,90 ppm.
Anal. Calc pour C₇₁H₈₆O₃₀ (1419,46): C, 60,08; H, 6,11. Trouvé: C, 60,06; H, 6,40.

### PREPARATION 19

### O-(4-O-lévulinyl-2,3-di-O-méthyl-α-L-idopyranosyluronate de benzyle)-(1-4)-O-(3,6-di-O-acétyl-2-O-benzyl-α-D-glucopyranosyl)-(1-4)-O-(2,3-di-O-méthyl-α-L-idopyranosyluronate de benzyle)-(1-4)-3,6-di-O-acétyl-2-O-benzyl-α,β-D-glucopyranose (24).

De l'éthanolamine (80 µl; 1,31 mmol) est ajoutée à une solution du tétrasaccharide **23** (465 mg; 327 µmol) dans le tétrahydrofurane (5 ml), puis la solution est laissé la nuit à 4 °C. Après neutralisation par l'acide chlorhydrique (1M; 2 ml), du dichlorométhane (20 ml) est ajouté, puis la solution est lavée à l'eau, séchée (sulfate de sodium), et concentrée. Une chromatographie sur colonne (3:1 toluène/acétone) donne **24** (326 mg; 79%). CCM, R_{F} 0,33, 3:1 toluène/acétone. LSIMS, mode positif: m/z thioglycérol + NaCl, 1399 (M+Na)⁺; thioglycérol + KF, 1415 (M+K)⁺.
¹H RMN (CDCl₃) δ: 5,18; 5,10; 4,96; 4,93; 4,75

### PREPARATION 20

### O-(4-O-lévulinyl-2,3-di-O-méthyl-α-L-idopyranosyluronate de benzyle)-(1-4)-O-(3,6-di-O-acétyl-2-O-benzyl-α-D-glucopyranosyl)-(1-4)-O-(2,3-di-O-méthyl-α-L-idopyranosyluronate de benzyle)-(1-4)-3,6-di-O-acétyl-2-O-benzyl-α,β-D-glucopyranose trichloroacétimidate (25).

Un mélange de trichloroacétonitrile (151 µl; 1,5 mmol), du tétrasaccharide **24** (343 mg; 249 µM), et de carbonate de potassium (62 mg; 448 µmol) dans le dichlorométhane (2 ml) est agitée une nuit à température ambiante. Du dichlorométhane est ajouté, et après filtration la solution est concentrée. Une chromatographie sur colonne (3:1 toluène/acétone) donne **25** (346 mg; 91%). CCM, R_{F} 0,42; 0,63, 2:1 dichlorométhane/acétate d'éthyle.

¹H RMN (CDCl₃) δ: 8,67 (s, NH-b), 8,59 (s, NH-a), 7,40-7,20 (m, 10H, 2Ph),6,40 (d, J = 3,5 Hz, H-1α), 5,90 (d, J = 7,5 Hz, H-1β), 3,45; 3,44; 3,42; 3,40; 3,39; 3,37 (6s, 12H, 4 OMe), 2,7-2,2 (m, 4H, O(C:O)C*H*₂C*H*₂(C:O)CH₃), 2,12; 2,08; 2,07; 2,04; 2,02; 1,91; 1,89 (7s, 15H, 4 Ac, et O(C:O)CH₂CH₂(C:O)C*H*₃). NH-a et NH-b désignent les signaux obtenus pour chacun des isomères syn et anti.

### PREPARATION 21

### Méthyl O-(4-O-lévulinyl-2,3-di-O-méthyl-α-L-idopyrano-syluronate de benzyle)-(1-4)-[O-(3,6-di-O-acétyl-2-O-benzyl-α-D-glucopyranosyl)-(1-4)-O-(2,3-di-O-méthyl-α-L-idopyranosyluronate de benzyle)-(1-4)]₃-2,3,6-tri-O-benzyl-α-D-glucopyranoside (26).

A partir de **17** et **25**. ― Un mélange de **17** et **25** (459 mg, 0,3 mmol) est traité selon la méthode 3. Une chromatographie sur colonne (Sephadex® LH 20, 195 x 3,7 cm; 1:1 dichlorométhane/éthanol), suivie d'une chromatographie sur colonne de gel de silice (3:2 cyclohexane-acétone) donne **26** pur ( 420 mg; 59%). [α]_{D} +20 (c = 0,20, dichlorométhane). LSIMS, mode positif: m/z thioglycérol + NaCI, 2771 (M+Na)⁺; thioglycérol + KF, 2787 (M+K)⁺.
¹H RMN (CDCl₃) δ: 5,26; 5,14; 5,10; 4,93; 4,92; 4,90; 4,56

### PREPARATION 22

**26** à partir de **15** et **19.** ― Un mélange de **15** (332 mg, 373 µmol) et **19** (377 mg; 186 µmol) est traité selon la méthode 2. Une chromatographie sur colonne (Sephadex® LH 20, 195 × 3,7 cm; 1:1 dichlorométhane/éthanol) donne l'octasaccharide **26** pur ( 460 mg; 90%).

### PREPARATION 23

### Méthyl O-(2,3-di-O-méthyle-α-idopyranosyluronate de benzyle)-(1-4)-[O-(3,6-di-O-acétyl-2-O-benzyl-α-D-glucopyranosyl)-(1-4)-O-(2,3-di-O-méthyl-α-L-idopyranosyluronate de benzyle)-(1-4)]₃-2,3,6-tri-O-benzyl-α-D-glucopyranoside (27).

Le composé **26** (275 mg; 100 µmol) est traité selon la méthode 1 pour donner **27** (265 mg; 96%); [α]_{D} +27 (c = 0,56, dichlorométhane). LSIMS, mode positif: m/z thioglycérol + NaCI, 2673 (M+Na)⁺; thioglycérol + KF, 2689 (M+K)⁺.
¹H RMN (CDCl₃) δ: 5,26; 5,15; 5,10; 5,08; 4,89; 4,88; 4,55

### PREPARATION 24

### Méthyl O-(4-O-lévulinyl-2,3-di-O-méthyl-α-L-idopyranosyluronate de benzyle)-(1-4)-[O-(3,6-di-O-acétyl-2-O-benzyl-α-D-glucopyranosyl)-(1-4)-O-(2,3-di-O-méthyl-α-L-idopyranosyluronate de benzyle)-(1-4)]₅-2,3,6-tri-O-benzyl-α-D-glucopyranoside (28).

Un mélange de 27 (97,3 mg; 36 µmol) et de 25 (83,8 mg, 55 µmol) est traité selon la méthode 3. Une chromatographie sur colonne (cyclohexane-acétone 2:1, puis 7:4, puis 3:2) donne 28 pur ( 102 mg; 69%). [α]_{D} + 22 (c = 0,51, dichlorométhane). CCM, R_{F} 0,18, 3:2 cyclohexane-acétone.
¹H RMN (CDCl₃) δ: 5,26; 5,14; 5,10; 5,08; 4,93; 4,92; 4,90; 4,56

### PREPARATION 25

### Méthyl O-(2,3-di-O-méthyl-α-L-idopyranosyluronate de benzyle)-(1-4)-[O-(3,6-di-O-acétyl-2-O-benzyl-α-D-glucopyranosyl)-(1-4)-O-(2,3-di-O-méthyt-α-L-idopyranosyluronate de benzyle)-(1-4)]₅-2,3,6-tri-O-benzyl-α-D-glucopyranoside (29).

Le composé **28** (216 mg; 54 µmol) est traité selon la méthode 1 pour donner **29** (199 mg; 95%). CCM, RF 0,56, 1:1 cyclohexane-acétone; R_{F} 0,55, 2:1 toluène-acétone. LSIMS, mode positif: m/z thioglycérol + NaCI, 3934 (M+Na)⁺; thioglycérol + KF, 3950 (M+K)⁺.

### PREPARATION 26

### Méthyl O-(4-O-lévulinyl-2,3-di-O-méthyl-α-L-idopyranosyluronate de benzyle)-(1-4)-[O-(3,6-di-O-acétyl-2-O-benzyl-α-D-glucopyranosyl)-(1-4)-O-(2,3-di-O-méthyl-α-L-idopyranosyluronate de benzyle)-(1-4)]₇-2,3,6-tri-O-benzyl-α-D-glucopyranoside (30).

Un mélange de **25** (33 mg, 21,4 µmol) et de **29** (52,4 mg; 13,3 µmol) est traité selon la méthode 2. Une chromatographie sur colonne (7:4 cyclohexane-acétone) donne **30** pur ( 43,8 mg; 62%). [α]_{D} +19 (c = 0,5, dichlorométhane). CCM, RF 0,36, 3:2 cyclohexane-acétone. LSIMS, mode positif: m/z thioglycérol + KF, 5310 (M+K)⁺.
¹H RMN (CDCl₃) δ des protons anomères principaux : 5,26; 5,14; 5,10; 5,08; 4,92; 4,90; 4,56

### PREPARATION 27

### Méthyl O-(2,3-di-O-méthyl-α-L-idopyranosyluronate de benzyle)-(1-4)-[O-(3,6-di-O-acétyl-2-O-benzyl-α-D-glucopyranosyl)-(1-4)-O-(2,3-di-O-méthyl-α-L-idopyranosyluronate de benzyle)-(1-4)]₇-2,3,6-tri-O-benzyl-α-D-glucopyranoside (31).

L'hexadécasaccharide **30** (100 mg; 19 µmol) est traité selon la méthode 1 pour donner **31** utilisé directement dans l'étape suivante. [α]_{D} +20 (c = 0,38, dichlorométhane). CCM, R_{F} 0,31, 4:3 cyclohexane-acétone. LSIMS, mode positif: m/z thioglycérol + NaCI, 5196 (M+Na)⁺; thioglycérol + KF, 5212 (M+K)⁺.

### PREPARATION 28

### Méthyl O-(4-O-lévulinyl-2,3-di-O-méthyl-α-L-idopyranosyluronate de benzyle)-(1-4)-[O-(3,6-di-O-acétyl-2-O-benzyl-α-D-glucopyranosyl)-(1-4)-O-(2,3-di-O-méthyl-α-L-idopyranosyluronate de benzyle)-(1-4)]₉-2,3,6-tri-O-benzyl-α-D-glucopyranoside (32).

Un mélange de **25** (31 mg; 21,7 µmol) et de **31** (94,5 mg, 18,3 µmol) est traité selon la méthode 3 pour donner 32 après plusieurs chromatographies sur colonne ( 29,2 mg; 25%). [α]_{D} +22 (c = 0,33, dichlorométhane). CCM, R_{F} 0,26, 4:3 cyclohexane-acétone. ¹H RMN (CDCl₃) δ des protons anomères principaux : 5,26; 5,14; 5,10; 5,09; 4,92; 4,91; 4,90; 4,56 ppm.

### EXEMPLE 1

### Méthyl O-(acide 2,3-di-O-méthyl-4-O-sulfo-α-L-idopyranosyluronique-(1-4)-[O-(2,3,6-tri-O-sulfo-α-D-glucopyranosyl)-(1-4)-O-(acide 2,3-di-O-méthyl-α-L-idopyranosyluronique)-(1-4)]₉-2,3,6-tri-O-sulfo-α-D-glucopyranoside, sel de sodium (33).

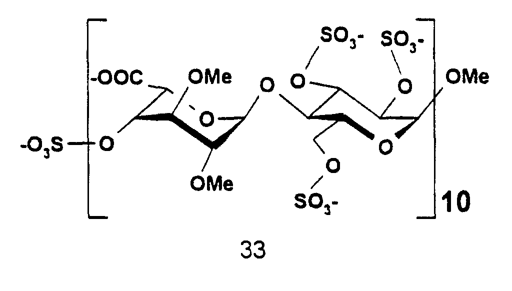

Le composé **32** est traité selon la méthode 4 pour donner **33** (60% sur les trois étapes). [α]_{D} +27 (c = 0,4, D₂O) ; ESIMS, mode négatif: masse expérimentale = 7077,3 ± 3,2 u.m.a.
¹H RMN (D₂O) δ principaux protons anomériques : δ 5,41; 5,40; 5,15; 5,09; 5,07; 5,06 ppm.

En procédant selon l'EXEMPLE 1 et selon le SCHEMA 1 ci-dessus on prépare les composés **34** à **38** (EXEMPLE 2 à 6) décrits dans le TABLEAU I ci-après.

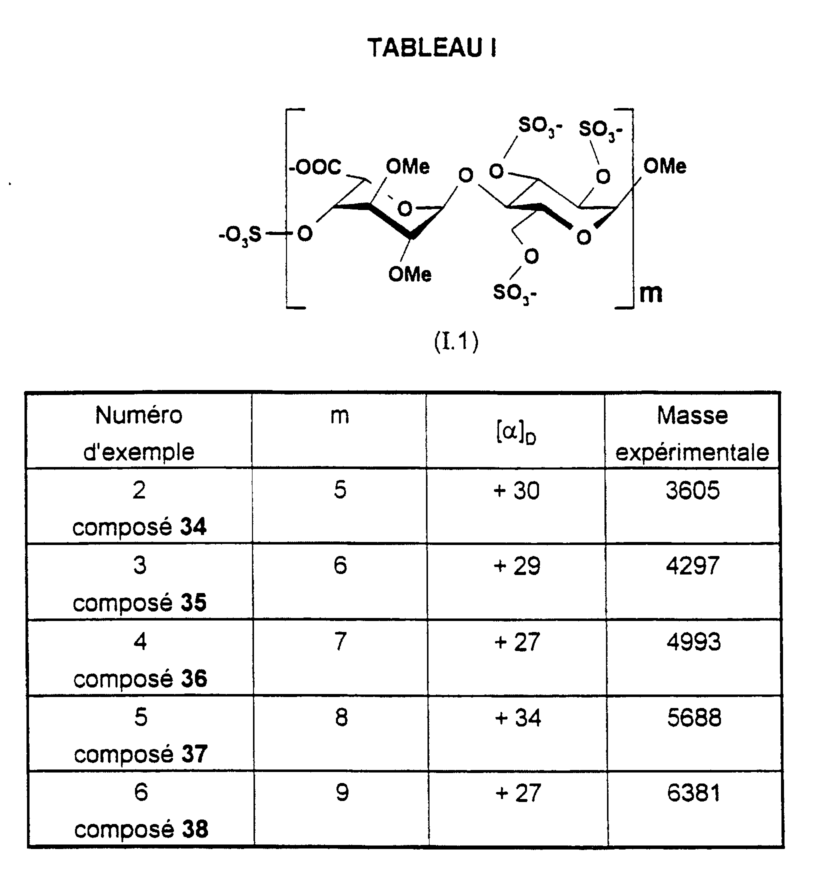

### EXEMPLE 7

### Méthyl O-(acide 2,3-di-O-méthyl-4-O-sulfo-β-D-glucopyranosyluronique)-(1-4)-[O-(2,3,6-tri-O-sulfo-α-D-glucopyranosyl)-(1-4)-O-(acide 2,3-di-O-méthyl-β-D-glucopyranosyluronique)-(1-4)]₄-2,3,6-tri-O-sulfo-α-D-glucopyranoside, sel de sodium (39).

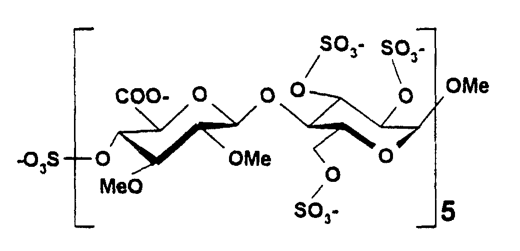

Le disaccharidique donneur de glycosyle 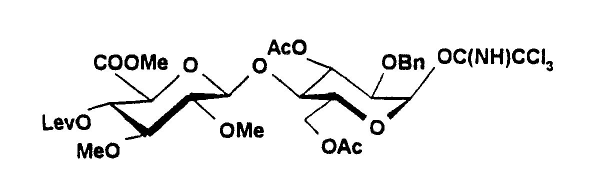 et le disaccharide accepteur de glycosyle 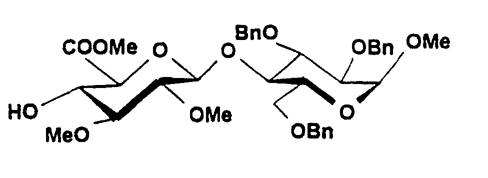 sont préparés selon les méthodes décrites dans Westerduin *et al.*, BioOrg. Med. Chem., 2, 1994, 1267, puis combinés comme décrit lors de la préparation de **34.** Le décasaccharide résultant est traité selon la méthode 4 pour donner **39.**
[α]_{D} + 45 (*c* = 1, H₂O). ¹H RMN (D₂O) δ des protons anomériques principaux : 5,53; 5,18; 4,65; 4,63 ppm.

En procédant selon l'EXEMPLE 7 et selon le SCHEMA 1 ci-dessus, on prépare le composé **40** (EXEMPLE 8) décrit dans le TABLEAU II ci-après.

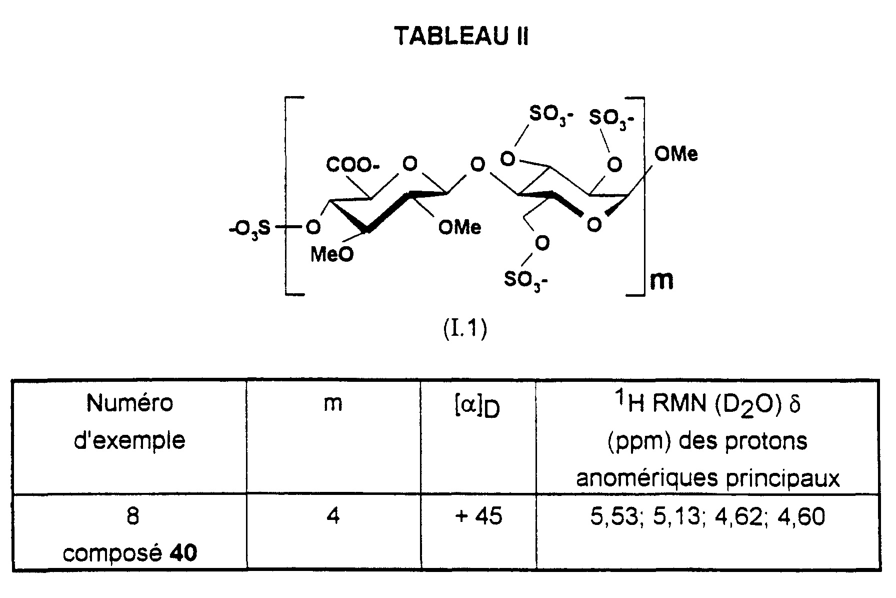

### EXEMPLE 9

### Méthyl O-(acide 3-O-méthyl-2,4-di-O-sulfo-α-L-idopyranosyluronique)-(1-4)-[O-(3-O-méthyl-2,6-di-O-sulfo-α-D-glucopyranosyl)-(1-4)-O-(acide 3-O-méthyl-2-O-sulfo -α-L-idopyranosyluronique)-(1-4)]₄-3-O-méthyl-2,6-di-O-sulfo-α-D-glucopyranoside, sel de sodium (41).

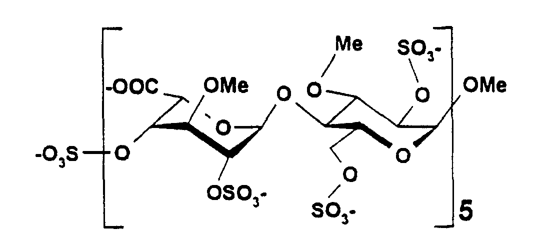

Le synthon disaccharidique

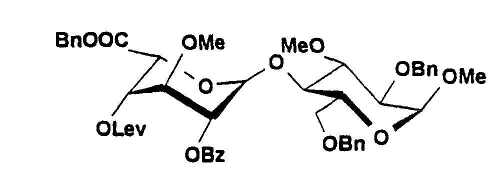

(CCM, R_{F} 0,54, 1:1 cyclohexane/EtOAc) est traité comme décrit pour **12** pour donner l'imidate donneur de glycosyle 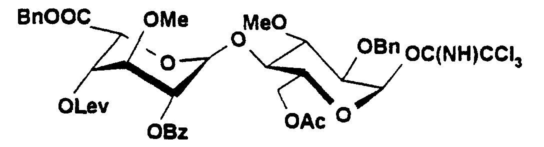 et l'accepteur 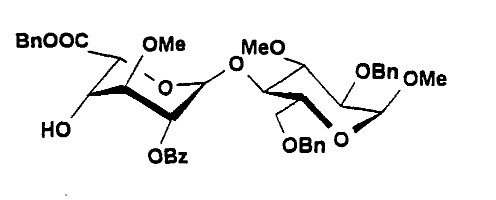 [¹H RMN (CDCl₃) δ 8,00-7,15 (m, 20H, 4Ph), 5,15 (d, 1H, H-1'), 4,57 (d, 1H, H-1), 3,48; 3,47; 3,32 (3s, 3 OCH₃)].

Ces trois synthons sont alors combinés comme il est décrit pour la préparation de **34.** Le décasaccharide résultant est alors traité selon la méthode 4 pour donner **41.**

[α]_{D} + 17 (*c* = 1, H₂O). ¹H RMN (D₂O) δ des protons anomériques principaux : 5,36; 5,34; 5,13; 5,11; 5,09; 5,05 ppm.

En procédant selon l'EXEMPLE 9 et selon le SCHEMA 1 ci-dessus on prépare les composés **42** et **43** (EXEMPLE 10 et 11) décrits dans le TABLEAU III ci-après.

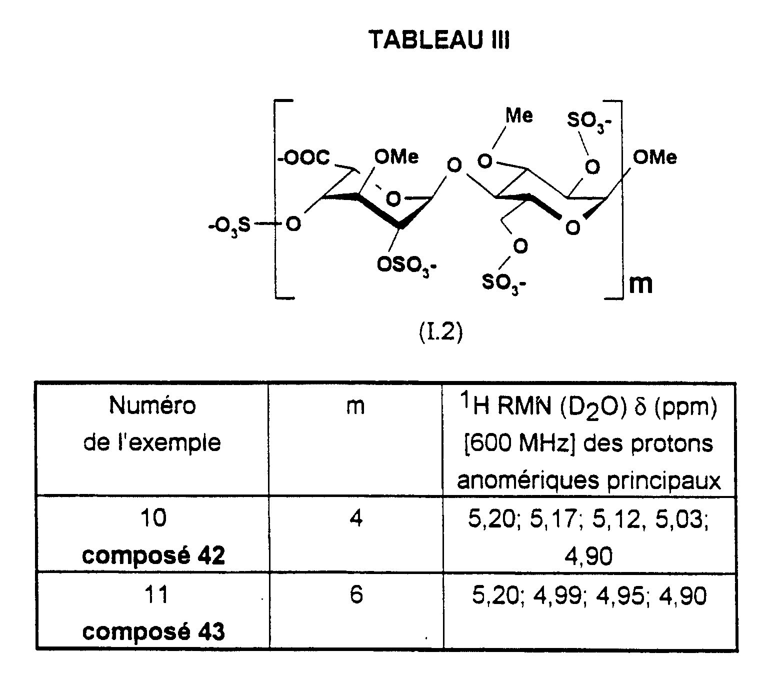

### EXEMPLE 12

### Méthyl O-(acide 3-O-méthyl-2,4-di-O-sulfo-β-D-glucopyranosyluronique)-(1-4)-[O-(3-O-méthyl-2,6-di-O-sulfo-α-D-glucopyranosyl)-(1-4)-O-(acide 3-O-méthyl-2-O-sulfo-β-D-glucopyranosyluronique)-(1-4)]₄-3-O-méthyl-2,6-di-O-sulfo-α-D-glucopyranoside, sel de sodium (44).

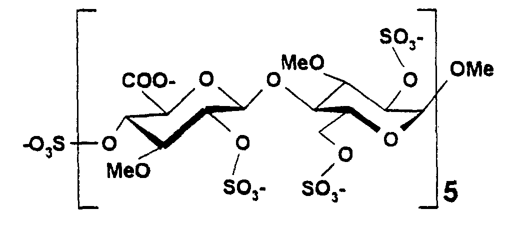

Les synthons disaccharidiques 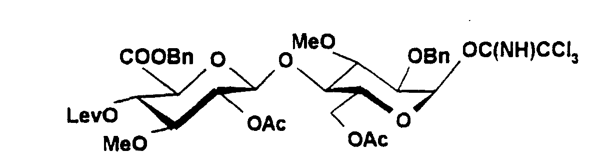 et 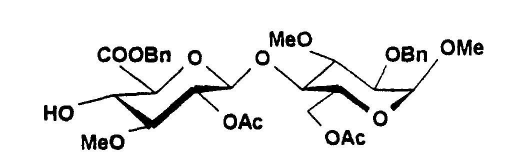 sont préparés puis combinés comme décrit pour **34.** Le décasaccharide obtenu traité selon la méthode 4 donne **44:**
[α]_{D} + 25 (c = 0,2, H₂O). ¹H RMN (D₂O) δ des protons anomériques principaux : 5,47; 5,07; 4,74; 4,71; 4,70 ppm.

### EXEMPLE 13

### Méthyl O-(acide 3-O-méthyl-2,4-di-O-sulfo-α-L-idopyranosyluronique)-(1-4)-[O-(3-O-méthyl-2,6-di-O-sulfo-α-D-glucopyranosyl)-(1-4)-O-(acide 3-O-méthyl-2-O-sulfo-α-L-idopyranosyluronique)-(1-4)]₂-O-(2,3,6-tri-O-sulfo-α -D-glucopyranosyl)-(1-4)-O-(acide 3-O-méthyl-2-O-sulfo-α-L-idopyranosyluronique)-(1-4)-3-O-méthyl-2,6-di-O-sulfo-α-D-glucopyranoside, sel de sodium (45).

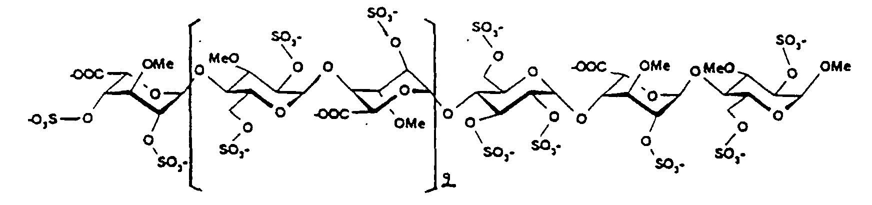

Le donneur de glycosyle **15** et l'accepteur de glycosyle 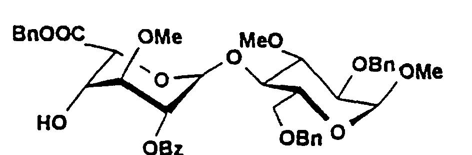 combinés selon la méthode 3 donnent le tétrasaccharide

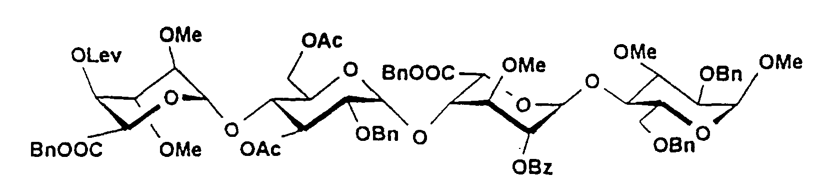

Après coupure du groupe Lev (méthode 1) et réaction répétée avec le disaccharide donneur de glycosyle suivant, selon le principe décrit dans le schéma 1,

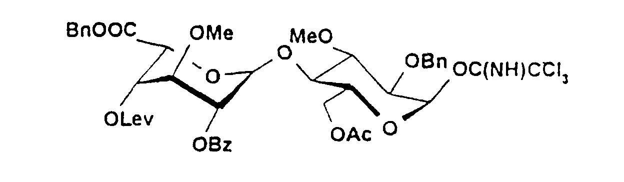

on obtient le précurseur complètement protégé de **45** qui est traité selon la méthode 4 pour donner **45.**
¹H RMH (D₂O) δ (ppm) [600 MHz] des protons anomériques principaux : 5,35; 5,33; 5,30; 5,22; 5,21; 5,18; 5,15; 4,98.

En procédant selon l'EXEMPLE 13 et selon le SCHEMA 1 ci-dessus à partir des précurseurs oligosaccharidiques complètement protégés on prépare le composé **46** (EXEMPLE 14) décrits dans le TABLEAU IV ci-après.

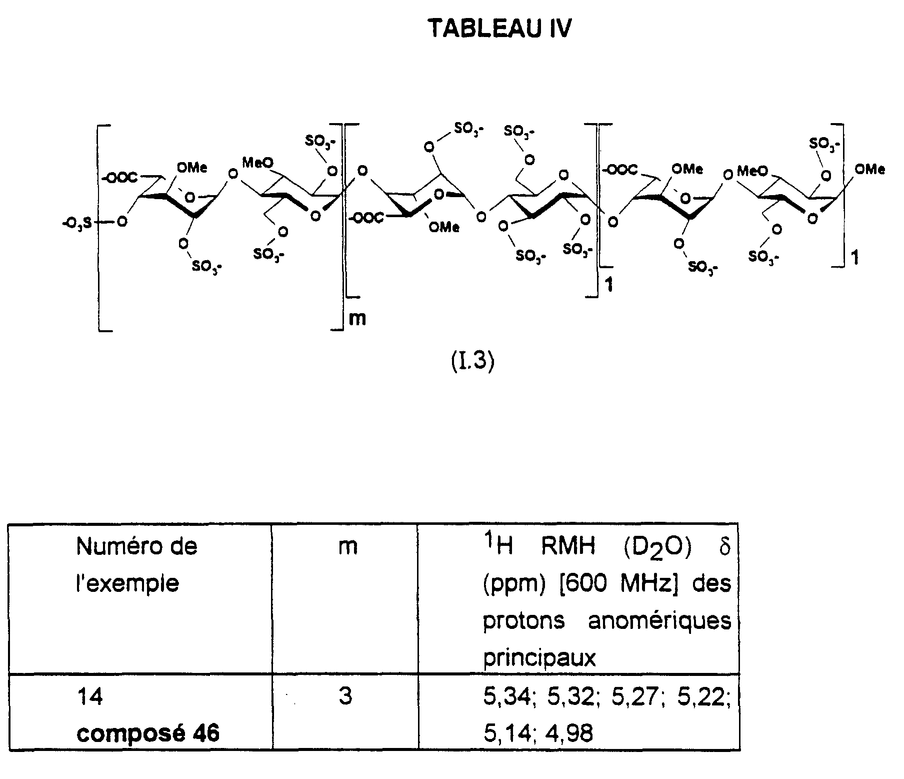

## Claims

1. Synthetic polysaccharide containing 8 to 24 monosaccharide units, formed by a disaccharide chain consisting of a uronic acid and a hexose, said polysaccharide being **characterised in that** all its hydroxyl groups are etherified with a (C₁₋₆)alkyl group or esterified in the form of a sulpho group, each disaccharide being at least monoetherified; and the salts thereof.

2. Salt consisting of an anion and a cation, the anion having the formula: 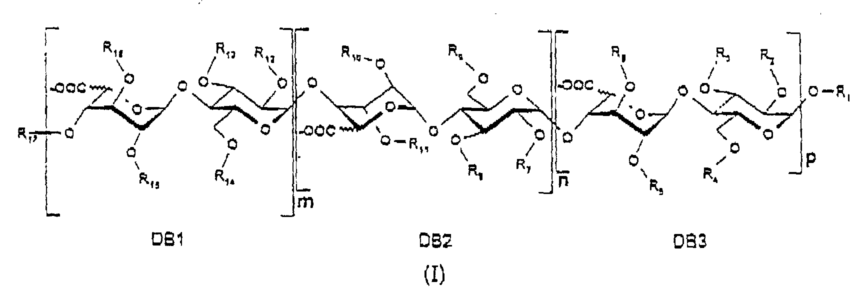 wherein
- the broken line designates a bond either below or above the plane of the pyranose ring;
- R₁, R₆, R₁₁ and R₁₆ denote a (C₁₋₆)alkyl;
- R₂, R₃, R₄, R₅, R₇, R₈, R₉, R₁₀, R₁₂, R₁₃, R₁₄, R₁₅ and R₁₇ denote a (C₁₋₆)alkyl or an SO₃⁻ group;
- m, n and p are such that the sum of m + n + p is greater than or equal to 4 and less than or equal to 12, while one or two of the three may represent zero;
the cation being a pharmaceutically acceptable monovalent cation, and the corresponding acid.

3. Salt according to claim 2, wherein the cation is selected from among the alkali metal cations, particularly sodium and potassium cations.

4. Salt according to one of claims 2 or 3 wherein the alkyls are methyls, and the corresponding acid.

5. Salt according to one of claims 2 or 3 wherein n and p are equal to zero, and the corresponding acid.

6. Salt according to one of claims 2 or 3 wherein n and p are equal to zero and m denotes 4 to 10, and the corresponding acid.

7. Salt according to one of claims 2 or 3 wherein n and p are equal to zero, m represents 4 to 10; at least one of the substituents R₁₂, R₁₃, R₁₄ and R₁₅ represents a sulphate group; R₁, R₁₆ and R₁₇ being as defined for (I), and the corresponding acid.

8. Salt according to one of claims 2 or 3 wherein n and p are equal to zero, m represents 4 to 10; at least two of the substituents R₁₂, R₁₃, R₁₄ and R₁₅ represent a sulphate group; R₁, R₁₆ and R₁₇ are as defined for (I), and the corresponding acid.

9. Salt according to one of claims 2 or 3 wherein n and p are equal to zero, m represents 4 to 10; at least three of the substituents R₁₂, R₁₃, R₁₄ and R₁₅ denote a sulphate group; R₁, R₁₆ and R₁₇ are as defined for (I), and the corresponding acid.

10. Salt according to claim 2 or 3 wherein the anion has the formula (I,1) : 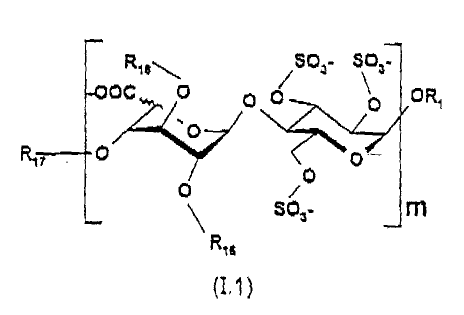 wherein m represents 4 to 10; R₁, R₁₅, R₁₆ and R₁₇ are as defined for (I), each uronic acid being either an iduronic or glucuronic acid, and the corresponding acid.

11. Salt according to one of claims 2 or 3 wherein the anion has the formula (I.2) : 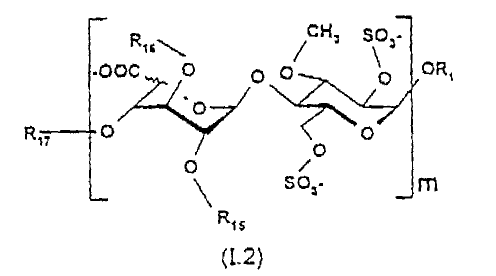 wherein m represents 4 to 10; R₁, R₁₅, R₁₆ and R₁₇ are as defined for (I), each uronic acid being either an iduronic or a glucuronic acid, and the corresponding acid.

12. Salt according to claim 2 or 3 wherein the anion has the formula (I.3): 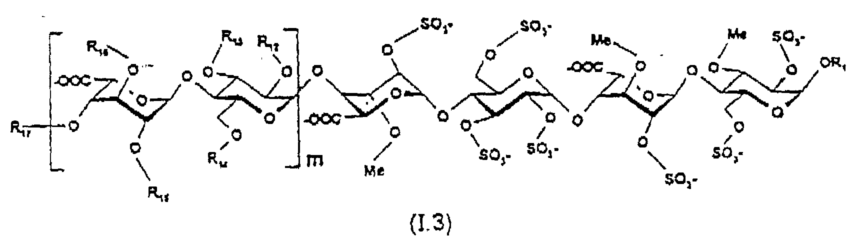 wherein m represents 2 or 3, R₁, R₁₂, R₁₃, R₁₄, R₁₅, R₁₆ and R₁₇ are as defined for (I), each uronic acid being either an iduronic or a glucuronic acid, and the corresponding acid.

13. Salt according to claim 12, wherein R₁ denotes a methyl, R₁₃ in the 3 position of the glucose denotes a methyl, R₁₂ in the 2 position and R₁₄ in the 6 position of the glucose denote an SO₃⁻ and R₁₆ in position 3 of the iduronic or glucuronic unit denotes a methyl, m being equal to 2 or 3.

14. Polysaccharide selected from among;
Methyl *O*-(2,3-di-*O*-methyl-4-*O*-sulpho-α-L-idopyranosyluronic acid) - (1-4)-[*O*-(2,3,6-tri-*O*-sulpho-α-D-glucopyranosyl)-(1-4)-*O*-(2,3-di-*O*-methyl-α-L-idopyranosyluronic acid)-(1-4)]₉-2,3,6-tri-*O*-sulpho-α-D-glucopyranoside, sodium salt;
Methyl *O*-(2,3-di-*O*-methyl-4-*O*-sulpho-α-L-idopyranosyluronic acid)-(1-4)-[*O*-(2,3,6-tri-*O*-sulpho-α-D-glucopyranosyl)-(1-4)-*O*-(2,3-di-*O*-methyl-α-L-idopyranosyluronic acid)-(1-4)]₄-2,3,6-tri-*O*-sulpho-α-D-glucopyranoside, sodium salt;
Methyl *O*-(2,3-di-*O*-methyl-4-O-sulpho-α-L-idopyranosyluronic acid)-(1-4)-[*O*-(2,3,6-tri-*O*-sulpho-α-D-glucopyranosyl)-(1-4)-*O*-(2,3-di-*O*-methyl-α-L-idopyranosyluronic acid)-(1-4)]₅-2,3,6-tri-*O*-sulpho-α-D-glucopyranoside, sodium salt;
Methyl O-(2,3-di-*O*-methyl-4-O-sulpho-α-L-idopyranosyluronic acid)-(1-4)-[*O*-(2,3,6-tri-*O*-sulpho-α-D-glucopyranosyl)-(1-4)-*O*-(2,3-di-*O*-methyl-α-L-idopyranosyluronic acid) -(1-4)]₆-2,3,6-tri-*O*-sulpho-α-D-glucopyranoside, sodium salt;
Methyl *O*-(2,3-di-*O*-methyl-4-O-sulpho-α-L-idopyranosyluronic acid)-(1-4)-[*O*-(2,3,6-tri-*O*-sulpho-α-D-glucopyranosyl)-(1-4)-*O*-(2,3-di-*O*-methyl-α-L-idopyranosyluronic acid) - (1-4)]₇-2,3,6-tri-*O*-sulpho-α-D-glucopyranoside, sodium salt;
Methyl *O*-(2,3-di-*O*-methyl-4-O-sulpho-α-L-idopyranosyluronic acid)-(1-4)-[*O*-(2,3,6-tri-*O*-sulpho-α-D-glucopyranosyl)-(1-4)-*O*-(2,3-di-*O*-methyl-α-L-idopyranosyluronic acid)-(1-4)]₈-2,3,6-tri-*O*-sulpho-α-D-glucopyranoside, sodium salt;
Methyl O-(2,3-di-*O*-methyl-4-O-sulpho-β-D-glucopyranosyluronic acid)-(1-4)-[0-(2,3,6-tri-*O*-sulpho-α-D-glucopyranosyl)-(1-4)-*O*-(2,3-di-*O*-methyl-β-D-glucopyranosyluronic acid) - (1-4)]₄-2,3,6-tri-*O*-sulpho-α-D-glucopyranoside, sodium salt;
Methyl O-(2,3-di-*O*-methyl-4-O-sulpho-β-D-glucopyranosyluronic acid)-(1-4)-[*O*-(2,3,6-tri-*O*-sulpho-α-D-glucopyranosyl)-(1-4)-*O*-(2,3-di-O-methyl-β-D-glucopyranosyluronic acid)-(1-4)]₃-2,3,6-tri-*O*-sulpho-α-D-glucopyranoside, sodium salt;
Methyl *O*-(3-*O*-methyl-2,4-di-*O*-sulpho-α-L-idopyranosyluronic acid)-(1-4)-[*O*-[3-*O*-methyl-2,6-di-*O*-sulpho-α-D-glucopyranosyl)-(1-4)-O- [3-*O*-methyl-2-*O*-sulpho-α-L-idopyranosyluronic acid) - (1-4)]₄-3-*O*-methyl-2,6-di-*O*-sulpho-α-D-glucopyranoside, sodium salt;
Methyl *O*-(3-*O*-methyl-2,4-di-*O*-sulpho-α-L-idopyranosyluronic acid)-(1-4)-[*O*-(3-*O*-methyl-2,6-di-*O*-sulpho-α-D-glucopyranosyl)-(1-4)-*O*-(3-*O*-methyl-2-*O*-sulpho-α-L-idopyranosyluronic acid)-(1-4)]₃-3-*O*-methyl-2,6-di-*O*-sulpho-α-D-glucopyranoside, sodium salt;
Methyl *O*-(3-*O*-methyl-2,4-di-*O*-sulpho-α-L-idopyranosyluronic acid) - (1-4) - [*O*-(3-*O*-methyl-2,6-di-*O*-sulpho-α-D-glucopyranosyl)-(1-4)-*O*-(3-*O*-methyl-2-*O*-sulpho-α-L-idopyranosyluronic acid) - (1-4)]₅-3-*O*-methyl-2,6-di-*O*-sulpho-α-D-glucopyranoside, sodium salt;
Methyl *O*-(3-*O*-methyl-2,4-di-*O*-sulpho-β-D-glucopyranosyluronic acid)-(1-4)-[*O*-(3-*O*-methyl-2,6-di-*O*-sulpho-α-D-glucopyranosyl)-(1-4)-*O*-(3-*O*-methyl-2-*O*-sulpho-β-D-glucopyranosyluronic acid)-(1-4)]₄-3-*O*-methyl-2,6-di-*O*-sulpho-α-D-glucopyranoside, sodium salt;
Methyl *O*-(3-*O*-methyl-2,4-di-*O*-sulpho-α-L-idopyranosyluronic acid) - (1-4) - [*O*-(3-*O*-methyl-2,6-di-*O*-sulpho-α-D-glucopyranosyl)-(1-4)-*O*-(3-*O*-methyl-2-*O*-sulpho-α-L-idopyranosyluronic acid)-(1-4)]₂-*O*-(2,3,6-tri-*O*-sulpho-α-D-glucopyranosyl)-(1-4)-*O*-(3-*O*-methyl-2-*O*-sulpho-α-L-idopyranosyluronic acid)-(1-4)-3-*O*-methyl-2,6-di-*O*-sulpho-α-D-glucopyranoside, sodium salt; and
Methyl *O*-(3-*O*-methyl-2,4-di-*O*-sulpho-α-L-idopyranosyluronic acid)-(1-4)-[*O*-(3-*O*-methyl-2,6-di-*O*-sulpho-α-D-glucopyranosyl)-(1-4)-*O*-(3-*O*-methyl-2-*O*-sulpho-α-L-idopyranosyluronic acid)-(1-4)]₃-*O*- (2,3,6-tri-*O*-sulpho-α-D-glucopyranosyl)-(1-4)-*O*-(3-*O*-methyl-2-*O*-sulpho-α-L-idopyranosyluronic acid)-(1-4)-3-*O*-methyl-2,6-di-*O*-sulpho-α-D-glucopyranoside, sodium salt.

15. Process for preparing compounds of formula (I) according to claim 2, **characterised in that**:
(a) a monosaccharide donating a glucoside bond is coupled with a monosaccharide which accepts a glycoside bond by the conventional methods of sugar chemistry to obtain a fully protected intermediate saccharide synthon of the disaccharide type of formula (A): 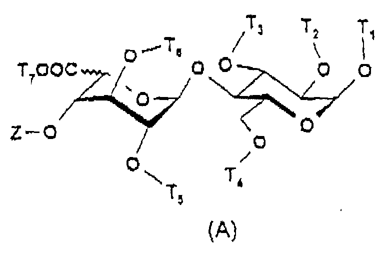 wherein the substituents T₁, T₂, T₃, T₄, T₅, T₆, T₇, T₈ and Z, which may be identical or different, are selected from among the protecting groups used in sugar chemistry as a permanent, semi-permanent or temporary protecting group,
(b) the disaccharide of formula (A) above is chemically modified to obtain an intermediate saccharide synthon of the disaccharide type which gives a glycoside bond of formula (B): 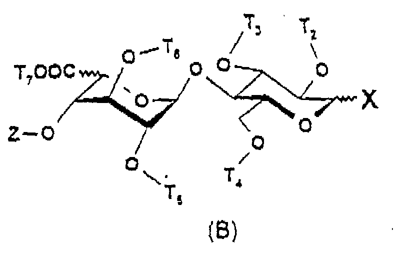 wherein T₂ to T₇ and Z are as hereinbefore defined for (A) and X denotes a group which activates the anomeric carbon, then
(c) the disaccharide of formula (A) above is chemically modified so as to obtain an intermediate saccharide synthon of the disaccharide type which accepts a glycoside bond of formula (C): 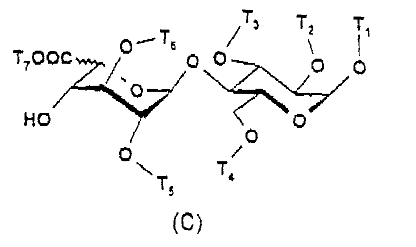 wherein T₁ to T₇ are as hereinbefore defined for (A), while selectively eliminating the protecting group Z by conventional methods of sugar chemistry, then
(d) a disaccharide which donates a glycoside bond of formula (B) obtained hereinbefore and a disaccharide which accepts a glycoside bond of formula (C) obtained hereinbefore are coupled so as to obtain a fully protected tetrasaccharide of formula (D): 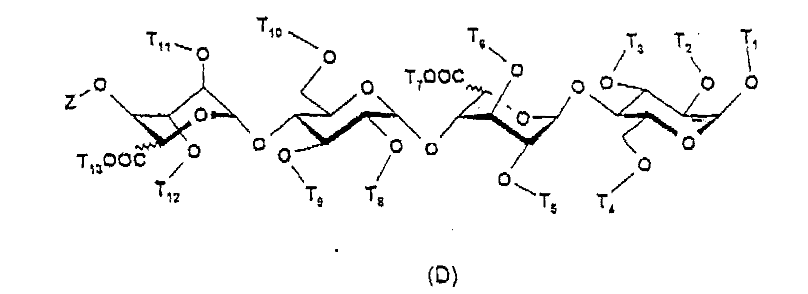 wherein T₁ to T₇ and Z are as hereinbefore defined for (A) and T₈, T₉, T₁₀, T₁₁, T₁₂ and T₁₃ are as hereinbefore defined for T₂ to T₇, then
(e) the intermediate saccharide synthon of the tetrasaccharide type of formula (D) is chemically modified to obtain an intermediate saccharide synthon of the tetrasaccharide type which donates a glycoside bond of formula (E): 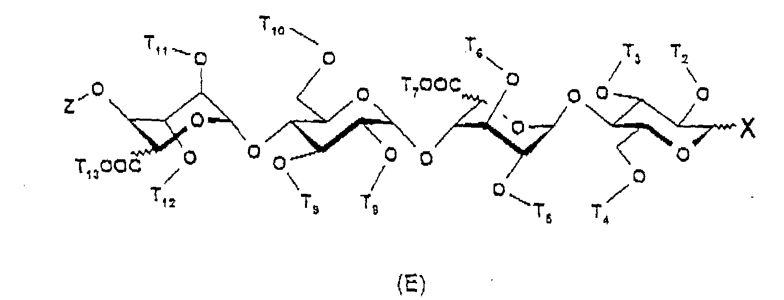 wherein X has the same definition as (B) and T₂ to T₁₃ are as hereinbefore defined for (D), then
(f) the tetrasaccharide of formula (D) is subsequently selectively deprotected so as to obtain a tetrasaccharide which accepts a glycoside bond of formula (F): 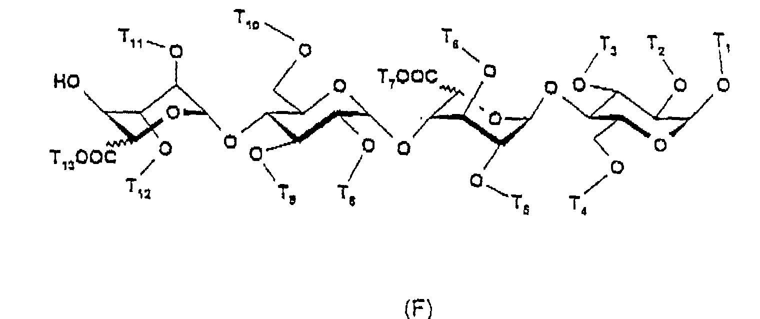 wherein T₁ to T₁₃ are as previously defined for (D), then
(g) the tetrasaccharide which accepts a glycoside bond of formula (F) and a disaccharide which donates a glycoside bond of formula (B) such as those obtained hereinbefore are coupled to form a fully protected intermediate synthon of the hexasaccharide type of formula (G): 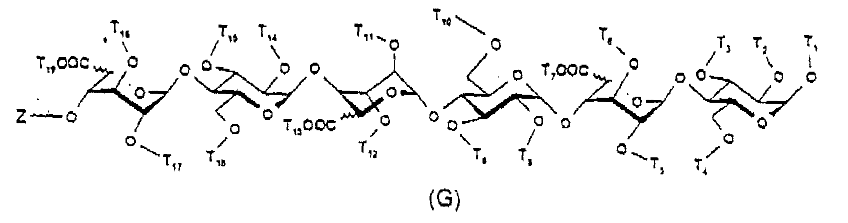 wherein T₁ to T₁₃ are as hereinbefore defined for (D) and T₁₄ to T₁₉ have the same definitions as T₂ to T₇ for (B);
or the glycoside bond accepting tetrasaccharide of formula (F) and a glycoside bond donating tetrasaccharide of formula (E) are coupled so as to obtain a fully protected octasaccharide of formula (H): 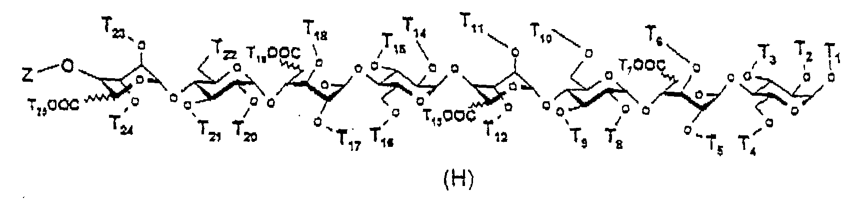 wherein T₁ to T₁₉ and Z are as hereinbefore defined and T₂₀ to T₂₅ have the same definitions as T₂ to T₇ for (B), then
(h) the hexasaccharide of formula (G) or the octasaccharide of formula (H) obtained hereinbefore are chemically modified so as to obtain a glycoside bond-accepting intermediate synthon of the hexasaccharide type of formula (G) wherein Z denotes hydrogen or a glycoside bond-accepting octasaccharide of formula (H) wherein Z denotes hydrogen,
(i) the previous steps of deprotection and coupling are repeated until the fully protected oligosaccharide having the desired structure is obtained, the glucosyl donating and glycosyl accepting intermediate saccharide synthons being selected as a function of the final structure so as to obtain the protected precursor of the final desired polysaccharide of formula (I) wherein the nature of the protective substituents determines the position of the sulphate and alkyl groups in the end product (I), and
(j) the alcohol functions which have to be sulphated are deprotected by eliminating substituents T₁ to T₂₅ which were protecting the functions during the steps of constructing the skeleton, then finally
(k) sulphating is carried out to obtain the compounds (I), or one of the salts thereof.

16. Pharmaceutical compositions containing as active principle a polysaccharide or salt according to any one of claims 1 to 14, in the form of a salt with a pharmaceutically acceptable base or in acid form, combined or mixed with a pharmaceutically acceptable non-toxic inert excipient.

17. Pharmaceutical composition according to claim 16 in the form of dosage units wherein the active principle is mixed with at least one pharmaceutical excipient.

18. Composition according to claim 17, wherein each dosage unit contains from 0.1 to 100 mg of active principle.

19. Composition according to claim 18, wherein each dosage unit contains from 0.5 to 50 mg of active principle.

20. Pharmaceutical composition containing a polysaccharide or salt according to claims 1 to 14 combined with another active ingredient which is antithrombotic, anticoagulant, anti-platelet aggregation or antagonistic to the glycoprotein IIb/IIIa complex.

21. Pharmaceutical composition according to claim 20, **characterised in that** the associated active principle is dipyridamole, aspirin, ticlopidine or clopidogrel.

22. Use of the polysaccharide and salt according to claims 1 to 14 for preparing a medicament which may be used in diseases depending on clotting disfunction.

23. Use of the polysaccharide and salt according to claims 1 to 14 for preparing a medicament which can be used to inhibit growth factors manifested by an inhibition of cell proliferation.

24. Use of the polysaccharide and salt according to claims 1 to 14 for the preparation of a medicament having antiviral, hypolipidaemic, anti-free radical, antimetastatic, antiangiogenic or anti-inflammatory properties.

## Revendications

1. Polysaccharide de synthèse contenant de 8 à 24 unités monosaccharidiques formée par un enchaînement de disaccharides constitués d'un acide uronique et d'un hexose, ledit polysaccharide étant **caractérisé en ce que** tous ses groupes hydroxyles sont éthérifiés avec un groupe (C₁-C₆)alkyle ou estérifiés sous la forme de groupe sulfo, chaque disaccharide étant au moins monoéthérifié ; ainsi que ses sels.

2. Sel constitué d'un anion et d'un cation, l'anion ayant pour formule : 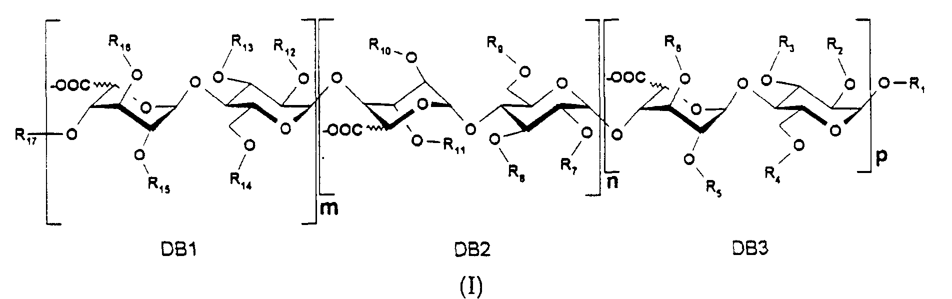 dans laquelle
- le trait ondulé désigne soit une liaison en dessous soit en dessus du plan du cycle pyranosique;
- R₁, R₆, R₁₁ et R₁₆ représentent un (C₁-C₆)alkyle;
- R₂, R₃, R₄, R₅, R₇, R₈, R₉, R₁₀, R₁₂, R₁₃, R₁₄, R₁₅ et R₁₇ représentent un (C₁-C₆)alkyle ou un groupe SO₃⁻;
- m, n et p sont tels que la somme m + n + p est supérieure ou égale à 4 et inférieure ou égale à 12, un ou deux des trois pouvant représenter zéro;
le cation étant un cation monovalent pharmaceutiquement acceptable, ainsi que l'acide correspondant.

3. Sel selon la revendication 2 dans lequel le cation est choisi parmi les cations des métaux alcalins, en particulier le sodium et le potassium.

4. Sel selon l'une des revendications 2 ou 3 dans lequel les alkyles sont des méthyles, ainsi que l'acide correspondant .

5. Sel selon l'une des revendications 2 ou 3 dans lequel n et p sont égaux à zéro, ainsi que l'acide correspondant.

6. Sel selon l'une des revendications 2 ou 3 dans lequel n et p sont égaux à zéro et m représente 4 à 10, ainsi que l'acide correspondant.

7. Sel selon l'une des revendications 2 ou 3 dans lequel n et p sont égaux à zéro, m représente 4 à 10 ; un au moins des substituants R₁₂, R₁₃, R₁₄ et R₁₅ représente un groupe sulfate ; R₁, R₁₆ et R₁₇ étant tels que définis pour (I), ainsi que l'acide correspondant.

8. Sel selon l'une des revendications 2 ou 3 dans lequel n et p sont égaux à zéro, m représente 4 à 10 ; deux au moins des substituants R₁₂, R₁₃, R₁₄ et R₁₅ représentent un groupe sulfate ; R₁, R₁₆ et R₁₇ étant tels que définis pour (I), ainsi que l'acide correspondant.

9. Sel selon l'une des revendications 2 ou 3, dans lequel n et p sont égaux à zéro, m représente 4 à 10 ; trois au moins des substituants R₁₂, R₁₃, R₁₄ et R₁₅ représentent un groupe sulfate ; R₁, R₁₆ et R₁₇ étant tels que définis pour (I), ainsi que l'acide correspondant.

10. Sel selon la revendication 2 ou 3 dont l'anion a pour formule (I.1): 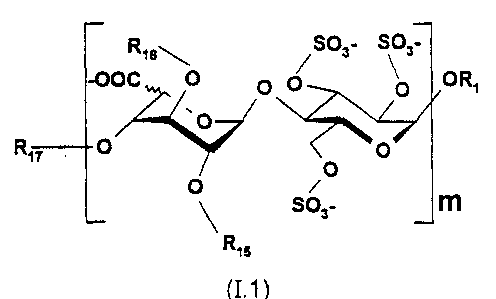 dans laquelle m représente 4 à 10 ; R₁, R₁₅, R₁₆ et R₁₇ sont tels que définis pour (I), chaque acide uronique étant soit un acide iduronique soit glucuronique, ainsi que l'acide correspondant.

11. Sel selon l'une des revendications 2 ou 3 dont l'anion a pour formule (I.2): 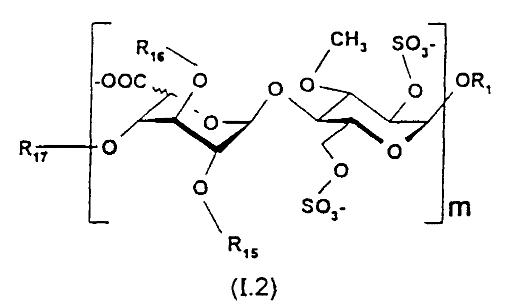 dans laquelle m représente 4 à 10 ; R₁, R₁₅, R₁₆ et R₁₇ sont tels que définis pour (I), chaque acide uronique étant soit un acide iduronique soit glucuronique, ainsi que l'acide correspondant.

12. Sel selon les revendications 2 ou 3 dont l'anion a pour formule (I.3) : 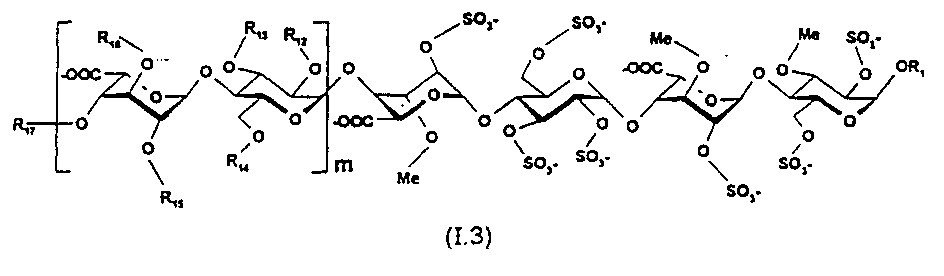 dans laquelle m représente 2 ou 3, R₁, R₁₂, R₁₃, R₁₄, R₁₅, R₁₆ et R₁₇ étant tels que définis pour (I), chaque acide uronique étant soit un acide iduronique soit glucuronique, ainsi que l'acide correspondant.

13. Sel selon la revendication 12 dans lequel R₁ représente un méthyle, R₁₃ en position 3 du glucose représente un méthyle, R₁₂ en position 2 et R₁₄ en position 6 du glucose représentent un SO₃- et R₁₆ en position 3 de l'unité iduronique ou glucuronique représente un méthyle, m étant égal à 2 ou 3.

14. Polysaccharide choisi parmi :
Méthyl *O*-(acide 2,3-di-*O*-méthyl-4-O-sulfo-α-L-idopyranosyluronique)-(1-4)-[*O*-(2,3,6-tri-*O*-sulfo-α-D-glucopyranosyl)-(1-4)-*O*-(acide 2,3-di-*O*-méthyl-α-L-idopyranosyluronique)-(1-4)]₉-2,3,6-tri-*O*-sulfo-α-D-glucopyranoside, sel de sodium;
Méthyl *O*-(acide 2,3-di-*O*-méthyl-4-O-sulfo-α-L-idopyranosyluronique)-(1-4)-[*O*-(2,3,6-tri-*O*-sulfo-α-D-glucopyranosyl)-(1-4)-*O*-(acide 2,3-di-*O*-méthyl-α-L-idopyranosyluronique)-(1-4)]₄-2,3,6-tri-*O*-sulfo-α-D-glucopyranoside, sel de sodium;
Méthyl *O*-(acide 2,3-di-*O*-méthyl-4-O-sulfo-α-L-idopyranosyluronique)-(1-4)-[*O*-(2,3,6-tri-*O*-sulfo-α-D-glucopyranosyl)-(1-4)-*O*-(acide 2,3-di-*O*-méthyl-α-L-idopyranosyluronique)-(1-4)]₅-2,3,6-tri-*O*-sulfo-α-D-glucopyranoside, sel de sodium;
Méthyl *O*-(acide 2,3-di-*O*-méthyl-4-O-sulfo-α-L-idopyranosyluronique)-(1-4)-[*O*-(2,3,6-tri-*O*-sulfo-α-D-glucopyranosyl)-(1-4)-*O*-(acide 2,3-di-*O*-méthyl-α-L-idopyranosyluronique)-(1-4)]₆-2,3,6-tri-*O*-sulfo-α-D-glucopyranoside, sel de sodium;
Méthyl *O*-(acide 2,3-di-*O*-méthyl-4-O-sulfo-α-L-idopyranosyluronique)-(1-4)-[*O*-(2,3,6-tri-*O*-sulfo-α-D-glucopyranosyl)-(1-4)-*O*-(acide 2,3-di-*O*-méthyl-α-L-idopyranosyluronique)-(1-4)]₇-2,3,6-tri-*O*-sulfo-α-D-glucopyranoside, sel de sodium;
Méthyl *O*-(acide 2,3-di-*O*-méthyl-4-O-sulfo-α-L-idopyranosyluronique)-(1-4)-[*O*-(2,3,6-tri-*O*-sulfo-α-D-glucopyranosyl)-(1-4)-*O*-(acide 2,3-di-*O*-méthyl-α-L-idopyranosyluronique)-(1-4)]₈-2,3,6-tri-*O*-sulfo-α-D-glucopyranoside, sel de sodium;
Méthyl *O*-(acide 2,3-di-*O*-méthyl-4-O-sulfo-β-D-glucopyranosyluronique)-(1-4)-[*O*-(2,3,6-tri-*O*-sulfo-α-D-glucopyranosyl)-(1-4)-*O*-( acide 2,3-di-*O*-méthyl-β-D-glucopyranosyluronique)-(1-4)]₄-2,3,6-tri-*O*-sulfo-α-D-glucopyranoside, sel de sodium;
Méthyl *O*-(acide 2,3-di-*O*-méthyl-4-O-sulfo-β-D-glucopyranosyluronique)-(1-4)-[*O*-(2,3,6-tri-*O*-sulfo-α-D-glucopyranosyl)-(1-4)-*O*-(acide 2,3-di-*O*-méthyl-β-D-glucopyranosyluronique)-(1-4)]₃-2,3,6-tri-*O*-sulfo-α-D-glucopyranoside, sel de sodium;
Méthyl *O*-(acide 3-*O*-méthyl-2,4-di-*O*-sulfo-α-L-idopyranosyluronique)-(1-4)-[*O*-(3-*O*-méthyl-2,6-di-*O*-sulfo-α-D-glucopyranosyl)-(1-4)-*O*-(acide 3-*O*-méthyl-2-*O*-sulfo-α-L-idopyranosyluronique)-(1-4)]₄-3-*O*-méthyl-2,6-di-*O*-sulfo-α-D-glucopyranoside, sel de sodium;
Méthyl *O*-(acide 3-*O*-méthyl-2,4-di-*O*-sulfo-α-L-idopyranosyluronique)-(1-4)-[*O*-(3-*O*-méthyl-2,6-di-*O*-sulfo-α-D-glucopyranosyl)-(1-4)-*O*-(acide 3-*O*-méthyl-2-*O*-sulfo-α-L-idopyranosyluronique)-(1-4)]₃-3-*O*-méthyl-2,6-di-*O*-sulfo-α-D-glucopyranoside, sel de sodium;
Méthyl *O*-(acide 3-*O*-méthyl-2,4-di-*O*-sulfo-α-L-idopyranosyluronique)-(1-4)-[*O*-(3-*O*-méthyl-2,6-di-*O*-sulfo-α-D-glucopyranosyl)-(1-4)-*O*-(acide 3-*O*-méthyl-2-O-sulfo-α-L-idopyranosyluronique)-(1-4)]₅-3-*O*-méthyl-2,6-di-*O*-sulfo-α-D-glucopyranoside, sel de sodium;
Méthyl *O*-(acide 3-*O*-méthyl-2,4-di-*O*-sulfo-β-D-glucopyranosyluronique)-(1-4)-[*O*-(3-*O*-méthyl-2,6-di-*O*-sulfo-α-D-glucopyranosyl)-(1-4)-*O*-(acide 3-*O*-méthyl-2-*O*-sulfo-β-D-glucopyranosyluronique)-(1-4)]₄-3-*O*-méthyl-2,6-di-*O*-sulfo-α-D-glucopyranoside, sel de sodium;
Méthyl *O*-(acide 3-*O*-méthyl-2,4-di-*O*-sulfo-α-L-idopyranosyluronique)-(1-4)-[*O*-(3-*O*-méthyl-2,6-di-*O*-sulfo-α-D-glucopyranosyl)-(1-4)-*O*-(acide 3-*O*-méthyl-2-*O*-sulfo-α-L-idopyranosyluronique)-(1-4)]₂-*O*-(2,3,6-tri-*O*-sulfo-α -D-glucopyranosyl)-(1-4)-*O*-(acide 3-*O*-méthyl-2-*O*-sulfo-α-L-idopyranosyluronique)-(1-4)-3-*O*-méthyl-2,6-di-*O*-sulfo-α-D-glucopyranoside, sel de sodium; et
Méthyl *O*-(acide 3-*O*-méthyl-2,4-di-*O*-sulfo-α-L-idopyranosyluronique)-(1-4)-[*O*-(3-*O*-méthyl-2,6-di-*O*-sulfo-α-D-glucopyranosyl)-(1-4)-*O*-(acide 3-*O*-méthyl-2-*O*-sulfo-α-L-idopyranosyluronique)-(1-4)]₃-*O*-(2,3,6-tri-*O*-sulfo-α -D-glucopyranosyl)-(1-4)-*O*-(acide 3-*O*-méthyl-2-*O*-sulfo-α-L-idopyranosyluronique)-(1-4)-3-*O*-méthyl-2,6-di-*O*-sulfo-α-D-glucopyranoside, sel de sodium.

15. Procédé de préparation des composés de formule (I) selon la revendication 2, **caractérisé en ce que** :
(a) on couple selon les méthodes classiques de la chimie des sucres un monosaccharide donneur de liaison glycosidique à un monosaccharide accepteur de liaison glycosidique pour obtenir un synthon saccharidique intermédiaire de type disaccharide complètement protégé de formule (A): 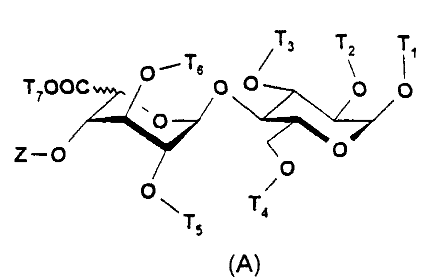 dans laquelle les substituants T₁, T₂, T₃, T₄, T₅, T₆, T₇, T₈ et Z identiques ou différents sont choisis parmi les groupes protecteurs utilisés en chimie des sucres comme groupe protecteur permanent, semi-permanent ou temporaire,
(b) on modifie chimiquement le disaccharide de formule (A) ci-dessus de façon à obtenir un synthon saccharidique intermédiaire de type disaccharide donneur de liaison glycosidique de formule (B) : 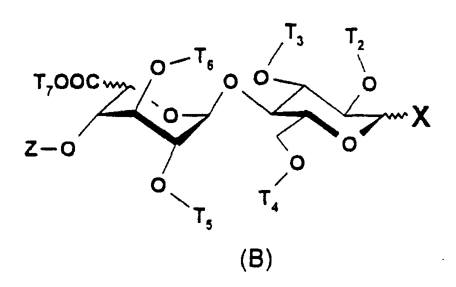 dans laquelle T₂ à T₇ et Z sont tels que définis ci-dessus pour (A) et X représente un groupe activateur du carbone anomérique, puis
(c) on modifie chimiquement le disaccharide de formule (A) ci-dessus de façon à obtenir un synthon saccharidique intermédiaire de type disaccharide accepteur de liaison glycosidique de formule (C) : 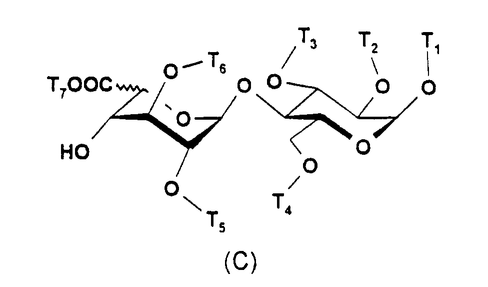 dans laquelle T₁ à T₇ sont tels que définis ci-dessus pour (A), en éliminant sélectivement le groupe protecteur Z selon des méthodes classiques de la chimie des sucres, puis
(d) on couple un disaccharide donneur de liaison glycosidique de formule (B) obtenu ci-dessus et un disaccharide accepteur de liaison glycosidique de formule (C) obtenu ci-dessus de façon à obtenir un tétrasaccharide complètement protégé de formule (D) : 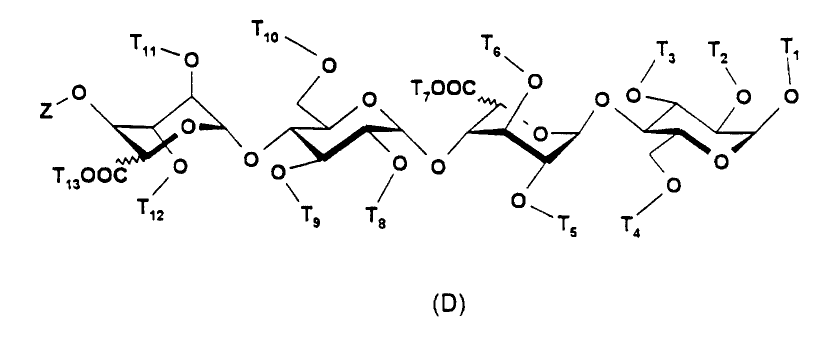 dans laquelle T₁ à T₇ et Z sont tels que définis ci-dessus pour (A) et T₈, T₉, T₁₀, T₁₁, T₁₂ et T₁₃ sont tels que définis pour T₂ à T₇ puis,
(e) on modifie ensuite chimiquement le synthon saccharidique intermédiaire de type tétrasaccharide de formule (D) de façon à obtenir un synthon saccharidique intermédiaire de type tétrasaccharide donneur de liaison glycosidique de formule (E) : 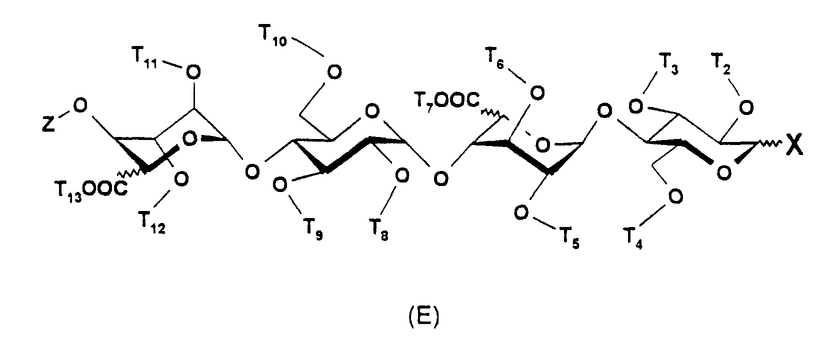 dans lequel X a la même définition que pour (B) et T₂ à T₁₃ sont tels que définis pour (D) puis,
(f) on déprotège ensuite sélectivement le tétrasaccharide de formule (D) de façon à obtenir un tétrasaccharide accepteur de liaison glycosidique de formule (F) : 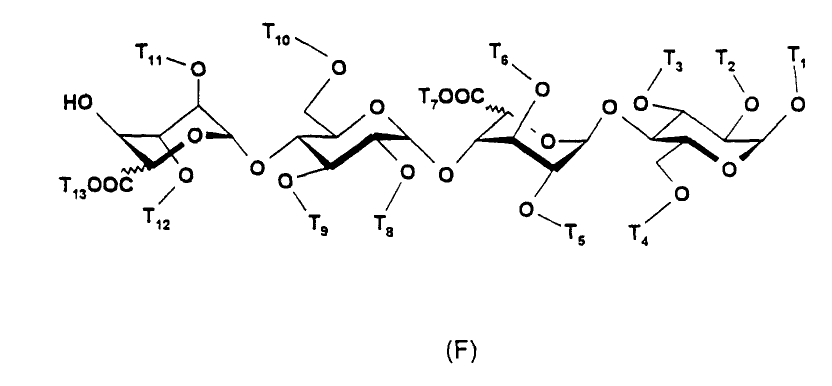 dans laquelle T₁ à T₁₃ sont tels que définis précédemment pour (D) puis,
(g) on couple le tétrasaccharide accepteur de liaison glycosidique de formule (F) et un disaccharide donneur de liaison glycosidique de formule (B) tel que ceux obtenus ci-dessus pour former un synthon intermédiaire de type hexasaccharide complètement protégé de formule (G) : 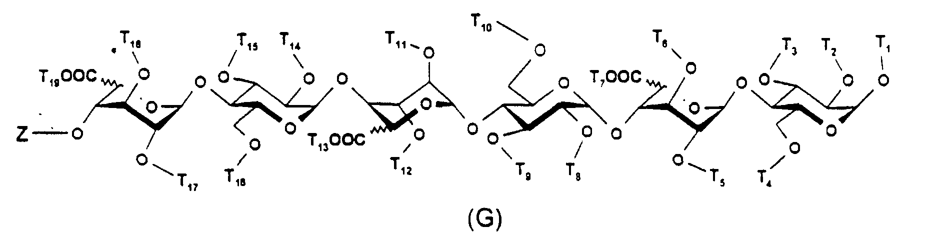 dans laquelle T₁ à T₁₃ sont tels que définis précédemment pour (D) et T₁₄ à T₁₉ sont tels que définis pour T₂ à T₇ pour (B);
ou bien on couple le tétrasaccharide accepteur de liaison glycosidique de formule (F) et un tétrasaccharide donneur de liaison glycosidique de formule (E) de façon à obtenir un octasaccharide complètement protégé de formule (H) : 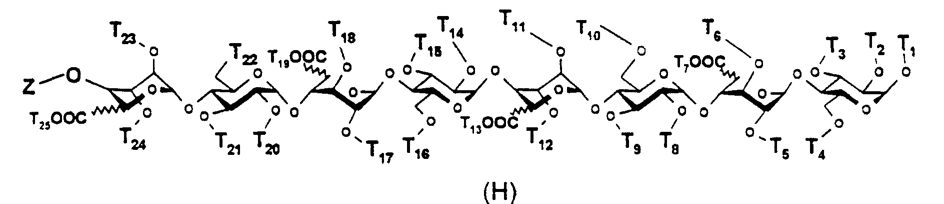 dans laquelle T₁ à T₁₉ et Z sont tels que définis précédemment et T₂₀ à T₂₅ sont tels que définis pour T₂ à T₇ pour (B) puis,
(h) on modifie chimiquement l'hexasaccharide de formule (G) ou l'octasaccharide de formule (H) obtenus ci-dessus de façon à obtenir un synthon intermédiaire de type hexasaccharide accepteur de liaison glycosidique de formule (G) dans lequel Z représente l'hydrogène ou bien un octasaccharide accepteur de liaison glycosidique de formule (H) dans lequel Z représente l'hydrogène,
(i) on répète les étapes de déprotection et de couplage précédentes jusqu'à l'obtention de l'oligosaccharide complètement protégé possédant la structure désirée, les synthons intermédiaires saccharidiques donneurs de glycosyle et accepteur de glycosyle étant choisis en fonction de la structure finale pour obtenir ainsi le précurseur protégé du polysaccharide final désiré de formule (I), dans lequel la nature des substituants protecteurs détermine la position des groupes sulfates et alkyles sur le produit final (I), et
(j) on procède à la déprotection des fonctions alcools qui doivent être sulfatées, en éliminant les substituants T₁ à T₂₅ qui protégeaient les fonctions au cours des étapes d'élaboration du squelette, puis, finalement
(k) on procède à la sulfatation pour obtenir les composés (I), ou un de leurs sels.

16. Compositions pharmaceutiques contenant comme principe actif un polysaccharide ou sel selon l'une quelconque des revendications 1 à 14, sous forme de sel avec une base pharmaceutiquement acceptable ou sous forme acide, en association ou en mélange avec un excipient inerte, non toxique, pharmaceutiquement acceptable.

17. Composition pharmaceutique selon la revendication 16, sous forme d'unités de dosage, dans laquelle le principe actif est mélangé à au moins un excipient pharmaceutique.

18. Composition selon la revendication 17 dans laquelle chaque unité de dosage contient de 0,1 à 100 mg de principe actif.

19. Composition selon la revendication 18 dans laquelle chaque unité de dosage contient de 0,5 à 50 mg de principe actif.

20. Composition pharmaceutique contenant un polysaccharide ou sel selon les revendications 1 à 14 en association avec un autre principe actif antithrombotique, anticoagulant, antiagrégant plaquettaire ou antagoniste du complexe de la glycoproteine IIb/IIIa.

21. Composition pharmaceutique selon la revendication 20 **caractérisé en ce que** le principe actif associé est le dipyridamole, l'aspirine, la ticlopidine ou le clopidogrel.

22. Utilisation des polysaccharide et sel selon les revendications 1 à 14 pour la préparation d'un médicament utile dans les pathologies dépendantes d'un dysfonctionnement de la coagulation.

23. Utilisation des polysaccharide et sel selon les revendications 1 à 14 pour la préparation d'un médicament utile pour l'inhibition des facteurs de croissance se traduisant par une inhibition de la prolifération cellulaire.

24. Utilisation des polysaccharide et sel selon les revendications 1 à 14 pour la préparation d'un médicament présentant des propriétés antivirales, hypolipidémiantes, antiradicaux libres, antimétastasiques, antiangiogéniques, anti-inflammatoires.

## Patentansprüche

1. Synthetisches Polysaccharid enthaltend 8 bis 24 Monosaccharid-Einheiten gebildet durch Verknüpfung von Disacchariden, die aus einer Uronsäure und einer Hexose gebildet sind, welches Polysaccharid **dadurch gekennzeichnet** ist, daß sämtliche Hydroxylgruppen mit einer (C₁-C₆)-Alkylgruppe verethert oder in Form der Sulfogruppe verestert sind, wobei jedes Disaccharid mindestens eine Ethergruppe aufweist, sowie deren Salze.

2. Salz aus einem Anion und einem Kation, wobei das Anion der folgenden Formel entspricht: 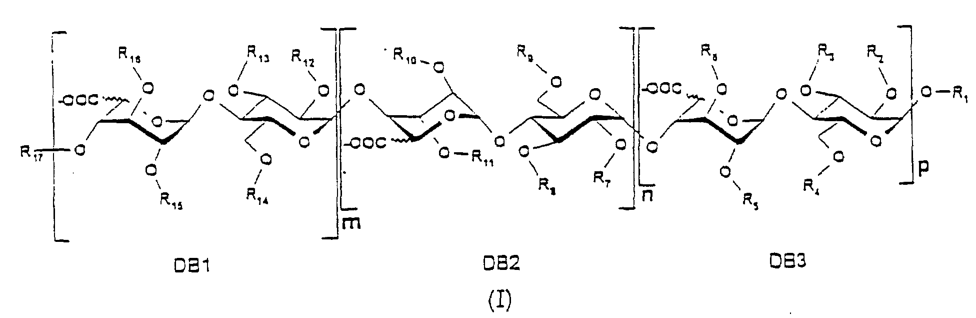 in der
- der gewellte Strich entweder eine Bindung unterhalb oder oberhalb der Ebene des Pyranoserings bedeutet;
- R₁, R₆, R₁₁ und R₁₆ eine (C₁-C₆)-Alkylgruppe bedeuten;
- R₂, R₃, R₄, R₅, R₇, R₈, R₉, R₁₀, R₁₂, R₁₃, R₁₄, R₁₅ und R₁₇ eine (C₁-C₆)-Alkylgruppe oder eine SO₃⁻-Gruppe bedeuten;
- m, n und p solche Werte besitzen, daß die Summe m + n + p gleich oder größer 4 und kleiner oder gleich 12 ist, wobei einer oder zwei dieser drei Indizes Null bedeuten kann;
wobei das Kation ein einwertiges, pharmazeutisch annehmbares Kation ist, sowie die entsprechende Säure.

3. Salz nach Anspruch 2, worin das Kation aus Kationen der Alkalimetalle, insbesondere von Natrium und Kalium, ausgewählt ist.

4. Salz nach einem der Ansprüche 2 oder 3, worin die Alkylgruppen Methylgruppen sind, sowie die entsprechende Säure.

5. Salz nach einem der Ansprüche 2 oder 3, worin n und p Null bedeuten, sowie die entsprechende Säure.

6. Salz nach einem der Ansprüche 2 oder 3, worin n und p Null und m 4 bis 10 bedeuten, sowie die entsprechende Säure.

7. Salz nach einem der Ansprüche 2 oder 3, worin n und p Null sind, m 4 bis 10 bedeutet; mindestens einer der Substituenten R₁₂, R₁₃, R₁₄ und R₁₅ eine Sulfatgruppe bedeutet; und R₁, R₁₆ und R₁₇ die für (I) angegebenen Bedeutungen besitzen, sowie die entsprechende Säure.

8. Salz nach einem der Ansprüche 2 oder 3, worin n und p Null und m 4 bis 10 bedeuten; mindestens zwei der Substituenten R₁₂, R₁₃, R₁₄ und R₁₅ eine Sulfatgruppe bedeuten; und R₁, R₁₆ und R₁₇ die für (I) angegebenen Bedeutungen besitzen, sowie die entsprechende Säure.

9. Salz nach einem der Ansprüche 2 oder 3, worin n und p Null und m 4 bis 10 bedeuten; mindestens drei der Substituenten R₁₂, R₁₃, R₁₄ und R₁₅ eine Sulfatgruppe bedeuten; und R₁, R₁₆ und R₁₇ die für (I) angegebenen Bedeutungen besitzen, sowie die entsprechende Säure.

10. Salz nach Anspruch 2 oder 3, worin das Anion der Formel (I.1) entspricht: 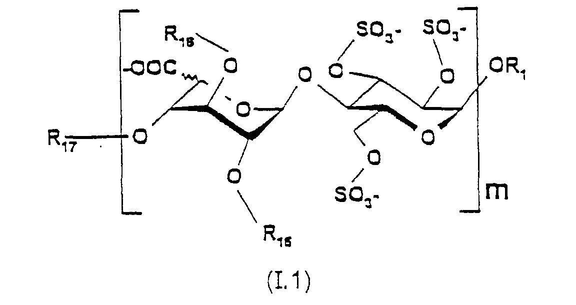 in der m 4 bis 10 bedeutet; und R₁, R₁₅, R₁₆ und R₁₇ die für (I) angegebenen Bedeutungen besitzen, wobei jede Uronsäure entweder eine Iduronsäure oder eine Glucuronsäure ist, sowie die entsprechende Säure.

11. Salz nach einem der Ansprüche 2 oder 3, worin das Anion der Formel (I.2) entspricht: 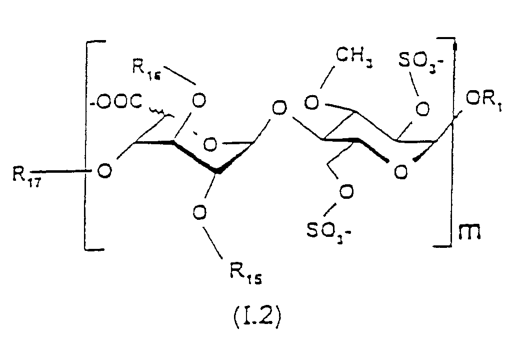 in der m 4 bis 10 bedeutet; und R₁, R₁₅, R₁₆ und R₁₇ die für (I) angegebenen Bedeutungen besitzen, wobei jede Uronsäure entweder eine Iduronsäure oder eine Glucuronsäure ist, sowie die entsprechende Säure.

12. Salz nach den Ansprüchen 2 oder 3, worin das Anion der Formel (I.3) entspricht: 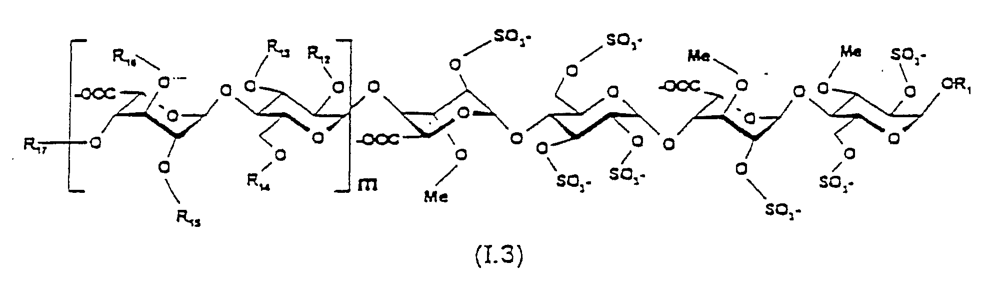 in der m 2 oder 3 bedeutet und R₁, R₁₂, R₁₃, R₁₄, R₁₅, R₁₆ und R₁₇ die für (I) angegebenen Bedeutungen besitzen, wobei jede Uronsäure entweder eine Iduronsäure oder eine Glucuronsäure ist, sowie die entsprechende Säure.

13. Salz nach Anspruch 12, worin R₁ eine Methylgruppe, R₁₃ in der 3-Stellung der Glucose eine Methylgruppe, R₁₂ in der 2-Stellung und R₁₄ in der 6-Stellung der Glucose eine Gruppe SO₃⁻ und R₁₆ in der 3-Stellung der Iduronsäure-Einheit oder der Glucuronsäure-Einheit eine Methylgruppe und m 2 oder 3 bedeuten.

14. Polysaccharid ausgewählt aus:
Methyl-*O*-(2,3-di-*O*-methyl-4-O-sulfo-α-L-idopyranosyluronsäure)-(1-4)-[*O*-(2,3,6-tri-*O*-sulfo-α-D-glucopyranosyl)-(1-4)-*O*-(2,3-di-*O*-methyl-α-L-idopyranosyluronsäure)-(1-4)]₉-2,3,6-tri-*O*-sulfo-α-D-glucopyranosid, Natriumsalz;
Methyl-*O*-(2,3-di-*O*-methyl-4-O-sulfo-α-L-idopyranosyluronsäure)-(1-4)-[*O*-(2,3,6-tri-*O*-sulfo-α-D-glucopyranosyl)-(1-4)-*O*-(2,3-di-*O*-methyl-α-L-idopyranosyluronsäure)-(1-4)]₄-2,3,6-tri-*O*-sulfo-α-D-glucopyranosid, Natriumsalz;
Methyl-*O*-(2,3-di-*O*-methyl-4-O-sulfo-α-L-idopyranosyluronsäure)-(1-4)-[*O*-(2,3,6-tri-*O*-sulfo-α-D-glucopyranosyl)-(1-4)-*O*-(2,3-di-*O*-methyl-α-L-idopyranosyluronsäure)-(1-4)]₅-2,3,6-tri-*O*-sulfo-α-D-glucopyranosid, Natriumsalz;
Methyl-*O*-(2,3-di-0-methyl-4-O-sulfo-α-L-idopyranosyluronsäure)-(1-4)-[*O*-(2,3,6-tri-*O*-sulfo-α-D-glucopyranosyl)-(1-4)-*O*-(2,3-di-*O*-methyl-α-L-idopyranosyluronsäure)-(1-4)]₆-2,3,6-tri-*O*-sulfo-α-D-glucopyranosid, Natriumsalz;
Methyl-*O*-(2,3-di-*O*-methyl-4-O-sulfo-α-L-idopyranosyluronsäure)-(1-4)-[*O*-(2,3,6-tri-*O*-sulfo-α-D-glucopyranosyl)-(1-4)-*O*-(2,3-di-*O*-methyl-α-L-idopyranosyluronsäure)-(1-4)]₇-2,3,6-tri-*O*-sulfo-α-D-glucopyranosid, Natriumsalz;
Methyl-*O*-(2,3-di-*O*-methyl-4-O-sulfo-α-L-idopyranosyluronsäure)-(1-4)-[*O*-(2,3,6-tri-*O*-sulfo-α-D-glucopyranosyl)-(1-4)-*O*-(2,3-di-*O*-methyl-α-L-idopyranosyluronsäure)-(1-4)]₈-2,3,6-tri-*O*-sulfo-α-D-glucopyranosid, Natriumsalz;
Methyl-*O*-(2,3-di-*O*-methyl-4-O-sulfo-β-D-glucopyranosyluronsäure)-(1-4)-[*O*-(2,3,6-tri-*O*-sulfo-α-D-glucopyranosyl)-(1-4)-*O*-(2,3-di-*O*-methyl-β-D-glucopyranosyluronsäure)-(1-4)]₄-2,3,6-tri-*O*-sulfo-α-D-glucopyranosid, Natriumsalz;
Methyl-*O*-(2,3-di-*O*-methyl-4-O-sulfo-β-D-glucopyranosyluronsäure)-(1-4)-[*O*-(2,3,6-tri-*O*-sulfo-α-D-glucopyranosyl)-(1-4)-*O*-(2,3-di-*O*-methyl-β-D-glucopyranosyluronsäure)-(1-4)]₃-2,3,6-tri-*O*-sulfo-α-D-glucopyranosid, Natriumsalz;
Methyl-*O*-(3-*O*-methyl-2,4-di-0-sulfo-α-L-idopyranosyluronsäure)-(1-4)-[*O*-(3-*O*-methyl-2,6-di-*O*-sulfo-α-D-glucopyranosyl)-(1-4)-*O*-(3-*O*-methyl-2-*O*-sulfo-α-L-idopyranosyluronsäure)-(1-4)]₄-3-*O*-methyl-2,6-di-*O*-sulfo-α-D-glucopyranosid, Natriumsalz;
Methyl-*O*-(3-*O*-methyl-2,4-di-*O*-sulfo-α-L-idopyranosyluronsäure)-(1-4)-[*O*-(3-*O*-methyl-2,6-di-*O*-sulfo-α-D-glucopyranosyl)-(1-4)-*O*-(3-*O*-methyl-2-*O*-sulfo-α-L-idopyranosyluronsäure)-(1-4)]₃-3-*O*-methyl-2,6-di-*O*-sulfo-α-D-glucopyranosid, Natriumsalz;
Methyl-*O*-(3-*O*-methyl-2,4-di-*O*-sulfo-α-L-idopyranosyluronsäure)-(1-4)-[*O*-(3-*O*-methyl-2,6-di-*O*-sulfo-α-D-glucopyranosyl)-(1-4)-*O*-(3-*O*-methyl-2-*O*-sulfo-α-L-idopyranosyluronsäure)-(1-4)]₅-3-*O*-methyl-2,6-di-*O*-sulfo-α-D-glucopyranosid, Natriumsalz;
Methyl-*O*-(3-*O*-methyl-2,4-di-*O*-sulfo-β-D-glucopyranosyluronsäure)-(1-4)-[*O*-(3-*O*-methyl-2,6-di-*O*-sulfo-α-D-glucopyranosyl)-(1-4)-*O*-(3-*O*-methyl-2-*O*-sulfo-β-D-glucopyranosyluronsäure)-(1-4)]₄-3-*O*-methyl-2,6-di-*O*-sulfo-α-D-glucopyranosid, Natriumsalz;
Methyl-*O*-(3-*O*-methyl-2,4-di-*O*-sulfo-α-L-idopyranosyluronsäure)-(1-4)-[*O*-(3-*O*-methyl-2,6-di-*O*-sulfo-α-D-glucopyranosyl)-(1-4)-*O*-(3-*O*-methyl-2-*O*-sulfo-α-L-idopyranosyluronsäure)-(1-4)]₂-*O*-(2,3,6-tri-*O*-sulfo-α-D-glucopyranosyl)-(1-4)-*O*-(3-*O*-methyl-2-*O*-sulfo-α-L-idopyranosyluronsäure-(1-4)-3-*O*-methyl-2,6-di-*O*-sulfo-α-D-glucopyranosid, Natriumsalz; und
Methyl-*O*-(3-*O*-methyl-2,4-di-*O*-sulfo-α-L-idopyranosyluronsäure)-(1-4)-[*O*-(3-*O-*methyl-2,6-di-*O*-sulfo-α-D-glucopyranosyl)-(1-4)-*O*-(3-*O*-methyl-2-*O*-sulfo-α-L-idopyranosyluronsäure)-(1-4)]₃-*O*-(2,3,6-tri-*O*-sulfo-α-D-glucopyranosyl)-(1-4)-*O*-(3-*O*-methyl-2-*O*-sulfo-α-L-idopyranosyluronsäure-(1-4)-3-*O*-methyl-2,6-di-*O*-sulfo-α-D-glucopyranosid, Natriumsalz.

15. Verfahren zur Herstellung der Verbindungen der Formel (I) nach Anspruch 2, **dadurch gekennzeichnet**, daß man:
(a) ein Monosaccharid, welches einen Donor für die glykosidische Bindung darstellt, nach klassichen Methoden der Zuckerchemie mit einem Monosaccharid, welches einen Akzeptor für die glykosidische Bindung darstellt, kuppelt zur Bildung eines vollständig geschützten Zwischenprodukt-Saccharid-Synthons des Disaccharid-Typs der Formel (A): 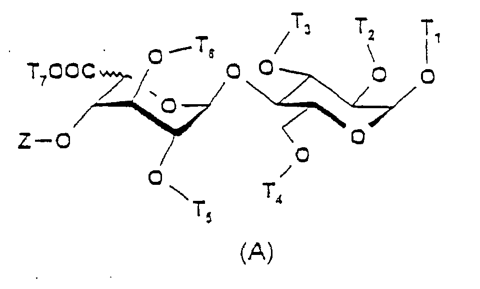 worin die Substituenten T₁, T₂, T₃, T₄, T₅, T₆, T₇, T₈ und Z, die gleichartig oder verschieden sind, ausgewählt sind aus Schutzgruppen, die in der Zukkerchemie als permanente, semipermanente oder temporäre Schutzgruppen verwendet werden,
(b) man das obige Disaccharid der Formel (A) chemisch modifiziert, derart, daß man ein Zwischenprodukt-Saccharid-Synthon des Typs eines Disaccharid-Donors für die glykosidische Bindung der Formel (B) erhält: 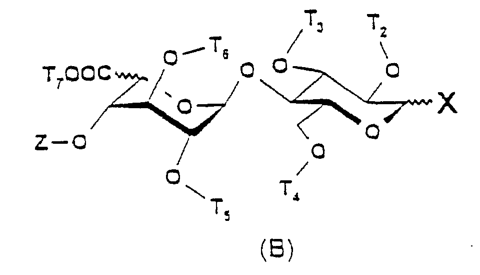 in der T₂ bis T₇ und Z die oben für (A) angegebenen Bedeutungen besitzen und X eine Aktivatorgruppe des anomeren Kohlenstoffatoms bedeutet, anschließend
(c) das obige Disaccharid der Formel (A) durch selektive Abspaltung der Schutzgruppe Z gemäß klassischen Methoden der Zuckerchemie chemisch modifiziert, so daß man ein Zwischenprodukt-Saccharid-Synthon des Typs eines Disaccharid-Akzeptors für die glykosidische Bindung der Formel (C) erhält: 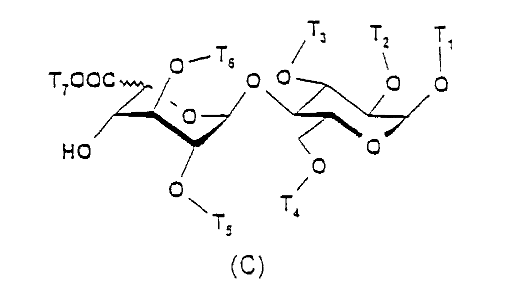 in der T₁ bis T₇ die oben für (A) angegebenen Bedeutungen besitzen, dann
(d) ein in der obigen Weise erhaltenes Donor-Disaccharid für die glykosidische Bindung der Formel (B) mit einem in der obigen Weise erhaltenen Akzeptor-Disaccharid für die glykosidische Bindung der Formel (C) kuppelt zur Bildung eines vollständig geschützten Tetrasaccharids der Formel (D): 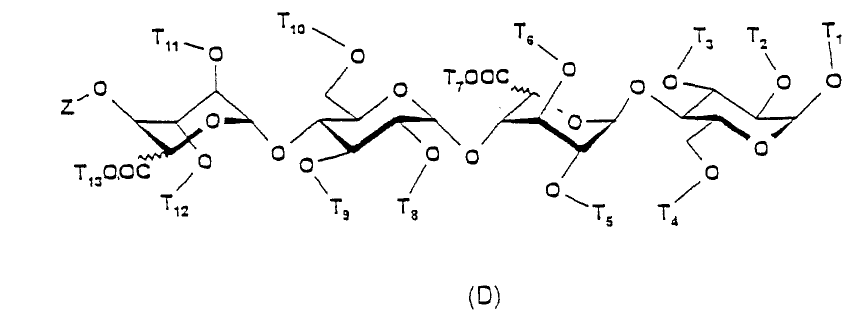 in der T₁ bis T₇ und Z die oben für (A) angegebenen Bedeutungen besitzen und T₈, T₉, T₁₀, T₁₁, T₁₂ und T₁₃ die oben für T₂ bis T₇ angegebenen Bedeutungen besitzen, und dann
(e) man anschließend das Zwischenprodukt-Saccharid-Synthon des Tetrasaccharid-Typs der Formel (D) chemisch modifiziert zur Bildung eines Zwischenprodukt-Saccharid-Synthons des Typs Donor-Tetrasaccharid für die glykosidische Bindung der Formel (E): 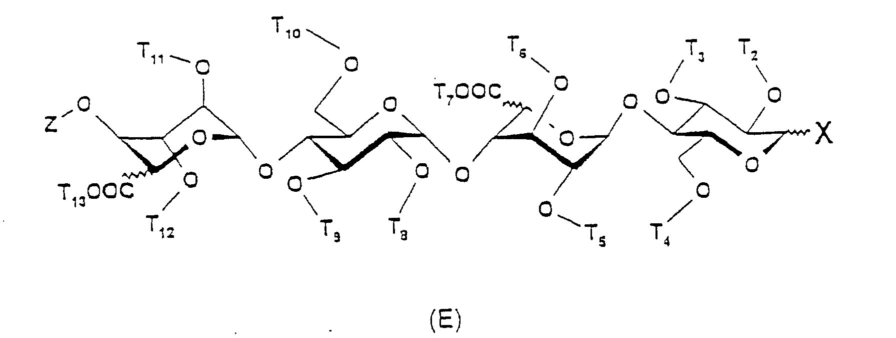 in der X die für (B) angegebenen Bedeutungen besitzt und T₂ bis T₁₃ die für (D) angegebenen Bedeutungen besitzen, dann
(f) man anschließend selektiv die Schutzgruppen von dem Tetrasaccharid der Formel (D) abspaltet zur Bildung eines Akzeptor-Tetrasaccharids für die glykosidische Bindung der Formel (F): 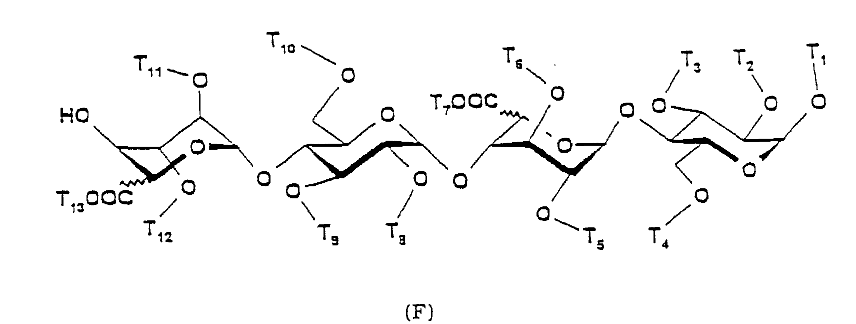 in der T₁ bis T₁₃ die oben für (D) angegebenen Bedeutungen besitzen, dann
(g) man das Akzeptor-Tetrasaccharid für die glykosidische Bindung der Formel (F) und ein Donor-Disaccharid für die glykosidische Bindung der Formel (B), wie man sie oben erhalten hat, kuppelt zur Bildung eines vollständig geschützten Zwischenprodukt-Synthons des Hexasaccharid-Typs der Formel (G): 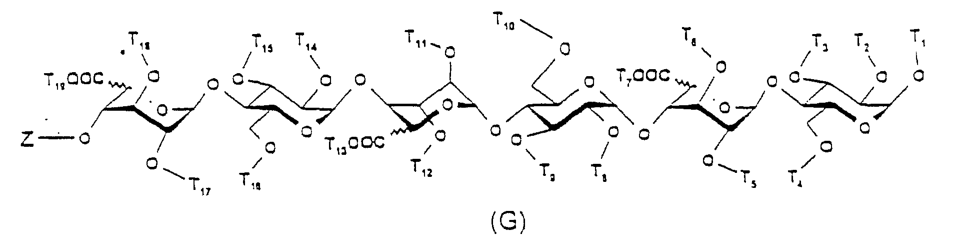 in der T₁ bis T₁₃ die für (D) angegebenen Bedeutungen besitzen und T₁₄ bis T₁₉ die Bedeutungen besitzen, wie sie für T₂ bis T₇ für (B) definiert worden sind;
oder man das Akzeptor-Tetrasaccharid für die glykosidische Bindung der Formel (F) und ein Donor-Tetrasaccharid für die glykosidische Bindung der Formel (E) kuppelt, derart, daß man ein vollständig geschütztes Octasaccharid der Formel (H) erhält: 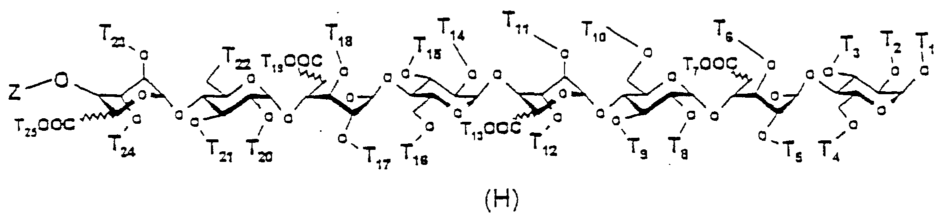 in der T₁ bis T₁₉ und Z die oben angegebenen Bedeutungen besitzen und T₂₀ bis T₂₅ die für (B) angegebenen Bedeutungen von T₂ bis T₇ besitzen, dann
(h) man das in der obigen Weise erhaltene Hexasaccharid der Formel (G) oder Octasaccharid der Formel (H) chemisch modifiziert zur Bildung eines Zwischenprodukt-Synthons vom Typ Akzeptor-Hexasaccharid für die glykosidische Bindung der Formel (G), worin Z ein Wasserstoffatom bedeutet, oder eines Akzeptor-Octasaccharids für die glykosidische Bindung der Formel (H), in der Z ein Wasserstoffatom bedeutet,
(i) man die oben angegebenen Stufen der Schutzgruppenabspaltung und der Kupplung wiederholt zur Bildung eines vollständig geschützten Oligosaccharids mit der gewünschten Struktur, wobei die Zwischenprodukt-SaccharidSynthons als Glykosyl-Donoren und Glykosyl-Akzeptoren in Abhängigkeit von der angestrebten Endstruktur ausgewählt werden, so daß man in dieser Weise den geschützten Vorläufer des gewünschten End-Polysaccharids der Formel (I) erhält, in der die Art der Schutzgruppensubstituenten die Stellung der Sulfatgruppen und der Alkylgruppen an dem Endprodukt (I) bestimmen, und
(k) man die Abspaltung der Alkoholfunktionen, welche sulfatiert werden sollen, bewirkt durch Eliminieren der Substituenten T₁ bis T₂₅, welche die Funktionen im Verlaufe der Stufen des Aufbaus des Skeletts schützen, und schließlich
(k) man die Sulfatierung bewirkt zur Bildung der Verbindungen (I) oder eines ihrer Salze.

16. Pharmazeutische Zubereitungen enthaltend als Wirkstoff ein Polysaccharid nach einem der Ansprüche 1 bis 14 in Form des Salzes mit einer pharmazeutisch annehmbaren Base oder in Form der Säure in Kombination oder in Mischung mit einem inerten, nichttoxischen, pharmazeutisch annehmbaren Trägermaterial.

17. Pharmazeutische Zubereitung nach Anspruch 16 in Form von Dosiseinheiten, worin der Wirkstoff mit mindestens einem pharmazeutischen Trägermaterial vermischt ist.

18. Zubereitung nach Anspruch 17, worin jede Dosiseinheit 0,1 bis 100 mg des Wirkstoffs enthält.

19. Zubereitung nach Anspruch 18, worin jede Dosiseinheit 0,5 bis 50 mg des Wirkstoffs enthält.

20. Pharmazeutische Zubereitung enthaltend ein Polysaccharid oder ein Salz nach den Ansprüchen 1 bis 14 in Kombination mit einem weiteren Wirkstoff mit antithrombotischer Wirkung, antikoagulierender Wirkung, gegen die Plättchenaggregation gerichteter Wirkung oder antagonistischer Wirkung bezüglich des Glykoprotein IIb/IIIa-Komplexes.

21. Pharmazeutische Zubereitung nach Anspruch 20, **dadurch gekennzeichnet**, daß der weitere Wirkstoff Dipyridamol, Aspirin, Ticlopidin oder Clopidogrel ist.

22. Verwendung der Polysaccharide und der Salze nach den Ansprüchen 1 bis 14 für die Herstellung eines Arzneimittels für die Behandlung von pathologischen Zuständen, die von einer Dysfunktion der Blutgerinnung abhängen.

23. Verwendung der Polysaccharide und der Salze nach den Ansprüchen 1 bis 14 für die Herstellung eines Arzneimittels für die Inhibierung von Wachstumsfaktoren, die sich in einer Inhibierung der Zellproliferation manifestieren.

24. Verwendung der Polysaccharide und der Salze nach den Ansprüchen 1 bis 14 für die Herstellung eines Arzneimittels mit antiviraler Wirkung, hypolipidämischer Wirkung, gegen freie Radikale gerichteter Wirkung, Antimetastase-Wirkung, antiangiogener Wirkung und antiinflammatorischer Wirkung.
